# EUROPEAN PATENT APPLICATION

(11) **EP 4 722 370 A1**
(43) Date of publication of application: **08.04.2026**
(21) Application number: 24835345.0
(22) Date of filing: 02.07.2024
(51) Int. Cl.: C12N 15/62, C12N 5/10, C07K 19/00, A61P 35/00

(54) **COMPOSITION FOR TREATING CANCER AND USE THEREOF**

(30) Priority: 03.07.2023 US 202363511750 P; 16.10.2023 CN 202311339565
(71) Applicant: Shanghai Simnova Biotechnology Co., Ltd., Shanghai 201321 (CN)
(72) Inventor: HUANG, Xue, Cambridge Massachusetts 02140 (US); HUANG, Pengyu, Cambridge Massachusetts 02140 (US); WANG, Yinghua, Cambridge Massachusetts 02140 (US); WU, Cheng-Ying, Cambridge Massachusetts 02140 (US); LO, Chi-Wen, Cambridge Massachusetts 02140 (US); WANG, Chao, Shanghai 201318 (CN); GAO, Fanxiang, Shanghai 201318 (CN); JIANG, Fuwei, Shanghai 201318 (CN); YANG, Yu, Shanghai 201318 (CN); YANG, Cuiqing, Shanghai 201318 (CN); CAO, Zhuoxiao, Shanghai 201318 (CN)
(74) Representative: V.O.
(86) International application number: PCT/CN2024/103178
(87) International publication number: WO 2025/007864

(57) **Abstract**

Provides are polynucleotides and vectors comprising sequences encoding a CAR and an armoring molecule capable of activating a CD70/CD27 signaling pathway. The disclosure also provides immune effector cells comprising one or more of the polynucleotides or vectors; compositions and kits comprising one or more of the immune effector cells; methods of treating a cancer in mammalian subject, and methods of inducing T cell-mediated cytolysis of cancer cells (e.g., solid tumor cells).

## Description

### CROSS-REFERENCE TO RELATED APPLICATION

The present disclosure claims priority to the U.S. Provisional Application No. 63/511,750 filed on July 3, 2023, and the Chinese Patent Application No. 202311339565.8 filed on October 16, 2023, which are incorporated herein by reference in their entireties for all purposes.

### TECHNICAL FIELD

The present disclosure relates to the field of cancer treatment, and in particular, to a composition for treating cancer and use thereof.

### BACKGROUND

Therapies that attack tumors by engaging the immune system have been effective against a growing number of cancers. Human T cells have been transduced to express chimeric antigen receptors (CAR) for specific and efficient targeting and killing of cancer cells. In some cancer types, particularly in solid tumors, however, a hostile microenvironment limits effector function, expansion, and survival of CAR-T cells. Eliciting sustained antitumor activity remains a major challenge for CAR-T cell therapy for solid tumors.

### SUMMARY

There is a critical need to develop therapeutic agents for treating cancer, particularly immune effector cells capable of expanding and/or having persistent antitumor activity in the tumor microenvironment. The disclosure provides such therapeutic agents.

The disclosure provided herein is based, in part, on the discovery that immune effector cells secreting CD70 show enhanced persistence and prolonged antitumor activity in human cancer models. Accordingly, the disclosure generally relates to immune effector cells (e.g., CAR-T cells), polypeptides, polynucleotides, pharmaceutical compositions, and methods that are useful for generating immune effector cells having enhanced persistence and/or prolonged antitumor activity.

In one aspect, the disclosure provides polynucleotides encoding: (a) a chimeric antigen receptor (CAR), wherein the CAR comprises an antigen-binding domain specific for a cell surface antigen, and (b) an armoring molecule capable of activating a CD70/CD27 signaling pathway.

In some specific embodiments, the polypeptides comprise an amino acid sequence that has at least 90% sequence identity to an amino acid sequence set forth in any one of SEQ ID NO: 25, 37, 44 and 63.

In another aspect, the disclosure provides vectors, wherein the vectors comprise one or more of the polynucleotides disclosed herein.

In another aspect, the disclosure provides fusion proteins encoded by any one of the polynucleotides or vectors disclosed herein.

In another aspect, the disclosure provides polypeptides, wherein the polypeptides comprise an amino acid sequence that has at least 90% sequence identity to an amino acid sequence set forth in any one of SEQ ID NO: 25, 37, 44 and 63.

In another aspect, the disclosure provides fusion proteins, wherein the fusion proteins comprise: (a) a CAR comprising an antigen-binding domain specific for a cell surface antigen, and (b) an armoring molecule capable of activating a CD70/CD27 signaling pathway.

In another aspect, the disclosure provides host cells, wherein the host cells comprise one or more of the polynucleotides, vectors, or fusion proteins disclosed herein.

In another aspect, the disclosure provides immune effector cells (e.g., T lymphocytes), wherein the immune effector cells comprise one or more of the polynucleotides, vectors, or fusion proteins disclosed herein.

In another aspect, the disclosure provides immune effector cells (e.g., T lymphocytes), wherein the immune effector cells comprise a polynucleotide encoding a soluble CD70.

In another aspect, the disclosure provides immune effector cells (e.g., T lymphocytes), wherein the immune effector cells express a CAR comprising an antigen-binding domain specific for a cell surface antigen, and wherein the immune effector cells secret a recombinant armoring molecule capable of activating a CD70/CD27 signaling pathway.

In another aspect, the disclosure provides immune effector cells (e.g., T lymphocytes), wherein the immune effector cells express (a) a CAR comprising an antigen-binding domain specific for a cell surface antigen, and (b) a recombinant armoring molecule capable of activating a CD70/CD27 signaling pathway.

In another aspect, the disclosure provides compositions comprising any one or more of the polynucleotides, vectors, fusion proteins, host cells, or immune effector cells (e.g., T lymphocytes) disclosed herein. In some embodiments, a composition comprises any one or more of the immune effector cells (e.g., T lymphocytes) disclosed herein.

In another aspect, the disclosure provides pharmaceutical compositions comprising any one or more of the compositions disclosed herein and a pharmaceutically acceptable carrier, excipient, stabilizer, diluent or tonifier.

In another aspect, the disclosure provides kits, wherein the kits comprise a container and, optionally, an instruction for use, wherein the container comprises one or more of the compositions (e.g., pharmaceutical compositions) disclosed herein.

In another aspect, the disclosure provides use of one or more polynucleotides, vectors, fusion proteins, host cells, immune effector cells (e.g., T lymphocytes), compositions (e.g., pharmaceutical compositions), or kits disclosed herein, for the preparation of a medicament for treating cancer in a subject in need thereof.

In another aspect, the disclosure provides one or more polynucleotides, vectors, fusion proteins, host cells, immune effector cells (e.g., T lymphocytes), compositions (e.g., pharmaceutical compositions), or kits disclosed herein, for use in treating cancer in a subject in need thereof. In some embodiments, the disclosure provides one or more immune effector cells (e.g., T lymphocytes), compositions, pharmaceutical compositions disclosed herein, for use in treating cancer in a subject in need thereof. In some embodiments, the disclosure provides one or more immune effector cells (e.g., T lymphocytes) disclosed herein, for use in treating cancer in a subject in need thereof.

In another aspect, the disclosure provides methods of treating cancer in a subject in need thereof, comprising administering to the subject an effective dosage of one or more immune effector cells (e.g., T lymphocytes), or compositions (e.g., pharmaceutical compositions).

In another aspect, the disclosure provides methods of inducing cytolysis of cancer cells, comprising contacting the cancer cells with any one or more of the immune effector cells (e.g., T lymphocytes), or pharmaceutical compositions disclosed herein. In some embodiments, the contacting occurs in vitro.

Without being bound by any theory or hypothesis, the polynucleotides, vectors, fusion proteins, host cells, T lymphocytes, or compositions (e.g., pharmaceutical compositions) described in this disclosure generally provide superior (sometimes unexpected) results in killing or otherwise rendering cancer cells less effective in comparison to existing polynucleotides, vectors, fusion proteins, host cells, T lymphocytes, or compositions (e.g., pharmaceutical compositions). Again, without being bound by any theory or hypothesis, one or more of the polynucleotides, vectors, fusion proteins, host cells, T lymphocytes, or compositions (e.g., pharmaceutical compositions) described in this disclosure can be used to effectively treat cancers, inter alia, with reduced side effects.

### BRIEF DESCRIPTION OF THE DRAWINGS

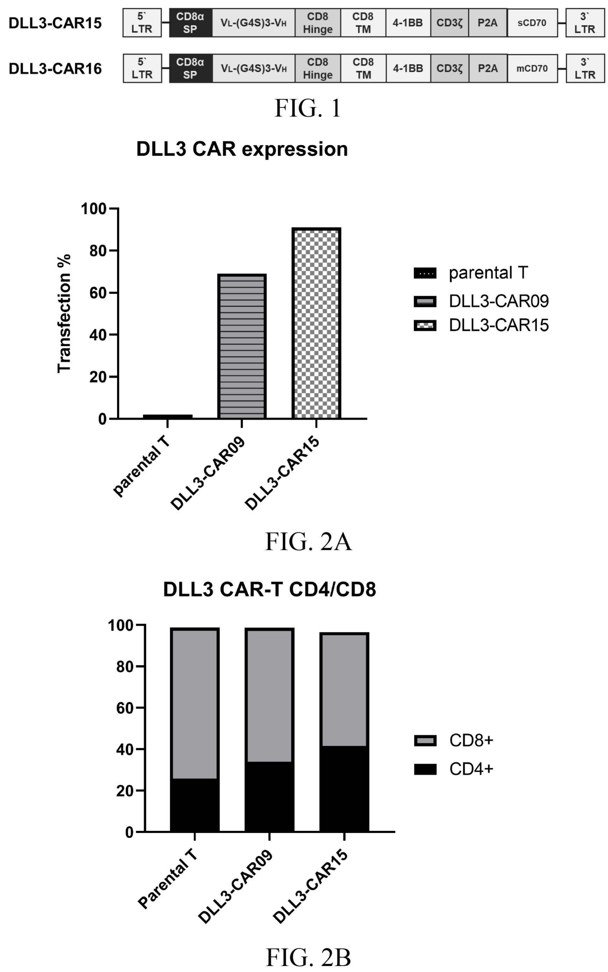

FIG. 1 shows diagrams of plasmid structure of sCD70-armored DLL3 CAR-T and membrane-bound CD70-armored DLL3 CAR-T.

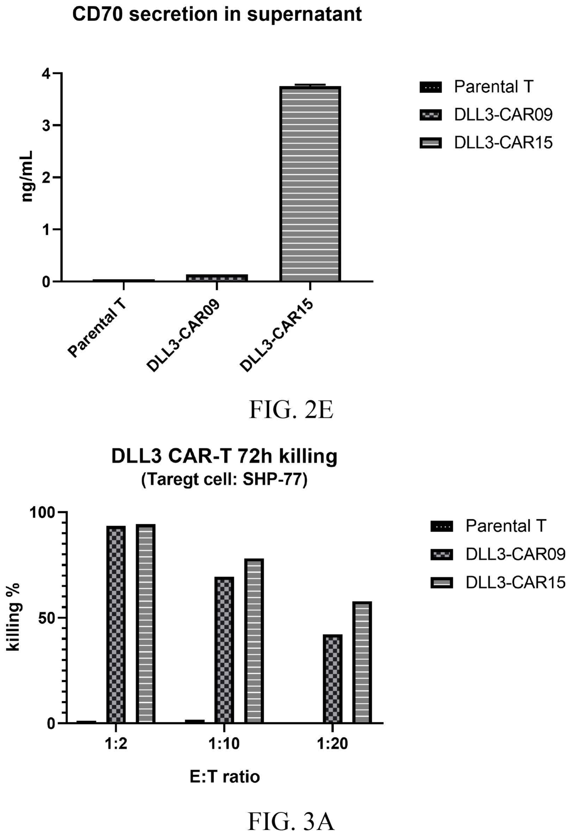

FIGs. 2A-2E show results of T cell characterization of DLL3 CAR-T cells and sCD70-armored DLL3 CAR-T cells after one week of in vitro expansion. FIGs. 2A-2D show the expression level of DLL3 CAR, CD4/CD8 ratio, and the expression level of T cell exhaustion phenotype and T cell memory phenotype after in vitro CAR-T expansion. FIG. 2E shows results of CD70 secretion by sCD70-armored DLL3 CAR-T. During in vitro culture, the supernatant of the culture medium (3-day culture) of CAR-T cells reaching a certain density (2E6/ml) was tested by ELISA for CD70 protein.

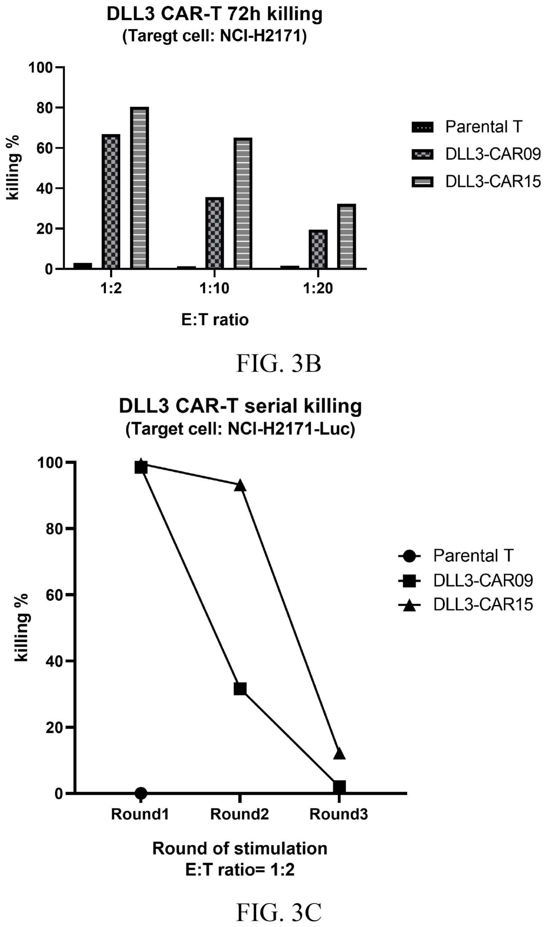

FIGs. 3A-3C show the results of in vitro killing evaluation of DLL3 CAR-T cells and sCD70-armored DLL3 CAR-T cells. FIG. 3A and FIG. 3B show the evaluation results of the in vitro tumor killing function of DLL3 CAR-T cells and sCD70-armored DLL3 CAR-T cells against SHP-77 (FIG. 3A) and NCI-H2171 cells (FIG. 3B) for 72 hours, with the effector-target ratio (E:T) being 1:2, 1:10, and 1:20, respectively. FIG. 3C shows evaluation results of the continuous killing function of DLL3 CAR-T cells and sCD70-armored DLL3 CAR-T cells against NCI-H2171-luc cells.

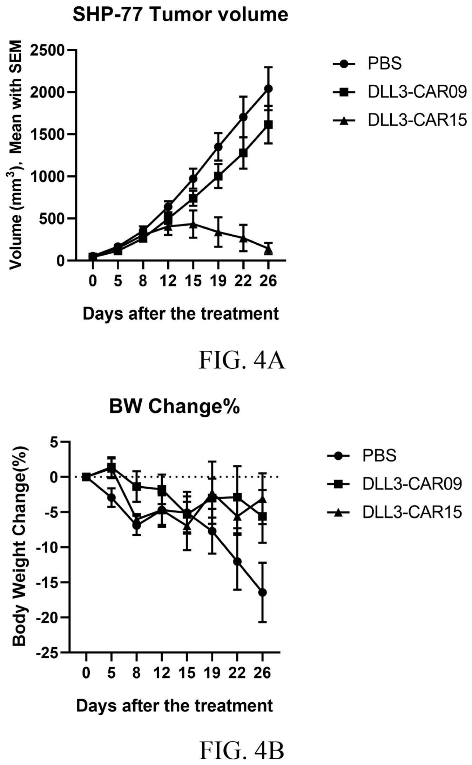

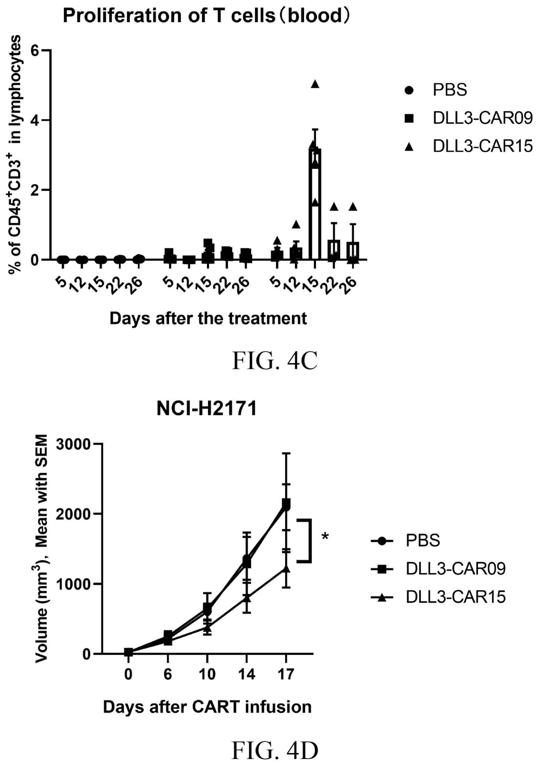

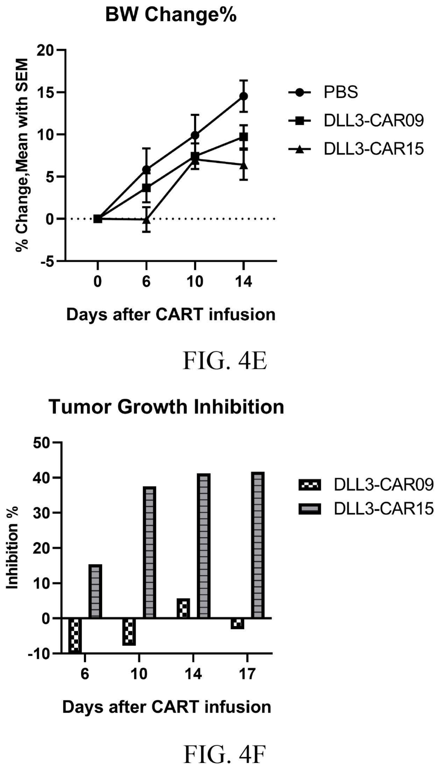

FIGs. 4A-4F show detection of the anti-tumor efficacy of sCD70-armored DLL3 CAR-T cells against mouse subcutaneous tumor model, wherein FIG. 4A shows a tumor growth curve of the SHP-77 NPG mouse subcutaneous tumor model; FIG. 4B shows a weight change curve of the SHP-77 NPG mouse subcutaneous tumor model; FIG. 4C shows the peripheral blood T cell detection results of the SHP-77 NPG mouse subcutaneous tumor model; FIG. 4D shows a tumor growth curve of the NCI-H2171 NPG mouse subcutaneous tumor model; FIG. 4E shows a weight change curve of the NCI-H2171 NPG mouse subcutaneous tumor model; FIG. 4F shows the tumor inhibition rate of each treatment group in the NCI-H2171 NPG mouse subcutaneous tumor model.

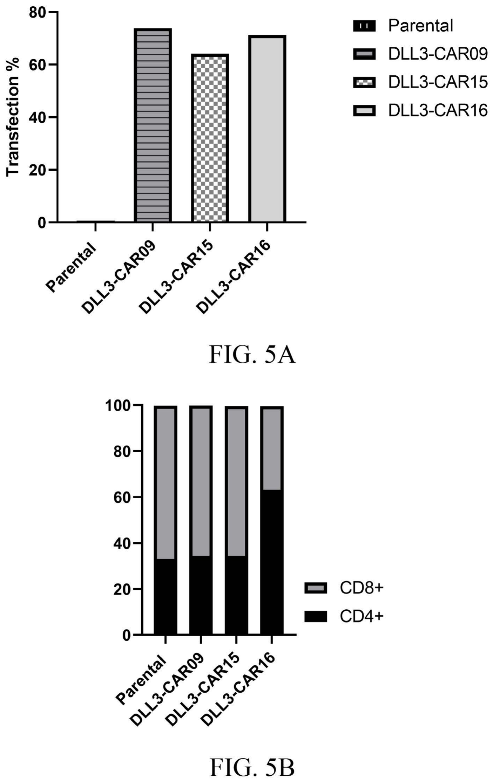

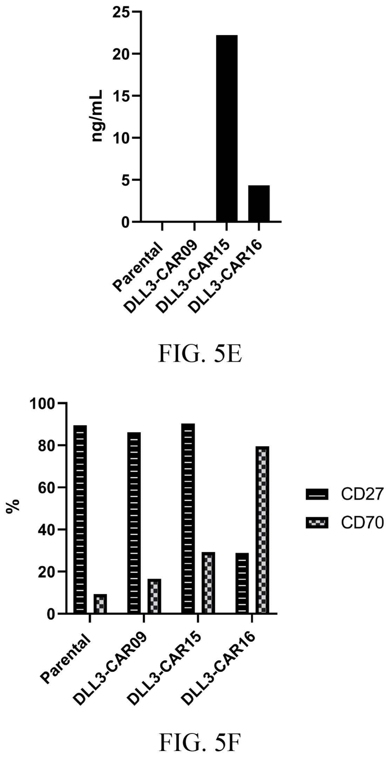

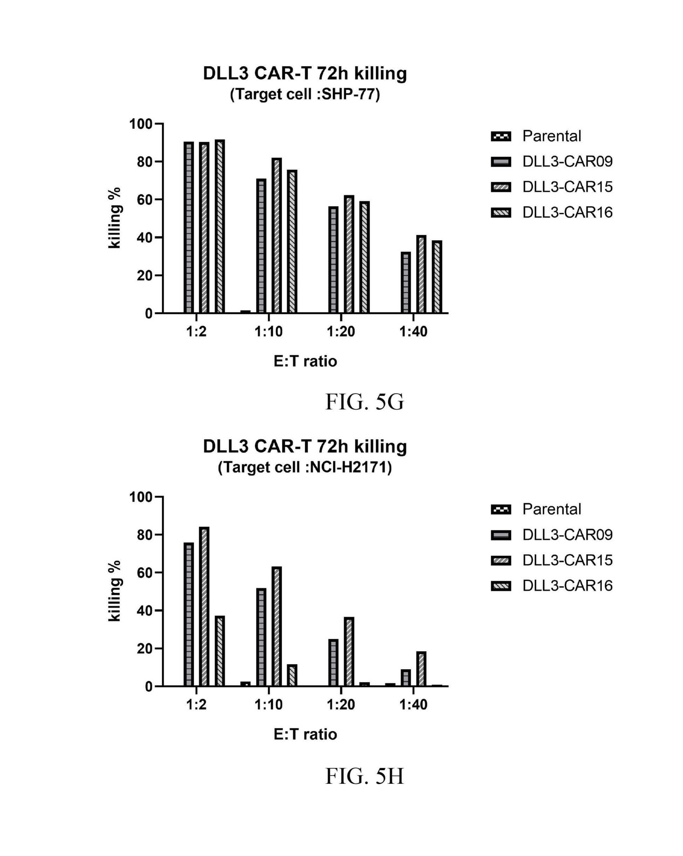

FIGs. 5A-5H show the results of T cell characterization after DLL3 CAR-T cells, sCD70-armored DLL3 CAR-T cells and membrane-bound CD70-armored DLL3 CAR-T cells were transfected and expanded in vitro for one week. FIGs. 5A-5D show the expression level of DLL3 CAR, CD4/CD8 ratio, and the expression level of T cell exhaustion phenotype and T cell memory phenotype after in vitro CAR-T expansion. FIG. 5E shows results of CD70 secretion by DLL3 CAR-T. During in vitro culture, the supernatant of the culture medium (5-day culture) of CAR-T cells reaching a certain density (2E6/ml) was tested by ELISA for CD70 protein. FIG. 5F shows the expression results of CD70 and CD27 on the surface of DLL3 CAR-T cells. FIG. 5G and FIG. 5H show the evaluation results of the in vitro tumor killing function of non-armored, sCD70-armored and membrane-bound CD70-armored DLL3 CAR-T cells against SHP-77 and NCI-H2171 cells for 72 hours, with the effector-target ratio (E:T) being 1:2, 1:10, 1:20 and 1:40, respectively.

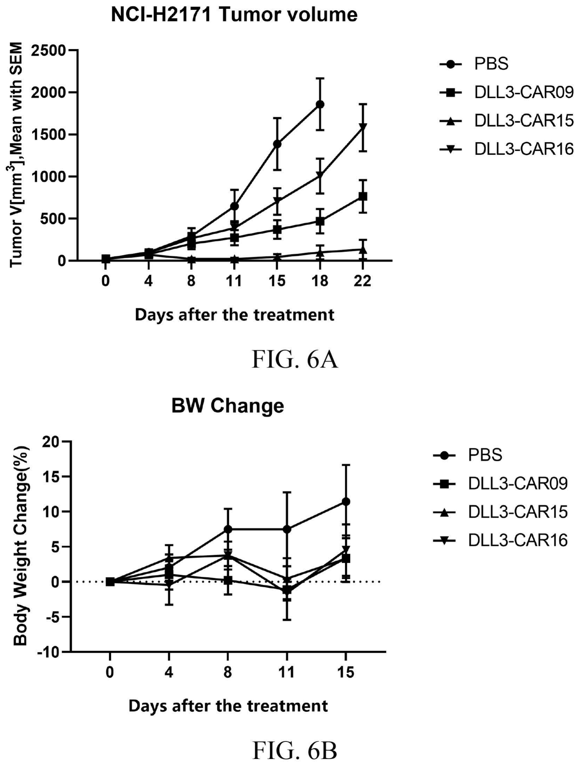

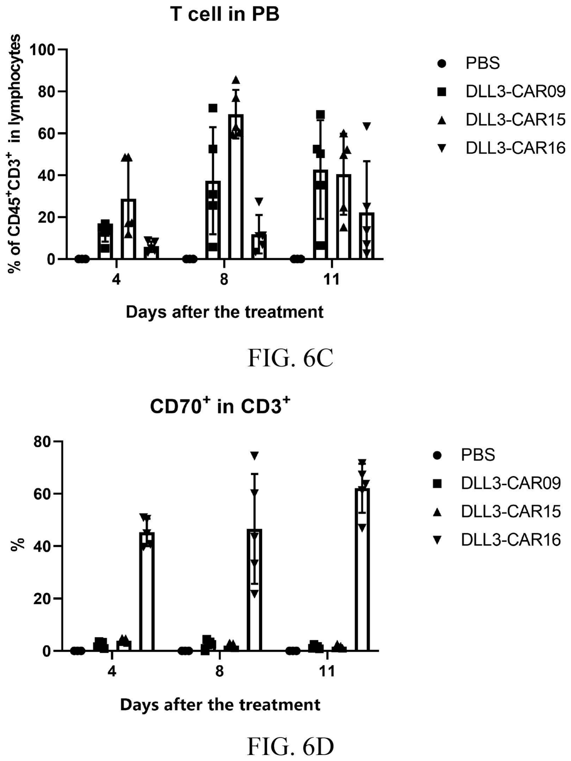

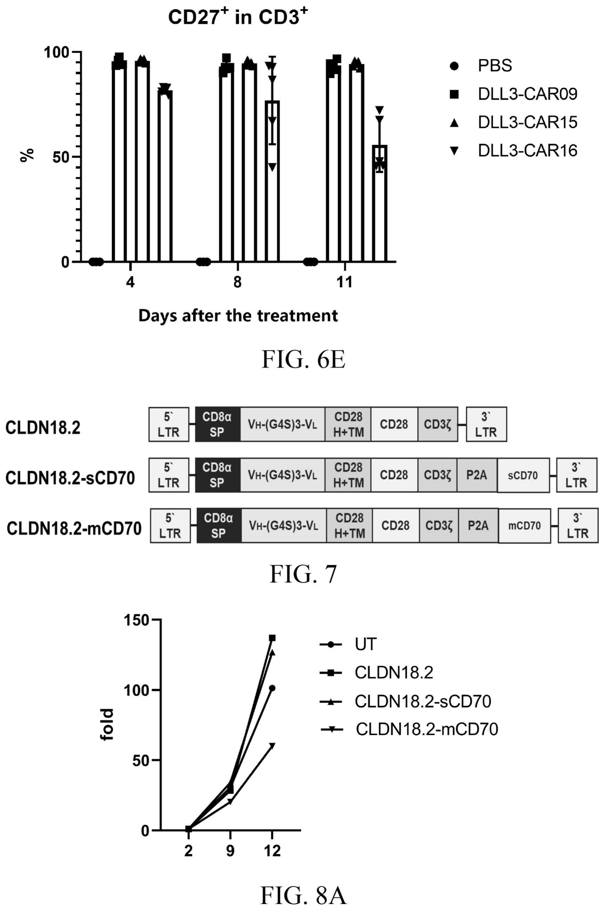

FIGs. 6A-6E show detection of the anti-tumor efficacy of DLL3 CAR-T CELLS, sCD70-armored DLL3 CAR-T cells and membrane-bound CD70-armored DLL3 CAR-T cells against mouse subcutaneous tumor model, wherein FIG. 6A shows a tumor growth curve of the NCI-H2171 NPG mouse subcutaneous tumor model; FIG.6B shows a weight change curve of the NCI-H2171 NPG mouse subcutaneous tumor model; FIG. 6C shows the peripheral blood T cell detection results of the NCI-H2171 NPG mouse subcutaneous tumor model; FIG. 6D and FIG. 6E show the detection results of CD70 and CD27 expression on the surface of mouse peripheral blood T cells.
FIG. 7 shows diagrams of plasmid structure of Claudin 18.2 (CLDN18.2) and armored CAR-T.

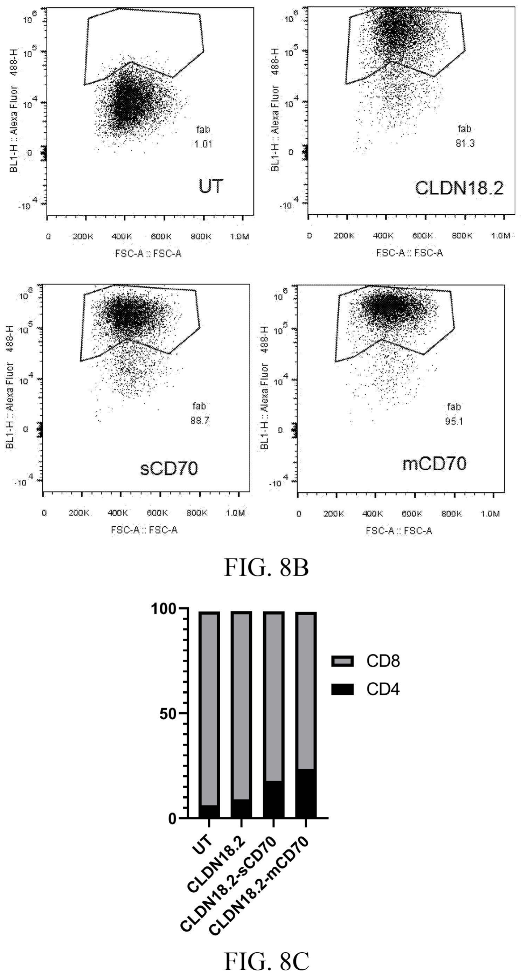

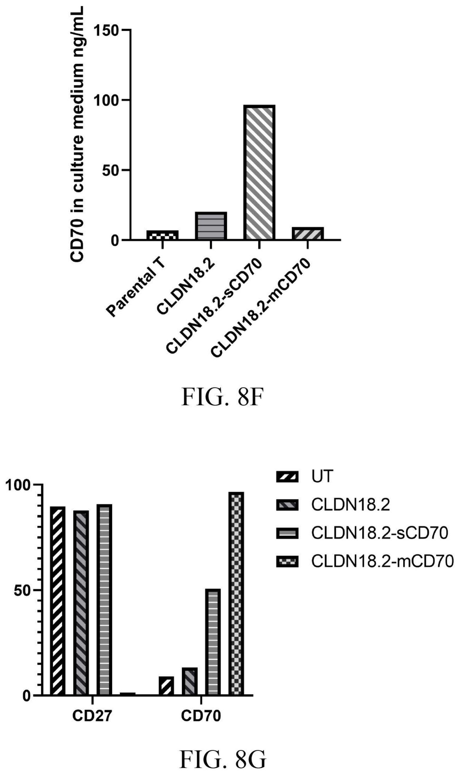

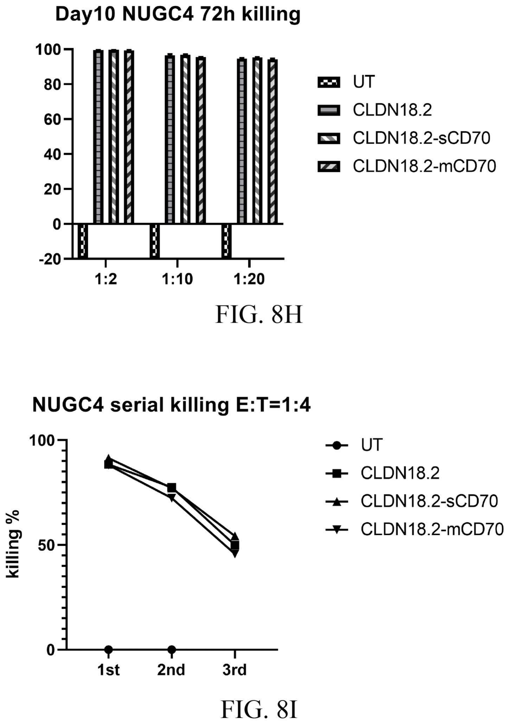

FIGs. 8A-8J show results of T cell characterization of CLDN18.2 CAR-T cells, sCD70-armored and membrane-bound CD70-armored CLDN18.2 CAR-T cells after one week of in vitro expansion. FIGs. 8A shows the results of T cell expansion fold. FIGs. 8B-8E show the expression level of CAR, CD4/CD8 ratio, and the expression level of T cell exhaustion phenotype and T cell memory phenotype after in vitro CAR-T expansion. FIG. 8F shows results of CD70 secretion by sCD70-armored CLDN18.2 CAR-T. During in vitro culture, the supernatant of the culture medium (5-day culture) of CAR-T cells reaching a certain density (3-5E6/ml) was tested by ELISA for CD70 protein. FIG. 8G shows the detection results of CD70 and CD27 expression on the surface of CLDN18.2 CAR-T cells. FIG. 8H-8J show the evaluation results of the in vitro tumor killing function of non-armored, sCD70-armored and membrane-bound CD70-armored CLDN18.2 CAR-T cells against NUGC4 cells (with the E:T ratio being 1:2, 1:10, 1:20) and NUGC4-luc cells (E:T=1:4) and SNU620 cells (E:T=1:2) for 72 hours. "UT" or "Parental T" represents the untransfected cell group.

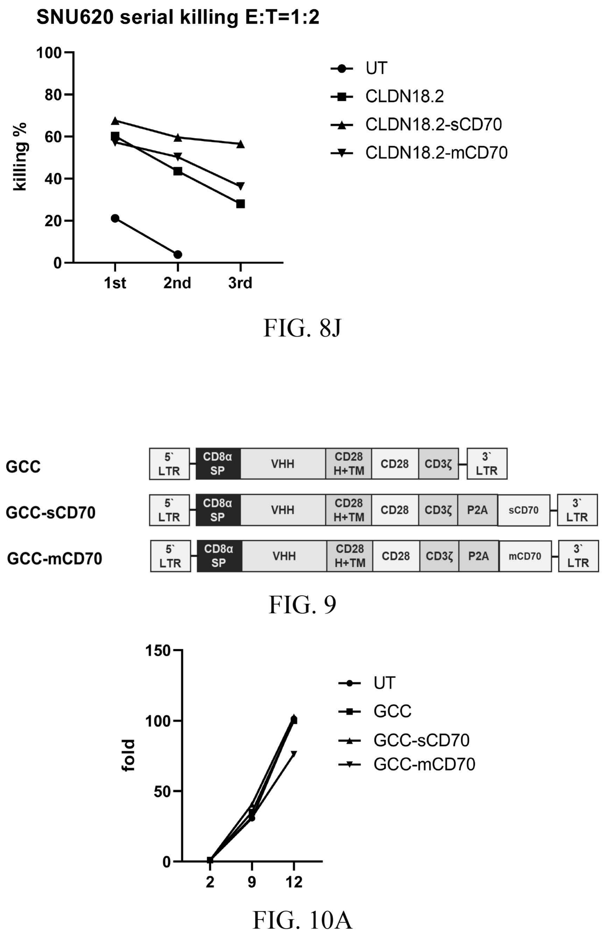

FIG. 9 shows diagrams of plasmid structure of GUCY2C (GCC) CAR-T and armored CAR-T.

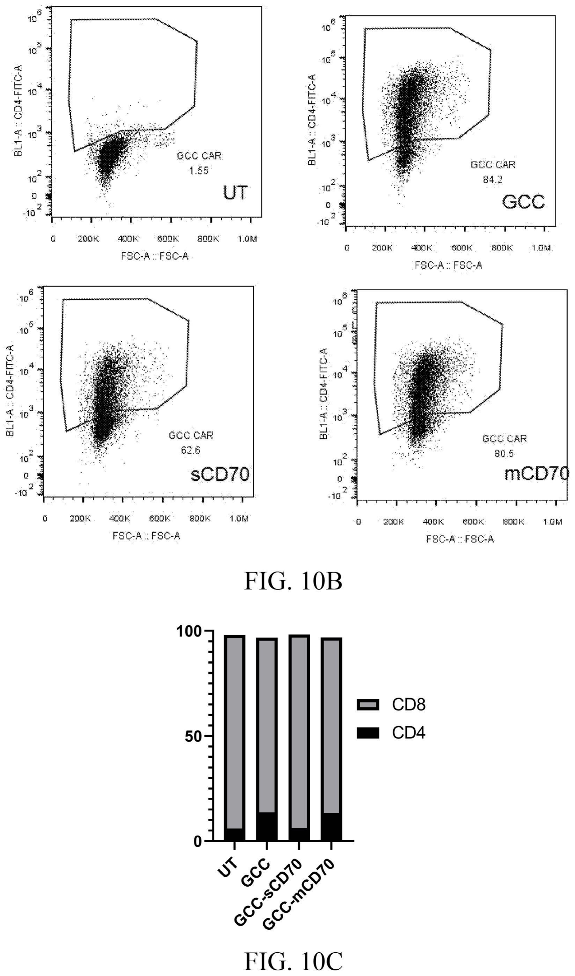

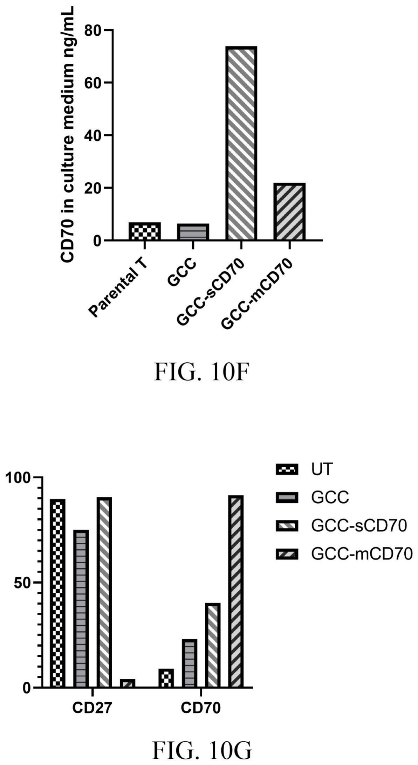

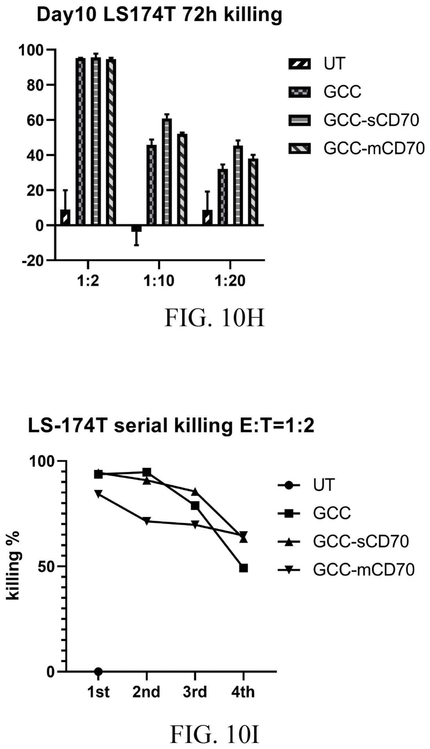

FIGs. 10A-10I show results of T cell characterization of GCC CAR-T cells, sCD70-armored and membrane-bound CD70-armored GCC CAR-T cells after one week of in vitro expansion. FIGs. 10A shows the results of T cell expansion fold. FIGs. 10B-10E show the expression level of CAR, CD4/CD8 ratio, and the expression level of T cell exhaustion phenotype and T cell memory phenotype after in vitro CAR-T expansion. FIG. 10F shows results of CD70 secretion by sCD70-armored GCC CAR-T. During in vitro culture, the supernatant of the culture medium (5-day culture) of CAR-T cells reaching a certain density (3-5E6/ml) was tested by ELISA for CD70 protein. FIG. 10G shows the detection results of CD70 and CD27 expression on the surface of GCC CAR-T cells. FIG. 10H and FIG. 10I show the evaluation results of the in vitro tumor killing function (with the E:T ratio being 1:2, 1:10, 1:20) and serial killing (E:T=1:2) of non-armored, sCD70-armored and membrane-bound CD70-armored GCC CAR-T cells against LS174T-luc cells. "UT" or "Parental T" represents the untransfected cell group.

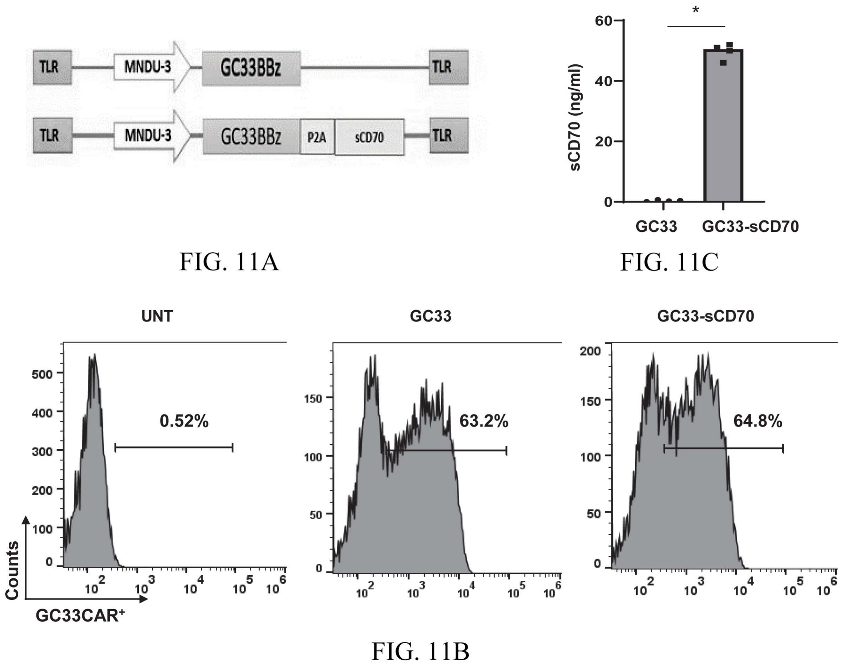

FIGs. 11A-11C show sCD70-armored anti-Glypican 3 (GPC-3; clone: GC33) CAR-T cells. FIG. 11A shows diagrams of the parental GC33 CAR construct (top) and sCD70-armored GC33 (GC33-sCD70) CAR construct (bottom). FIG. 11B shows GC33-CAR expression on Lentiviral (LV)-GC33 or LV-GC33-sCD70 transduced T cells, analyzed via immune staining/fluorescence-activated cell sorting (FACS). FIG. 11C quantifies soluble CD70 (sCD70) secreted from LV-GC33-sCD70-CAR transduced T cells (3.3x106 T cells/ml), analyzed via the enzyme-linked immunosorbent assay (ELISA) of the T-cell culture media. Data are presented as the mean ± s.d. *p<0.001. A two tailed, unpaired two sample t test was used for statistical analysis. Experiment was repeated twice using different donor-derived T cells.

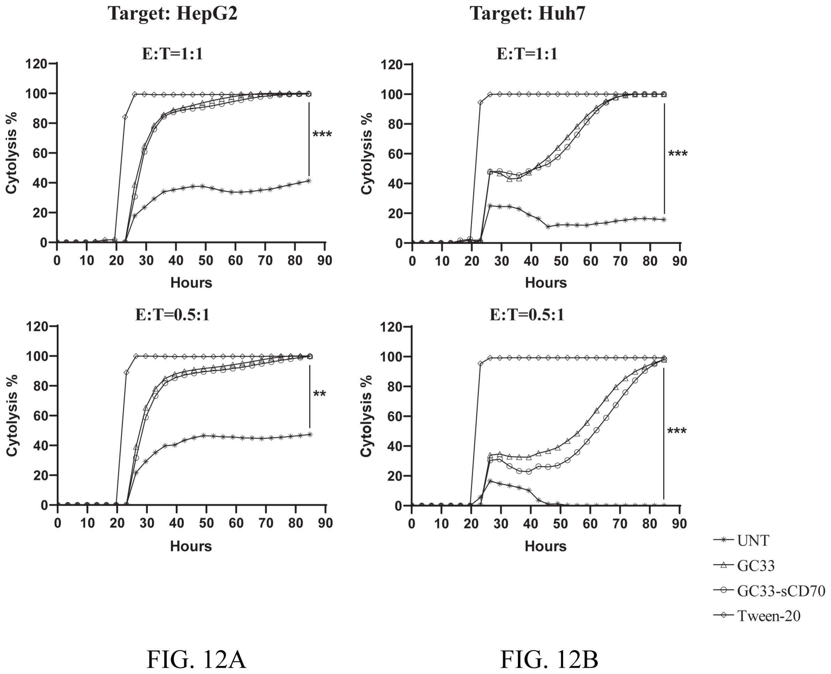

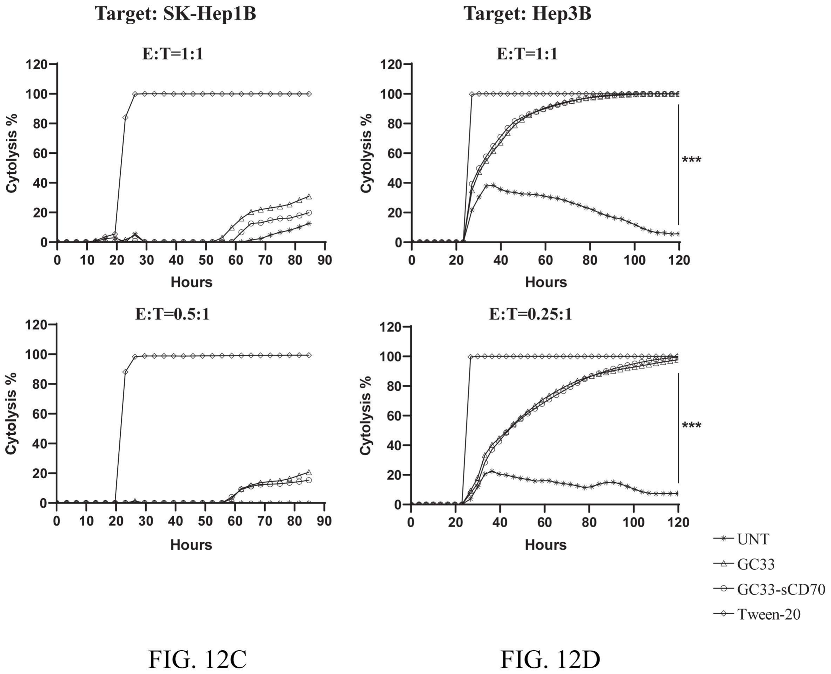

FIGs. 12A-12D cytotoxicity of GC33 CAR-T and GC33-sCD70 CAR-T against hepatocellular carcinoma cell lines HepG3 (FIG. 12A), Huh7 (FIG. 12B), SK-Hep1B (FIG. 12C), and Hep3B (FIG. 12D) at the indicated E/T ratios. Shown are results of real-time cytotoxicity analysis (RTCA) within about 90 hours or 120 hours post tumor cell seeding. Data are presented as the mean. **p<0.01, ***p<0.001; GC33 or GC33-sCD70 vs. untransduced T cells (UNT). A two tailed, unpaired two sample t test was used for statistical analysis. Experiment was repeated more than five times using different donor-derived T cells.

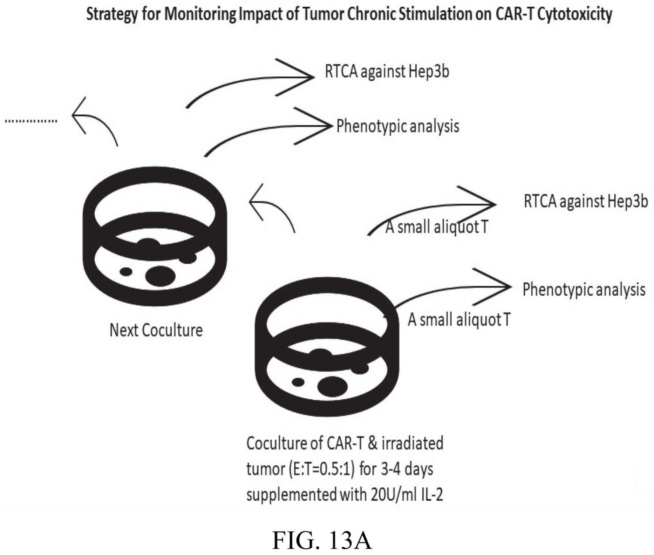

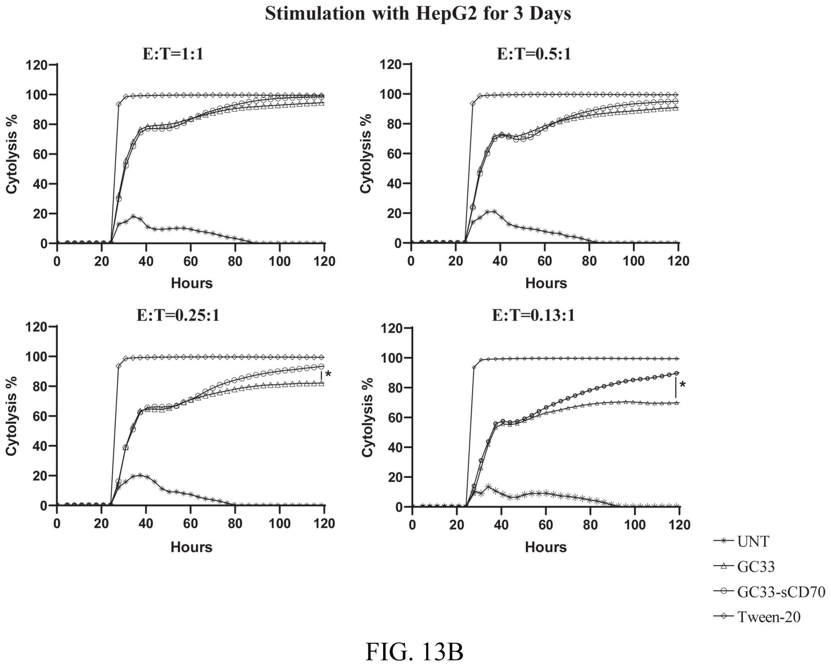

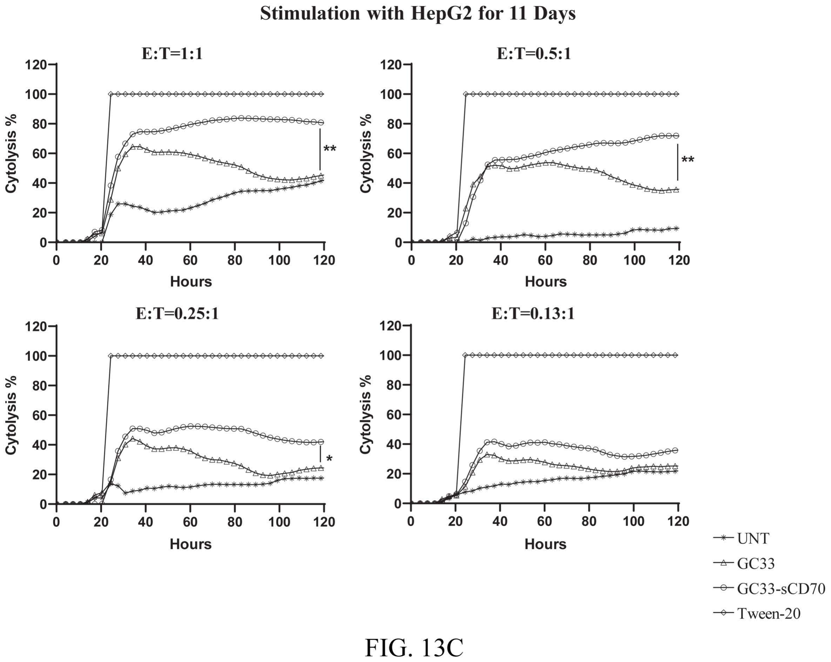

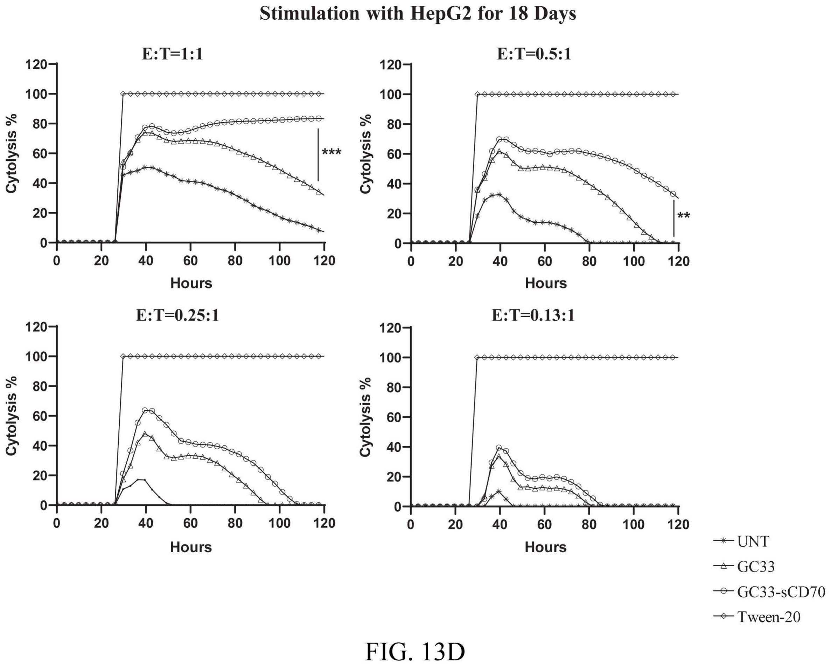

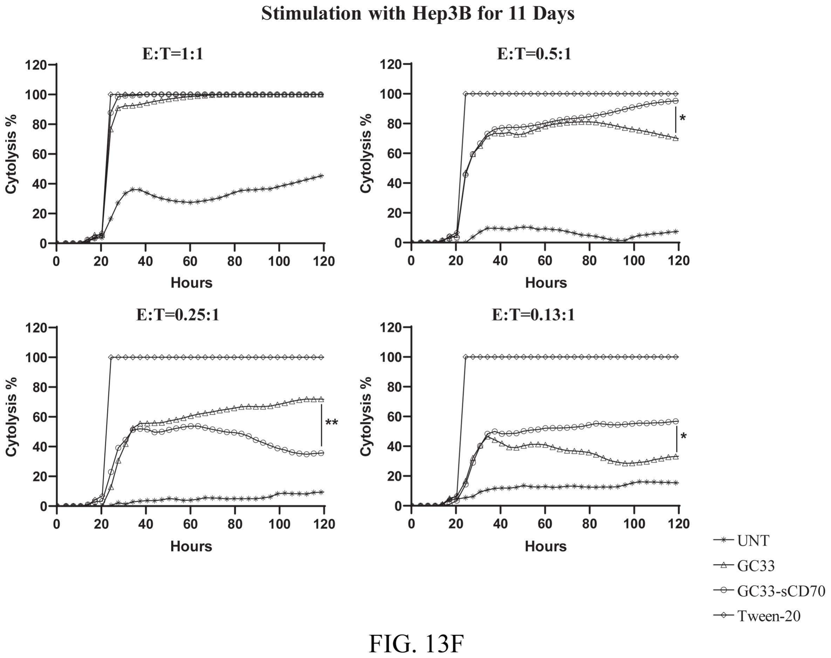

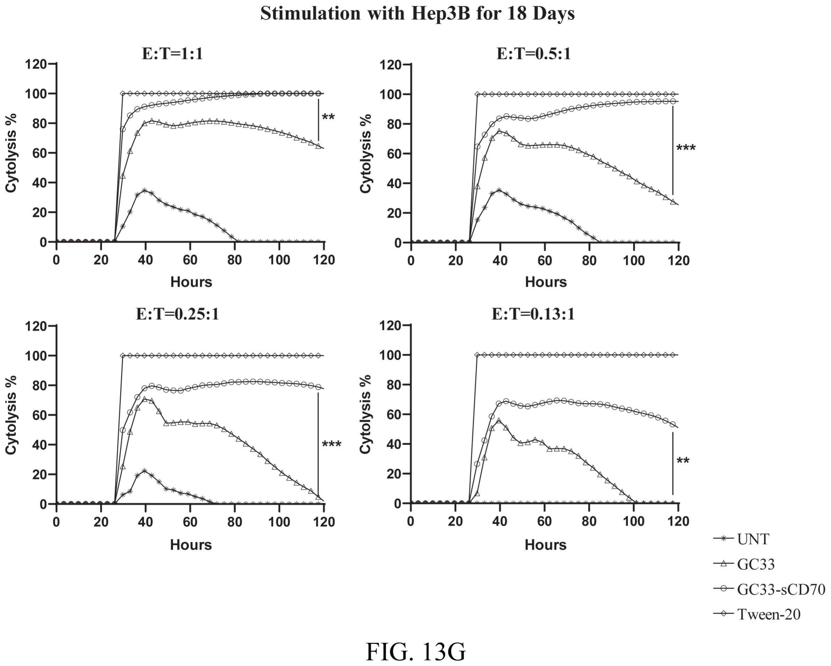

FIGs. 13A-13G GC33-sCD70 CAR-T resisted tumor chronic stimulation, retaining potent and durable cytotoxicity. FIG. 13A depicts the strategy for monitoring impact of tumor chronic stimulation on CAR-T cytotoxicity. FIGs. 13B-13G evaluate GC33 CAR-T and GC33-sCD70 CAR-T cytotoxicity against Hep3B after exposed to continuous tumor antigen stimulation by co-culture with irradiated HCC line HepG2 or Hep3B for 3 days, 11 days, or 18 days. Shown are results of RTCA of the CAR-T cells against Hep3B at the indicated E/T ratios. Data are presented as the mean. *p<0.05, **p<0.01, ***p<0.001, GC33-sCD70 vs. GC33. A two tailed, unpaired two sample t test was used for statistical analysis. Experiment was repeated twice using different donor-derived T cells.

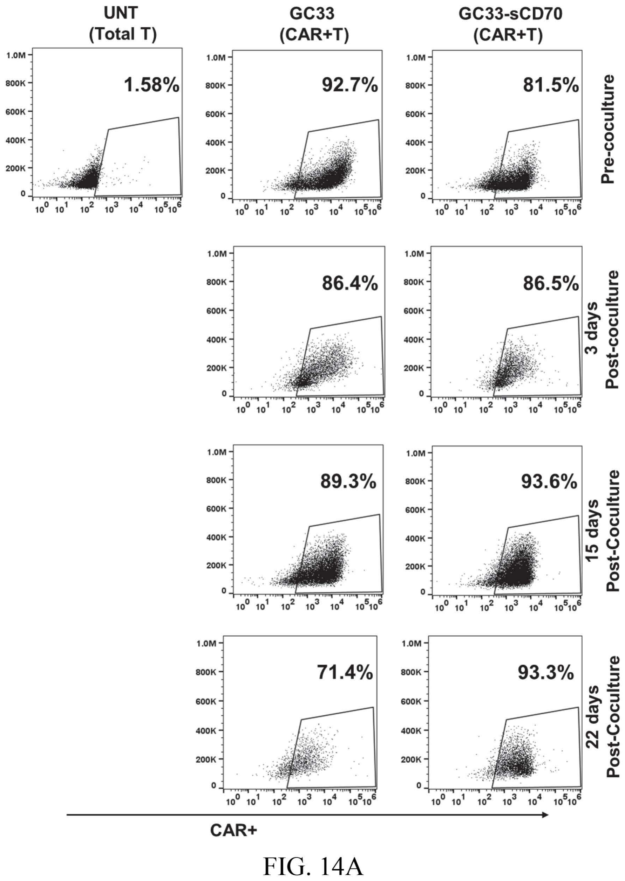

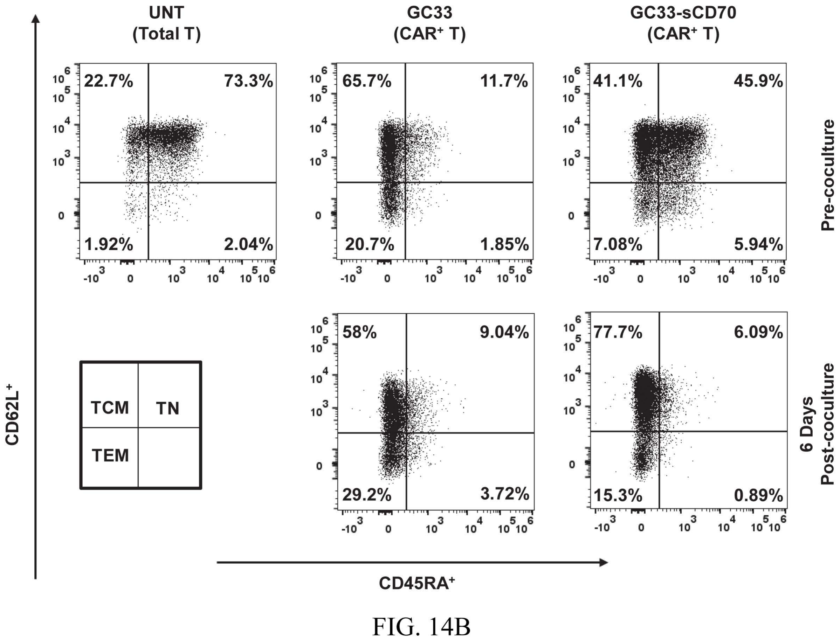

FIGs. 14A-14B GC33-sCD70 CAR-T resisted tumor chronic stimulation, retaining CAR expression and a central memory phenotype. FIG. 14A GC33-sCD70 or GC33 CAR-T cells were repetitively exposed to GPC-3 by coculturing with irradiated Hep3B cells for continuous antigen stimulation. Shown are results of GC33 CAR+ T cell percentage prior to the coculture or after 3, 15, or 22 days of coculture with irradiated tumor cells. FIG. 14B GC33-sCD70 or GC33 CAR-T cells were repetitively exposed to GPC-3 by coculturing with irradiated Hep3B cells for continuous antigen stimulation. Shown are results of phenotypic analyses of the CAR-T cells with naive phenotype (GC33CAR+CD45RA+CCD62+) or with central memory phenotype (GC33CAR+CD45RA-CD62+) prior to the coculture or after 6 days of coculture with irradiated tumor cells. UNT: untransduced T cells.

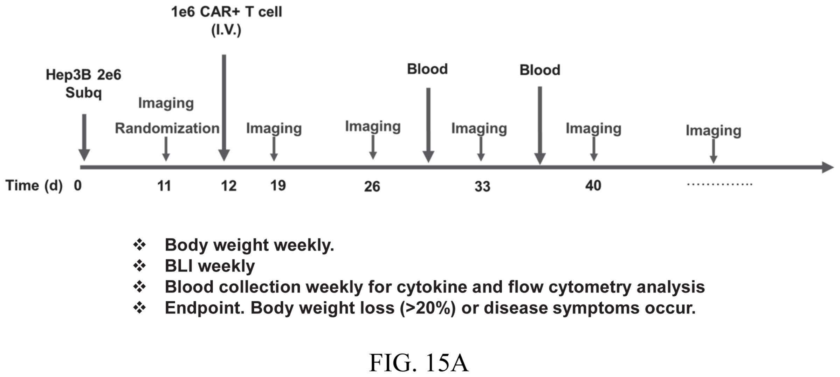

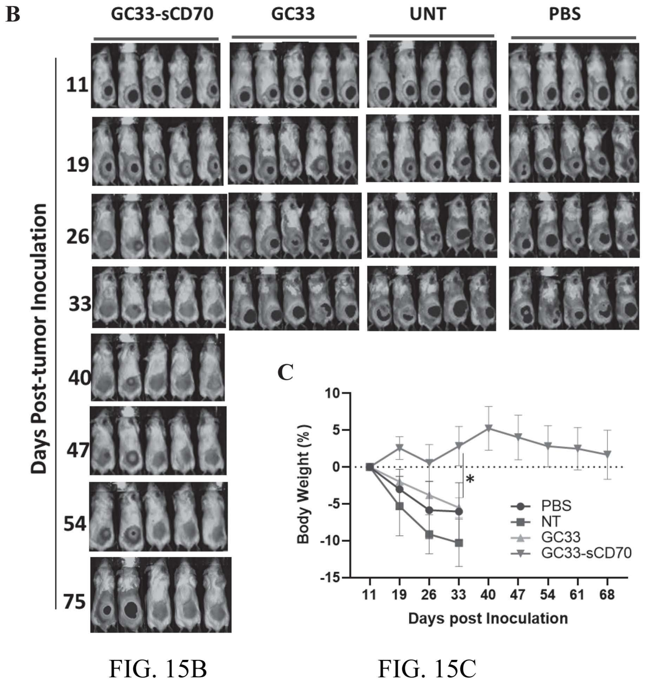

FIGs. 15A-15C GC33-sCD70 CAR-T cells exhibited more sustained anti-tumor activity in a xenografted NSG mouse model. FIG. 15A depicts the scheme for monitoring antitumor activities of CAR-T cells in the xenograft NSG mice. FIG. 15B Bioluminescence Imaging (BLI) longitudinally monitored tumor treatment. The NSG mice were subcutaneously (s.c.) inoculated with Hep3B tumor cells (2e6) followed by infusion of the indicated CAR-T cells (1e6), untransduced T cells (UNT), or PBS. Shown are results of weekly BLI of differently treated tumor-bearing mice. FIG. 15C Body weights (BW) of the treated mice. Mice were weighted on the indicated days and the percentage of BW change was calculated with formula [BW-BW(day 11)]/BW(day 11)x100%. Data are presented as the mean ± s.d. *p<0.05, GC33-sCD70 vs. GC33. A two tailed, unpaired two sample t test was used for statistical analysis. Experiment was repeated twice using different donor-derived CAR-T cells.

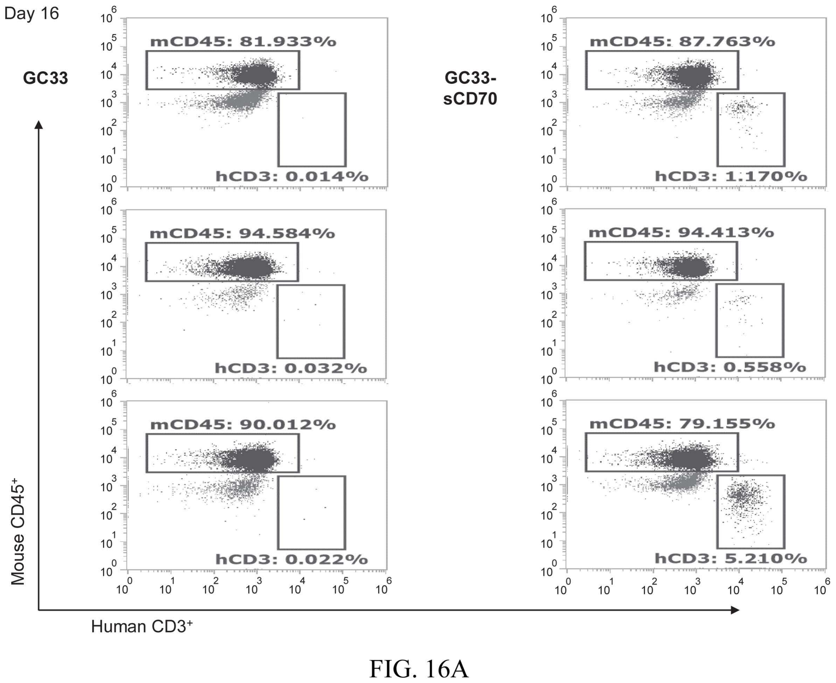

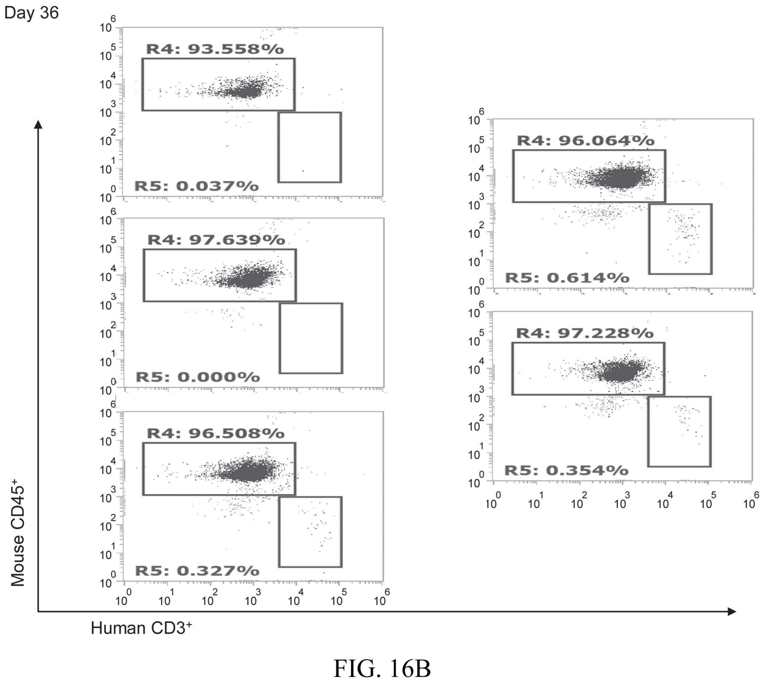

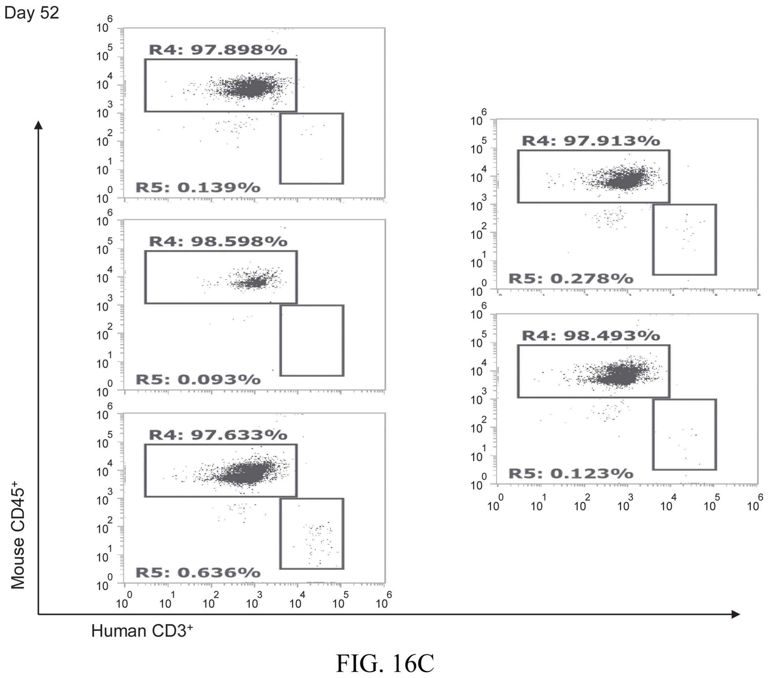

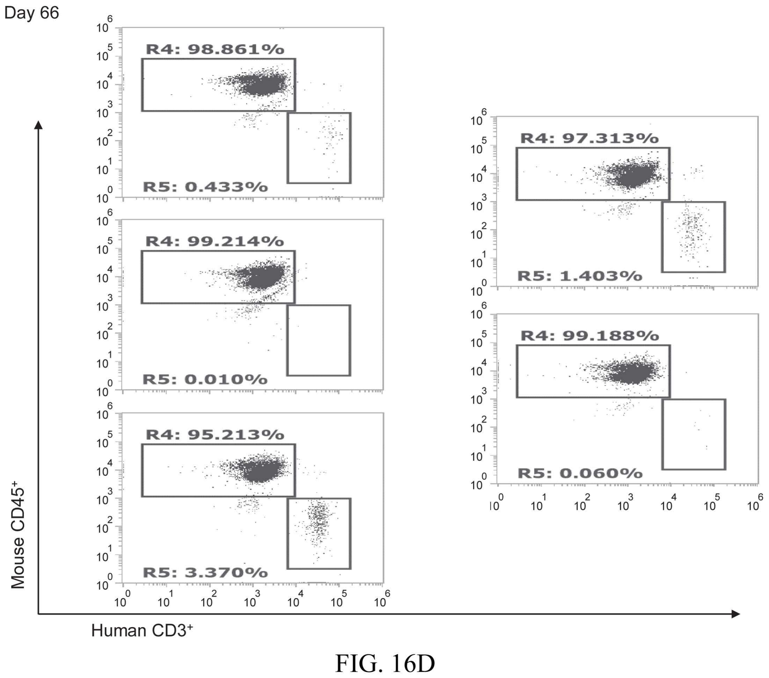

FIGs. 16A-16D GC33-sCD70 CAR-T cells showed enhanced persistence in the treated NSG mice. FIG. 16A GC33-sCD70 CAR-T detected in the blood of treated NSG. Tumor-bearing NSG mice were intravenously (i.v.) injected with indicated CAR+ T cells (1e6), UNT, or PBS. Three mice were randomly selected for bleeding at Day 16 post-CAR-T infusion, and leukocytes were analyzed by anti-mCD45 and anti-hCD3 via immune staining. Shown are the percentage of mCD45-hCD3+ T cells gated via FACS from GC33-sCD70 or GC33 CAR-T cell treated group (n=3). FIGs. 16B-16D GC33-sCD70 CAR-T cell treated mice longitudinally detected with human CD3+ T cells in the peripheral blood. GC33-sCD70 CAR-T cell treated mice were bled on Day 36, 52, or 66 post-tumor inoculation, and leukocytes were analyzed via immune staining/FACS. Shown are the percentage of mCD45-hCD3+ T cells gated via FACS (n=5). Experiment was repeated three times using different donor-derived CAR-T cells.

### TERMINOLOGY AND DEFINITIONS

A description of example embodiments follows.

Unless otherwise defined, all terms of art, notations and other scientific terms or terminology used herein are intended to have the meanings commonly understood by those of skill in the art to which this disclosure pertains. In some cases, terms with commonly understood meanings are defined herein for clarity and/or for ready reference, and the inclusion of such definitions herein should not necessarily be construed to represent a substantial difference over what is generally understood in the art. It will be further understood that terms, such as those defined in commonly used dictionaries, should be interpreted as having a meaning that is consistent with their meaning in the context of the relevant art and/or as otherwise defined herein. Also, unless otherwise defined herein, terms used in the singular form herein shall include the plural form, and vice versa. More specifically, as used in this specification and the appended claims, unless otherwise clearly indicated, the singular forms "a", "an", and "the" include referents in the plural form.

Certain terms used herein are for the purpose of describing particular embodiments only and are not intended to be limiting.

As used herein, the term "a," "an," or "the" should be understood to include plural reference unless the context clearly indicates otherwise.

As used herein, unless the context requires otherwise, the term "comprise,"will be understood to imply the inclusion of, e.g., a stated integer or step or group of integers or steps, but not the exclusion of any other integer or step or group of integer or step. As used herein, the term "comprising" can be substituted with the term "containing" or "including".

As used herein, "consisting of" excludes any element, step, or ingredient not specified in the claim element. As used herein, "consisting essentially of" does not exclude materials or steps that do not materially affect the basic and novel characteristics of the claim. Any of the terms "comprising," "containing," "including," and "having," whenever used herein in the context of an aspect or embodiment of the disclosure, can in some embodiments, be replaced with the term "consisting of," or "consisting essentially of" to vary scopes of the disclosure.

As used herein, the conjunctive term "and/or" between multiple recited elements is understood as encompassing both individual and combined options. For instance, where two elements are conjoined by "and/or," a first option refers to the applicability of the first element without the second. A second option refers to the applicability of the second element without the first. A third option refers to the applicability of the first and second elements together. Any one of these options is understood to fall within the meaning, and, therefore, satisfy the requirement of the term "and/or" as used herein. Concurrent applicability of more than one of the options is also understood to fall within the meaning, and, therefore, satisfy the requirement of the term "and/or."

When a list is presented, unless stated otherwise, it is to be understood that each individual element of that list, and every combination of that list, is a separate embodiment. For example, a list of embodiments presented as "A, B, or C" is to be interpreted as including the embodiments, "A," "B," "C," "A or B," "A or C," "B or C," or "A, B, or C."

Unless otherwise indicated or otherwise evident from the context and/or understanding of one of ordinary skill in the art, values herein that are expressed as ranges can assume any specific value or sub-range within the stated ranges in different embodiments disclosed herein, to the tenth of the unit of the lower limit of the range, unless the context clearly dictates otherwise.

"About" means within an acceptable error range for the particular value, as determined by one of ordinary skill in the art. Typically, an acceptable error range for a particular value depends, at least in part, on how the value is measured or determined, e.g., the limitations of the measurement system. For example, "about" can mean within an acceptable standard deviation, per the practice in the art. Alternatively, "about" can mean a range of ± 20%, e.g., ± 10%, ± 5% or ± 1% of a given value. It is to be understood that the term "about" can precede any particular value specified herein, except for particular values used in the Exemplification.

As used herein, the term "a polynucleotide" refers to a biopolymer comprising naturally occurring deoxyribonucleotide monomers, non-naturally occurring deoxyribonucleotide monomers (e.g., 7-deazaguanosine, inosine, or a methylated nucleotide such as 5-methyl dCTP or 5-hydroxymethyl cytosine), naturally occurring ribonucleotide monomers, or non-naturally occurring ribonucleotide monomers (e.g., a locked nucleic acid (LNA)), or a combination thereof. A polynucleotide described herein can be single stranded (ss) or double stranded (ds). In some embodiments, a polynucleotide is a DNA molecule. In some embodiments, a polynucleotide is an RNA molecule (e.g., a linear or a circular RNA molecule).

As used herein, the term "encoding" refers to specific sequences of nucleotides in a polynucleotide, such as a DNA (e.g., a cDNA) or an RNA (e.g., an mRNA), to serve as a template for synthesis of a protein having defined sequences of amino acids. Unless otherwise specified, a polynucleotide encoding an amino acid sequence can have any one nucleic acid sequence of all nucleic acid sequences that are degenerate versions of each other and that encode the amino acid sequence.

The term "polypeptide" "peptide" or "protein" denotes a polymer of at least two amino acids covalently linked by an amide bond, regardless of length or post-translational modification (e.g., glycosylation or phosphorylation). A protein, peptide or polypeptide can comprise any suitable Land/or D-amino acid, for example, common α-amino acids (e.g., alanine, glycine, valine), non-α-amino acids (e.g., α-alanine, 4-aminobutyric acid, 6-aminocaproic acid, sarcosine, statine), and unusual amino acids (e.g., citrulline, homocitruline, homoserine, norleucine, norvaline, ornithine). The amino, carboxyl and/or other functional groups on a peptide can be free (e.g., unmodified) or protected with a suitable protecting group. Suitable protecting groups for amino and carboxyl groups, and methods for adding or removing protecting groups are known in the art and are disclosed in, for example, Green and Wuts, "Protecting Groups in Organic Synthesis," John Wiley and Sons, 1991. The functional groups of a protein, peptide or polypeptide can also be derivatized (e.g., alkylated) or labeled (e.g., with a detectable label, such as a fluorogen or a hapten) using methods known in the art. A protein, peptide or polypeptide can comprise one or more modifications (e.g., amino acid linkers, acylation, acetylation, amidation, methylation, terminal modifiers (e.g., cyclizing modifications), N-methyl-α-amino group substitution), if desired. In addition, a protein, peptide or polypeptide can be an analog of a known and/or naturally-occurring peptide, for example, a peptide analog having conservative amino acid residue substitution(s).

As used herein, the term "chimeric antigen receptor" or "CAR" refers to an artificial immune cell receptor that is engineered to bind to an antigen present on the surface of target cells, for example, cancer cells. Immune cells (e.g., T cells) expressing CAR can redirect their specificity and reactivity toward target cells in a non-MHC-restricted manner.

As used herein, the term "antigen" refers to any substance that can be recognized by the immune system. "Antigen" broadly encompasses proteins, such as enzymes, peptides, such as polypeptides, carbohydrates, such as polysaccharides, haptens, nucleic acids such as polynucleotides, and grafts. An antigen can be a self-antigen, an antigen produced, under normal conditions or as part of a disorder, by the body, or a foreign antigen, a non-self-antigen. Examples of self-antigens include self-antigens associated with cancers.

As used herein, the term "tumor-associated antigen" or "TAA" refers to a protein or polypeptide antigen that is expressed by a cancer cell (e.g., a tumor cell). For example, a TAA may be one or more surface proteins or polypeptides, nuclear proteins or glycoproteins, or fragments thereof, of a cancer cell (e.g., a tumor cell).

Cancer cells may be cells of a solid tumor (e.g., a tumor of the breast, lung, prostate, colon, bladder, ovary, kidney, stomach, colon, rectum, testes, head and/or neck, pancreas, brain, skin) or a hematologic cancer (e.g., leukemia, lymphoma, myeloma).

Non-limiting examples of cancers include acute lymphoblastic leukemia (ALL); acute myeloid leukemia (AML); adrenocortical carcinoma; adrenocortical carcinoma, childhood; Acquired immunodeficiency syndrome (AIDS)-related cancer (e.g., Kaposi Sarcoma, AIDS-related lymphoma, primary central nervous system (CNS) lymphoma); anal cancer; appendix cancer; astrocytoma, childhood; atypical teratoid/rhabdoid tumor, childhood, CNS; basal cell carcinoma of the skin; bile duct cancer; bladder cancer; bladder cancer, childhood; bone cancer (including Ewing sarcoma, osteosarcoma and malignant fibrous histiocytoma); brain tumors/cancer; breast cancer; Burkitt lymphoma; carcinoid tumor (gastrointestinal); carcinoid tumor, childhood; cardiac (heart) tumors, childhood; embryonal tumors, childhood; germ cell tumor, childhood; primary CNS lymphoma; cervical cancer; childhood cervical cancer; cholangiocarcinoma; chordoma, childhood; chronic lymphocytic leukemia (CLL); chronic myelogenous leukemia (CML); chronic myeloproliferative neoplasms; colorectal cancer; childhood colorectal cancer; craniopharyngioma, childhood; cutaneous T-cell lymphoma (e.g., mycosis fungoides and Sézary syndrome); Ductal carcinoma in situ (DCIS); embryonal tumors, central nervous system, childhood; endometrial cancer (uterine cancer); ependymoma, childhood; esophageal cancer; childhood esophageal cancer; esthesioneuroblastoma; Ewing sarcoma; extracranial germ cell tumor, childhood; extragonadal germ cell tumor; eye (ocular) cancer; childhood intraocular melanoma; intraocular melanoma; retinoblastoma; fallopian tube cancer; fibrous histiocytoma of bone, malignant, and osteosarcoma; gallbladder cancer; gastric (stomach) cancer; childhood gastric (stomach) cancer; gastrointestinal carcinoid tumor; gastrointestinal stromal tumors (GIST); childhood gastrointestinal stromal tumors; germ cell tumors; childhood CNS germ cell tumors (e.g., childhood extracranial germ cell tumors, extragonadal germ cell tumors, ovarian germ cell tumors, testicular cancer); gestational trophoblastic disease; hairy cell leukemia; head and neck cancer; heart tumors, childhood; hepatocellular (liver) cancer; histiocytosis, Langerhans cell; Hodgkin lymphoma; hypopharyngeal cancer; intraocular melanoma; childhood intraocular melanoma; islet cell tumors, pancreatic neuroendocrine tumors; Kaposi sarcoma; kidney (renal cell) cancer; Langerhans cell histiocytosis; laryngeal cancer; leukemia; lip and oral cavity cancer; liver cancer; lung cancer (non-small cell and small cell); childhood lung cancer; lymphoma; male breast cancer; malignant fibrous histiocytoma of bone and osteosarcoma; melanoma; childhood melanoma; melanoma, intraocular (eye); childhood intraocular melanoma; Merkel cell carcinoma; mesothelioma, malignant; childhood mesothelioma; metastatic cancer; metastatic squamous neck cancer with occult primary; midline tract carcinoma with NUT gene changes; mouth cancer; multiple endocrine neoplasia syndromes; multiple myeloma/plasma cell neoplasms; mycosis fungoides; myelodysplastic syndromes, myelodysplastic/myeloproliferative neoplasms; myelogenous leukemia, chronic (CML); myeloid leukemia, acute (AML); myeloproliferative neoplasms, chronic; nasal cavity and paranasal sinus cancer; nasopharyngeal cancer; neuroblastoma; non-Hodgkin lymphoma; non-small cell lung cancer; oral cancer, lip and oral cavity cancer and oropharyngeal cancer; osteosarcoma and malignant fibrous histiocytoma of bone; ovarian cancer; childhood ovarian cancer; pancreatic cancer; childhood pancreatic cancer; pancreatic neuroendocrine tumors; papillomatosis (childhood laryngeal); paraganglioma; childhood paraganglioma; paranasal sinus and nasal cavity cancer; parathyroid cancer; penile cancer; pharyngeal cancer; pheochromocytoma; childhood pheochromocytoma; pituitary tumor; plasma cell neoplasm/multiple myeloma; pleuropulmonary blastoma; pregnancy and breast cancer; primary peritoneal cancer; prostate cancer; rectal cancer; recurrent cancer; renal cell (kidney) cancer; retinoblastoma; rhabdomyosarcoma, childhood; salivary gland cancer; sarcoma (e.g., childhood rhabdomyosarcoma, childhood vascular tumors, Ewing sarcoma, Kaposi sarcoma, osteosarcoma (bone cancer), soft tissue sarcoma, uterine sarcoma); Sézary syndrome; skin cancer; childhood skin cancer; small cell lung cancer; small intestine cancer; soft tissue sarcoma; squamous cell carcinoma of the skin; squamous neck cancer with occult primary, metastatic; T-cell lymphoma, cutaneous (e.g., mycosis fungoides and Sèzary syndrome); testicular cancer; childhood testicular cancer; throat cancer (e.g., nasopharyngeal cancer, oropharyngeal cancer, hypopharyngeal cancer); thymoma and thymic carcinoma; thyroid cancer; transitional cell cancer of the renal pelvis and ureter; ureter and renal pelvis, transitional cell cancer; urethral cancer; uterine cancer, endometrial; uterine sarcoma; vaginal cancer; childhood vaginal cancer; vascular tumors; vulvar cancer; and Wilms tumor and other childhood kidney tumors.

Examples of TAAs include, but are not limited to, colon cancer antigen 19.9; a gastric cancer mucin; antigen 4.2; glycoprotein A33 (gpA33); ADAM-9; gastric cancer antigen AH6; ALCAM; malignant human lymphocyte antigen APO-1; cancer antigen B1; B7 H3; beta-catenin; blood group ALeb/Ley; Burkitt's lymphoma antigen-38.13, colonic adenocarcinoma antigen C14; ovarian carcinoma antigen CA125; Carboxypeptidase M; CD5; CD19; CD20; CD22; CD23; CD25; CD27; CD30; CD33; CD36; CD45; CD46; CD52; CD79a/CD79b; CD103; CD317; CDK4; carcinoembryonic antigen (CEA); CEACAM5; CEACAM6; C017-iA; CO-43 (blood group Leb); CO-514 (blood group Lea); CTA-1; CTLA4; Cytokeratin 8; antigen D1.1; antigen D 156-22; DR5; Ei series (blood group B); EGFR (Epidermal Growth Factor Receptor); Ephrin receptor A2 (EphA2); ErbB1; ErbB3; ErbB4; GAGE-1; GAGE-2; GD2/GD3/GM2; lung adenocarcinoma antigen F3; antigen FC10.2; G49, ganglioside GD2; ganglioside GD3; ganglioside GM2; ganglioside GM3; GD2; GD3; GICA 19-9; GM2; gpOO; glypican-3 (GPC3); human leukemia T cell antigen Gp37; melanoma antigen gp75; gpA33; HER2 antigen (e.g., pi85 HER2); human milk fat globule antigen (HMFG); human papillomavirus E6/human papillomavirus-E7; high molecular weight melanoma antigen (HMW MAA); I antigen (differentiation antigen) I(Ma); Integrin Alpha-V-Beta-6 IntegrinP6 (ITGB6); Interleukin-13; Receptor a2 (IL13Rα2); JAM-3; KID3; KID31; KS 1/4 pan carcinoma antigen; human lung carcinoma antigens L6 and L20; LEA; LUCA-2; Mi:22:25:8; M18; M39; MAGE-1; MAGE-3; MART; MUC-1; MUM-1; Myl; N acetylglucosaminyltransferase; neoglycoprotein; NS-10; OFA-1; OFA-2; Oncostatin M; p15; melanoma-associated antigen p97; polymorphic epithelial mucin (PEM); polymorphic epithelial mucin antigen (PEMA); PIPA; prostate-specific antigen (PSA); prostate-specific membrane antigen (PSMA); prostatic acid phosphate; R2 4; RORi; sphingolipids; SSEA-1; SSEA-3; SSEA-4; sTn; T cell receptor derived peptide; T 5A7 ; TAG-72; TL5 (blood group A); TNF-a receptor; TNF-B receptor; TNF-y receptor; TRA-1-85 (blood group H); Transferrin Receptor; tumor-specific transplantation antigen (TSTA), oncofetal antigen-alpha-fetoprotein (AFP); VEGF; VEGFR, VEP8; VEP9; VIM-D5; and Y hapten, Ley. In some embodiments, TAA is CEA, GPC3, MUC-1, EpCAM, HER receptors, PEM, Caludi 6, Cluadi-18.2, mesothelin, A33, G250, carbohydrate antigens Ley, Lex, Leb, PSMA, TAG-72, STEAP1, CD166, CD24, CD44, E-cadherin, SPARC, ErbB2, ErbB3, MUC1, LMP2, idiotype, HPV E6&E7, EGFR, EGFRvIII, HER-2/neu, MAGE A3, NY-ESO-1, GD2, PSMA, PCSA, PSA, MelanA/MART1, CD19, CD20, CD22, CD33, CD5, CD70, or BCMA. In some embodiments, the TAA is on a cancer cell that is not a tumor cell. In other embodiments, the TAA is on a tumor cell.

As used herein, the term "antigen-binding fragment" refers to a portion of an immunoglobulin molecule (e.g., antibody) that retains the antigen binding properties of the parental immunoglobulin molecule (e.g., full-length antibody). Non-limiting examples of antigen-binding fragments include an antibody heavy chain variable region (VH region), an antibody light chain variable region (VL region), an Fab fragment, an Fab' fragment, an F(ab')2 fragment, an Fd fragment, an Fv fragment, disulfide-linked Fvs (sdFv, e.g., diabody, triabody or tetrabody), a single-chain variable fragment (scFv, e.g., comprising an antibody heavy chain variable region (VH region) and an antibody light chain variable region (VL region) in either orientation), a domain antibody (dAb) consisting of one VH domain or one VL domain, a small modular immunopharmaceutical (SMIP) and rlgG.

As used herein, the term "transmembrane domain" refers to a protein structure that is thermodynamically stable in a cell membrane, preferably in a eukaryotic cell membrane.

As used herein, the term "sequence identity" refers to the extent to which two nucleotide sequences, or two amino acid sequences, have the same residues at the same positions when the sequences are aligned to achieve a maximal level of identity, expressed as a percentage. For sequence alignment and comparison, typically one sequence is designated as a reference sequence, to which a test sequences are compared. The sequence identity between reference and test sequences is expressed as the percentage of positions across the entire length of the reference sequence where the reference and test sequences share the same nucleotide or amino acid upon alignment of the reference and test sequences to achieve a maximal level of identity. As an example, two sequences are considered to have 70% sequence identity when, upon alignment to achieve a maximal level of identity, the test sequence has the same nucleotide or amino acid residue at 70% of the same positions over the entire length of the reference sequence.

Alignment of sequences for comparison to achieve maximal levels of identity can be readily performed by a person of ordinary skill in the art using an appropriate alignment method or algorithm. In some instances, the alignment can include introduced gaps to provide for the maximal level of identity. Examples include the local homology algorithm of Smith & Waterman, Adv. Appl. Math. 2:482 (1981), the homology alignment algorithm of Needleman & Wunsch, J. Mol. Biol. 48:443 (1970), the search for similarity method of Pearson & Lipman, Proc. Nat'l. Acad. Sci. USA 85:2444 (1988), computerized implementations of these algorithms (GAP, BESTFIT, FASTA, and TFASTA in the Wisconsin Genetics Software Package, Genetics Computer Group, 575 Science Dr., Madison, Wis.), and visual inspection (see generally Ausubel et al., Current Protocols in Molecular Biology).

When using a sequence comparison algorithm, test and reference sequences are input into a computer, subsequent coordinates are designated, if necessary, and sequence algorithm program parameters are designated. The sequence comparison algorithm then calculates the percent sequence identity for the test sequence(s) relative to the reference sequence, based on the designated program parameters. A commonly used tool for determining percent sequence identity is Protein Basic Local Alignment Search Tool (BLASTP) available through National Center for Biotechnology Information, National Library of Medicine, of the United States National Institutes of Health. (Altschul et al., 1990).

In some embodiments, the amino acid substitutions are conservative substitutions. The term "conservative amino acid substitution(s)" or "conservative substitution(s)" refers to an amino acid substitution having a value of 0 or greater in BLOSUM62.

In some embodiments, the amino acid substitutions are highly conservative substitutions. The term "highly conservative amino acid substitution(s)" or "highly conservative substitution(s)" refers to an amino acid substitution having a value of at least 1 (e.g., at least 2) in BLOSUM62.

As used herein, the term "an immune effector cell" refers to a cell that is capable of mediating and/or modulating (e.g., enhancing) an immune response.

As used herein, the term "expression vector" refers to a replicable nucleic acid from which one or more proteins can be expressed when the expression vector is transformed into a suitable expression host cell. As used herein, the term "promoter" refers to a region of DNA to which RNA polymerase binds and initiates the transcription of a gene. As used herein, the term "operably linked" means that the nucleic acid is positioned in the recombinant polynucleotide, e.g., vector, in such a way that enables expression of the nucleic acid under control of the element (e.g., promoter) to which it is linked. As used herein, the term "selectable marker element" is an element that confers a trait suitable for artificial selection. Selectable marker elements can be negative or positive selection markers.

As used herein, the term "ex vivo" refers to methods conducted within or on cells or tissue in an artificial environment outside an organism with minimum alteration of natural conditions. As used herein, the term "in vivo" refers to a method that is conducted within living organisms in their normal, intact state. As used herein, the term "in vitro" method is conducted using components of an organism that have been isolated from its usual biological context.

As used herein, the term "fusion protein" refers to a synthetic, semi-synthetic or recombinant single protein molecule. A fusion protein can comprise all or a portion of two or more different proteins and/or polypeptides that are attached by covalent bonds (e.g., peptide bonds).

As used herein, the term "subject" or "patient" refers to a mammal (e.g., a human). In some embodiments, a subject is a mammal. In some embodiments, a subject is a mammal selected from a dog, a cat, a mouse, a rat, a hamster, a guinea pig, a horse, a pig, a sheep, a cow, a chimpanzee, a macaque, a cynomolgus, and a human. In some embodiments, a subject is a primate. In some embodiments, a subject is a human.

As used herein, the term "a therapeutically effective amount," "an effective amount" or "an effective dosage" is an amount effective, at dosages and for periods of time necessary, to achieve a desired therapeutic result (e.g., treatment, healing, inhibition or amelioration of physiological response or condition, etc.). The full therapeutic effect does not necessarily occur by administration of one dose and may occur only after administration of a series of doses. Thus, a therapeutically effective amount may be administered in one or more administrations. A therapeutically effective amount may vary according to factors such as disease state, age, sex, and weight of a mammal, mode of administration and the ability of a therapeutic, or combination of therapeutics, to elicit a desired response in an individual.

An effective amount of an agent to be administered can be determined by a clinician of ordinary skill using the guidance provided herein and other methods known in the art. Relevant factors include the given agent, the pharmaceutical formulation, the route of administration, the type of disease or disorder, the identity of the subject (e.g., age, sex, weight) or host being treated, and the like. For example, suitable dosages can be from about 0.001 mg/kg to about 100 mg/kg, from about 0.01 mg/kg to about 100 mg/kg, from about 0.01 mg/kg to about 10 mg/kg, from about 0.01 mg/kg to about 1 mg/kg body weight per treatment. Determining a dosage for a particular agent, subject and disease is well within the abilities of one of skill in the art. Preferably, a dosage does not cause or produces minimal adverse side effects.

In some embodiments, an effective amount of the CAR cells (*e.g.,* CAR-T cells) disclosed herein ranges from about 1×10⁶-1.8×10⁹ CAR⁺ cells, for example, about: 1×10⁶-9×10⁸, 3×10⁷-1×10⁸, 1×10⁸-3×10⁸, 3×10⁸-4.5×10⁸, 4.5×10⁸-6×10⁸, 6×10⁸-7.5×10⁸, 7.5×10⁸-9×10⁸ or 9×10⁸-1.8×10⁹ CAR⁺ cells, or about: 3×10⁷, 1×10⁸, 3×10⁸, 4.5×10⁸, 6×10⁸, 7.5×10⁸, 9×10⁸, or 1.8×10⁹ CAR⁺ cells.

Desired response or desired results include effects at the cellular level, tissue level, or clinical results. As such, "a therapeutically effective amount" or synonym thereto depends upon the context in which it is being applied. For example, in some embodiments, it is an amount of a composition sufficient to achieve a treatment response as compared to the response obtained without administration of the composition. In other embodiments, it is an amount that results in a beneficial or desired result in a subject as compared to a control. As defined herein, a therapeutically effective amount of a composition may be readily determined by one of ordinary skill by routine methods known in the art. Dosage regimen and route of administration may be adjusted to provide an optimum therapeutic response.

As used herein, the term "treating," or its equivalents (e.g., "treatment" or "treat"), refers to the medical management of a subject with the intent to improve, ameliorate, stabilize (i.e., not worsen), prevent or cure a disease, pathological condition, or disorder-such as the particular indications exemplified herein. This term includes active treatment (treatment directed to improve the disease, pathological condition, or disorder), causal treatment (treatment directed to the cause of the associated disease, pathological condition, or disorder), palliative treatment (treatment designed for the relief of symptoms), preventative treatment (treatment directed to minimizing or partially or completely inhibiting the development of the associated disease, pathological condition, or disorder); and supportive treatment (treatment employed to supplement another therapy). Treatment also includes diminishment of the extent of a disease or condition (e.g., a cancer such as a solid tumor); preventing spread of the disease or condition; delay or slowing the progress of the disease or condition; amelioration or palliation of the disease or condition; and remission (whether partial or total), whether detectable or undetectable. "Treatment" can also mean prolonging survival as compared to expected survival if not receiving treatment. Those in need of treatment include those already with the condition or disorder, as well as those prone to have the condition or disorder or those in which the condition or disorder is to be prevented.

As used herein, the term "ameliorating" or "palliating" a disease or condition means that the extent and/or undesirable clinical manifestations of the disease, disorder, or condition are lessened and/or time course of the progression is slowed or lengthened, as compared to the extent or time course in the absence of treatment.

As used herein, a "vector" refers to a nucleic acid molecule which may be employed to introduce a nucleic acid sequence or gene into a cell, either in vitro, ex vivo, or in vivo.

As used herein, a "host" cell refers to a cell into which a polynucleotide has been introduced by molecular biology techniques. All techniques by which a polynucleotide can be introduced into a host cell, including transfection with viral vectors, transformation with plasmid vectors, and introduction of naked DNA by electroporation, lipofection, and particle gun acceleration are contemplated herein.

### DETAILED DESCRIPTION

CAR-T therapy has achieved an impressive antitumor efficacy in treatment of hematologic cancers. The therapeutic success has not been translated into treatment of solid tumors. One of the major hurdles is that solid tumors maintain a hostile microenvironment where CAR-T cell expansion and persistence is limited, which inhibits the ability of the CAR-T cells to elicit sustained antitumor activity.

Two types of strategies have been developed to enhance CAR-T cell expansion and persistence in vivo. Optimization of in vitro expansion protocols to enrich the infusion of T cells with stem cell properties (stem-like T cells) was demonstrated to promote sustained antitumor efficacy of CAR-T cells. It was achieved by shortening in vitro expansion period (from day 9 to day 3) and/or culturing T cells with cytokines or agents able to delay T-cell differentiation. In vitro exposure of CAR-T cells to the γc cytokines such as IL-7, IL-15, and/or IL-21 that preserve a less-differentiated state of T cells and strengthen stem-like T cell development has been used as a standard protocol in manufacture of infusion products. Recent studies revealed that enrichment of the infusion product in Th17 by culturing T cells with Th17 polarizing cytokines and optimized ICOS co-stimulation had a potential to enforce CAR-T cell long-term survival.

Genetically engineering CAR-T cells to build up the intrinsic survival advantage represents another effective strategy to enhance CAR-T persistence after infused into cancer patients. While the first-generation CARs, containing only the CD3ζ chain, failed to persist in cancer patients, the addition of a costimulatory domain such as CD28 or 4-1BB in the second-generation CARs greatly enhanced T-cell persistence and antitumor effects and yielded significant tumor regressions in patients with hematologic malignancies. In addition to CD28 and 4-1BB, other costimulatory domains, including CD27, OX40, and ICOS, are also being investigated in preclinical or clinical trials, and have been reported to exhibit significant enhancement of CAR-T antitumor efficacy. Additional non-limiting examples of costimulatory domains include DAP10 and DAP12. In addition, γc cytokines IL-7, IL-15, or IL-21, when genetically engineered to be co-expressed in CAR-T cells, also increased CAR-T persistence and survival duration.

Despite the promising results, the currently available CAR-T cell therapeutics are not sufficient to induce sustained complete responses in the treatment of solid cancers. Therefore, new strategies are urgently needed to improve antitumor efficacy of CAR-T therapy.

Disclosed herein are compositions and methods for treating cancer using chimeric antigen receptor (CAR) cell therapy. In some embodiments, compositions and methods disclosed herein concern CAR cell therapies in which the transformed cells express soluble CD70 and a CAR. In various embodiments, therapies using cells transformed with the CAR constructs disclosed herein can provide robust cancer treatments with enhanced expansion and more persistent cytotoxicity against cancer cells.

### Polynucleotides

In one aspect, the disclosure provides polynucleotides encoding: (a) a chimeric antigen receptor (CAR), wherein the CAR comprises an antigen-binding domain specific for a cell surface antigen, and (b) an armoring molecule capable of activating a CD70/CD27 signaling pathway.

Although the present disclosure provides polynucleotides encoding (a) CAR and (b) molecules capable of activating the CD70/CD27 signaling pathway as a whole, combinations of polynucleotides encoding (a) and (b) are also within the scope of the present disclosure. The subject matter of the corresponding polynucleotide combinations and the vector combinations, host cells, immune effector cells, compositions (including pharmaceutical compositions), kits, and medical uses or methods derived therefrom are also applicable to the detailed description of each part below.

In some embodiments, a polynucleotide disclosed herein comprises a nucleotide sequence that is codon-optimized for a mammalian (e.g., human) cell.

In some embodiments, an armoring molecule (e.g., a molecule capable of activating a CD70/CD27 signaling pathway) described herein enhances or optimizes immune effector cell function. In some embodiments, an armoring molecule protects an immune effector cell against an environmental insult, such as an immunosuppressive cytokine or an immunosuppressive tumor microenvironment (TME) of solid tumors. In some embodiments, an armoring molecule increases persistence of an immune effector cell (e.g., a CAR-T cell) in a TME of a solid tumor (e.g., in vitro and/or in vivo). In some embodiments, an armoring molecule increases expansion of an immune effector cell (e.g., a CAR-T cell) in a TME of a solid tumor (e.g., in vitro and/or in vivo). In some embodiments, an armoring molecule prolongs antitumor activity of an immune effector cell (e.g., a CAR-T cell) in a TME of a solid tumor (e.g., in vitro and/or in vivo).

In some embodiments, an armoring molecule activates the CD70/CD27 signaling pathway by engaging CD27. In some embodiments, an armoring molecule is a soluble human CD70 or a variant thereof.

In another aspect, the disclosure provides polynucleotides, wherein the polynucleotides encode an amino acid sequence having at least about 90% sequence identity to any one of SEQ ID NO:25, 37, 44 and 63.
DLL3-CAR15 : 
MALPVTALLLPLALLLHAARPDIVMTQSPLSLPVTPGEPASISCRASQTISDYLHWYLQKP
GQSPQLLIKYASQSISGVPDRFSGSGSGSDFTLKISRVEAEDVGVYYCQQGHSFPLTFGQGT
KLEIKGGGGSGGGGSGGGGSEVQLVQSGAEVKKPGASVKVSCKASGYTFTSYWMHWV
RQAPGQRLEWMGTIYPGNSDTRYNQKFKDRVTITADTSASTAYMELSSLRSEDTAVYYCT
RSYDYGDLDYWGQGTTVTVSSTTTPAPRPPTPAPTIASQPLSLRPEACRPAAGGAVHTRGL
DFACDIYIWAPLAGTCGVLLLSLVITLYCKRGRKKLLYIFKQPFMRPVQTTQEEDGCSCRFP
EEEEGGCELRVKFSRSADAPAYQQGQNQLYNELNLGRREEYDVLDKRRGRDPEMGGKPR
RKNPQEGLYNELQKDKMAEAYSEIGMKGERRRGKGHDGLYQGLSTATKDTYDALHMQA
LPPRKRRSGSGATNFSLLKQAGDVEENPGPMALPVTALLLPLALLLHAARPQRFAQAQQQ
LPLESLGWDVAELQLNHTGPQQDPRLYWQGGPALGRSFLHGPELDKGQLRIHRDGIYMV
HIQVTLAICSSTTASRHHPTTLAVGICSPASRSISLLRLSFHQGCTIASQRLTPLARGDTLCTN
LTGTLLPSRNTDETFFGVQWVRP (SEQ ID NO:25)
CLDN18.2-sCD70 CAR: 
MALPVTALLLPLALLLHAARPQVQLVQSGAEVKKPGSSVKVSCKASGYAFSNYLIEWVK
QAPGQGLEWIGLINPGSGGTNYNEKFKGKATITADKSTSTAYMELSSLRSEDTAVYYCARV
YYGNSFAYWGQGTLVTVSSGGGGSGGGGSGGGGSDIVMTQSPDSLAVSLGERATISCKSS
QSLLNSGNQKNYLTWYQQKPGQPPKLLIYWASTRESGVPDRFSGSGSGTDFTLTISSLQAE
DVAVYYCQNDYFYPFTFGQGTKLEIKAAAIEVMYPPPYLDNEKSNGTIIHVKGKHLCPSPL
FPGPSKPFWVLVVVGGVLACYSLLVTVAFIIFWVRSKRSRLLHSDYMNMTPRRPGPTRKH
YQPYAPPRDFAAYRSRVKFSRSADAPAYQQGQNQLYNELNLGRREEYDVLDKRRGRDPE
MGGKPRRKNPQEGLYNELQKDKMAEAYSEIGMKGERRRGKGHDGLYQGLSTATKDTYD
ALHMQALPPRKRRSGSGATNFSLLKQAGDVEENPGPMALPVTALLLPLALLLHAARPQRF
AQAQQQLPLESLGWDVAELQLNHTGPQQDPRLYWQGGPALGRSFLHGPELDKGQLRIHR
DGIYMVHIQVTLAICSSTTASRHHPTTLAVGICSPASRSISLLRLSFHQGCTIASQRLTPLAR
GDTLCTNLTGTLLPSRNTDETFFGVQWVRP (SEQ ID NO: 37)
GCC CAR-sCD70 CAR: 
MALPVTALLLPLALLLHAARPEVQLVESGGGLVKPGGSLRLSCAASGRTSTIHRMGWFRQ
APGKEREFVSSISRVSSTTYYADSVKGRFTISRDNAKNSLYLQMNSLRAEDTAVYYCAASE
YEVSPGKSKYWGQGTMVTVSSAAAIEVMYPPPYLDNEKSNGTIIHVKGKHLCPSPLFPGP
SKPFWVLVVVGGVLACYSLLVTVAFIIFWVRSKRSRLLHSDYMNMTPRRPGPTRKHYQP
YAPPRDFAAYRSRVKFSRSADAPAYQQGQNQLYNELNLGRREEYDVLDKRRGRDPEMGG
KPRRKNPQEGLYNELQKDKMAEAYSEIGMKGERRRGKGHDGLYQGLSTATKDTYDALH
MQALPPRKRRSGSGATNFSLLKQAGDVEENPGPMALPVTALLLPLALLLHAARPQRFAQ
AQQQLPLESLGWDVAELQLNHTGPQQDPRLYWQGGPALGRSFLHGPELDKGQLRIHRDG
IYMVHIQVTLAICSSTTASRHHPTTLAVGICSPASRSISLLRLSFHQGCTIASQRLTPLARGDT
LCTNLTGTLLPSRNTDETFFGVQWVRP (SEQ ID NO: 44)
GC33-sCD70 CAR

MALPVTALLLPLALLLHAARPDVVMTQSPLSLPVTPGEPASISCRSSQSLVHSNANTYLHW
YLQKPGQSPQLLIYKVSNRFSGVPDRFSGSGSGTDFTLKISRVEAEDVGVYYCSQNTHVPP
TFGQGTKLEIKRGGGGSGGGGSGGGGSQVQLVQSGAEVKKPGASVKVSCKASGYTFTD
YEMHWVRQAPGQGLEWMGALDPKTGDTAYSQKFKGRVTLTADESTSTAYMELSSLRSE
DTAVYYCTRFYSYTYWGQGTLVTVSSASTTTPAPRPPTPAPTIASQPLSLRPEACRPAAGG
AVHTRGLDFACDIYIWAPLAGTCGVLLLSLVITLYCKRGRKKLLYIFKQPFMRPVQTTQEE
DGCSCRFPEEEEGGCELRVKFSRSADAPAYKQGQNQLYNELNLGRREEYDVLDKRRGRD
PEMGGKPRRKNPQEGLYNELQKDKMAEAYSEIGMKGERRRGKGHDGLYQGLSTATKDT
YDALHMQALPPRKRRSGSGATNFSLLKQAGDVEENPGPMALPVTALLLPLALLLHAARP
GGQRFAQAQQQLPLESLGWDVAELQLNHTGPQQDPRLYWQGGPALGRSFLHGPELDKG
QLRIHRDGIYMVHIQVTLAICSSTTASRHHPTTLAVGICSPASRSISLLRLSFHQGCTIASQRL
TPLARGDTLCTNLTGTLLPSRNTDETFFGVQWVRP (SEQ ID NO:63)

### Soluble CD70

The tumor necrosis factor (TNF) superfamily contains 19 members that bind to 29 members of TNF receptor superfamily, which plays an essential role in regulation of antitumor immune responses. CD70, the cognate ligand of CD27, belongs to the TNF superfamily. CD70 is expressed on antigen-presenting cells as well as on activated T cells and NK cells after antigenic stimulation as a type II transmembrane glycoprotein, whereas CD27 is constitutively expressed on resting T, B and NK cells as a type I transmembrane glycoprotein. CD27/CD70 interaction was shown to induce T-cell proliferation and enhance antigen-specific cytotoxic T-lymphocyte (CTL) activity. CD70/CD27 signaling played a role in mediating durable rejection of primary tumor by induction of a tumor-specific T-cell memory. CD70 expression favored the beginning of a long memory T-cell response. However, CD27 has a propensity to shed from the cell surface of T cells to generate soluble CD27 upon T cells activated. The generated soluble CD27 masks naturally expressed CD70 to block CD70 interaction with cell surface CD27 downregulating CD70/CD27 signaling of T cells. Several groups have generated soluble CD70 (sCD70) molecule that contains the intact extracellular domain to form functional CD70 trimers. The studies demonstrated that resembling the native CD70, sCD70 was able to trigger CD70/CD27 signaling and elicit potent anti-tumor cellular immune responses when it was overexpressed.

In some embodiments, a soluble CD70 is derived from a Homo sapiens (human) CD70 (e.g., UniProtKB Accession NO: A0A0U5JA32; Accession NOs: P32970, B4DPR8, Q53XX4 and Q96J57), a Pan troglodytes (chimpanzee) CD70 (e.g., UniProtKB Accession NO: K7CN06 or A0A6D2XKV0), a Gorilla gorilla gorilla (Western lowland gorilla) CD70 (e.g., UniProtKB Accession NO: A0A2I2YA30), a Pan paniscus (Pygmy chimpanzee) CD70 (e.g., UniProtKB Accession NO: A0A2R9A5Z7), or a combination thereof.

In some embodiments, a soluble CD70 is derived from a human CD70, for example, human CD70 antigen isoform 1 (NP_001243.1, GI:4507605 (SEQ ID NO:23)), human CD70 antigen isoform 2 (NP_001317261.1, GI:1057866039 (SEQ ID NO:46)) or a variant thereof.

MPEEGSGCSVRRRPYGCVLRAALVPLVAGLVICLVVCIQRFAQAQQQLPLESLGWDVAEL
QLNHTGPQQDPRLYWQGGPALGRSFLHGPELDKGQLRIHRDGIYMVHIQVTLAICSSTTA
SRHHPTTLAVGICSPASRSISLLRLSFHQGCTIASQRLTPLARGDTLCTNLTGTLLPSRNTDE
TFFGVQWVRP(SEQ ID NO:23)

MPEEGSGCSVRRRPYGCVLRAALVPLVAGLVICLVVCIQRFAQAQQQLPLESLGWDVAEL
QLNHTGPQQDPRLYWQGGPALGRSFLHGPELDKGQLRIHRDGIYMVHIQVTLAICSSTTA
SRHHPTTLAVGICSPASRSISLLRLSFHQGLFGFWNWGLKVKCFLRHLIWTAHCFIPLTQLV
FMQALQSWRNHHCSHFTDEENRGVNR (SEQ ID NO:46)

In some embodiments, a soluble CD70 is a recombinant soluble CD70. In some embodiments, a soluble CD70 is multimeric soluble CD70 (composed of two, three, four, five, six, or more soluble CD70 subunits). In some embodiments, the soluble CD70 is trimeric soluble CD70. In some embodiments, the soluble CD70 is hexameric soluble CD70. In some embodiments, a soluble CD70 can bind three CD27 homodimers, engage CD27, activate the CD70/CD27 signaling pathway, or a combination of the foregoing.

In some embodiments, at least one subunit of a soluble CD70 (e.g., each subunit of the soluble CD70) has at least about 50% amino acid sequence identity to a full-length CD70 (e.g., a human full-length CD70). For example, the sequence identity can be at least about: 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79% or 80%. For example, the sequence identity can be at least about: 50-100%, 55-100%, 55-95%, 60-95%, 60-90%, 65-90%, 65-85%, 70-85%, 70-80%, or 75-80%.

In some embodiments, at least one subunit of a soluble CD70 (e.g., each subunit of the soluble CD70) has at least about 50% amino acid sequence identity to SEQ ID NO:23, SEQ ID NO:46, or both. For example, at least one subunit of a soluble CD70 has at least about: 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79% or 80% amino acid sequence identity to SEQ ID NO:2, SEQ ID NO:3, or both. For example, at least one subunit of a soluble CD70 has at least about: 50-100%, 55-100%, 55-95%, 60-95%, 60-90%, 65-90%, 65-85%, 70-85%, 70-80%, or 75-80% amino acid sequence identity to SEQ ID NO:23, SEQ ID NO:46, or both.

In some embodiments, at least one subunit of a soluble CD70 (e.g., each subunit of the soluble CD70) has at least about 50% amino acid sequence identity to SEQ ID NO:23. For example, at least one subunit of a soluble CD70 has at least about: 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79% or 80% amino acid sequence identity to SEQ ID NO:2. For example, at least one subunit of a soluble CD70 has at least about: 50-100%, 55-100%, 55-95%, 60-95%, 60-90%, 65-90%, 65-85%, 70-85%, 70-80%, or 75-80% amino acid sequence identity to SEQ ID NO:23.

In some embodiments, at least one subunit of a soluble CD70 (e.g., each subunit of the soluble CD70) is derived from a human CD70 antigen isoform 1 variant.

In some embodiments, a human CD70 antigen isoform 1 variant comprises:
a)one or more of the following substitutions: P2R, E4G, E4K, G5D, G5R, G7C, C8F, S9L, R12L, Y15C, Y15H, V18I, L19M, R20Q, R20W, A21T, A21V, L23F, L23S, L23V, L23W, V24L, P25Q, P25T, L26S, V27L, A28S, A28V, G29S, V31M, I32V, C33F, C33W, L34R, V35A, V35M, V36A, V36L, V36L, C37G, C37R, Q39E, R40P, F41I, A42S, Q43R, A44V, Q46E, Q47H, P49R, L50P, E51K, S52L, V57I, A58T, N63Y, G66A, G66E, Q68E, D70N, P71L, P71S, R72K, R72T, G78A, G78C, G78D, G78S, P79S, G82D, R83C, R83H, S84F, F85L, E90D, E90G, D92N, K93T, G94R, R97C, R97H, I98N, R100H, D101H, D101N, I103T, I103V, M105V, V106L, H107Y, I108V, T111M, I114M, C115W, S117P, T118A, T118M, T119A, T119M, A120S, H123P, H124P, P125H, P125T, T126P, T1271, T127P, A129G, A129P, A129S, V130A, V130E, V130M, S134F, S134P, P135S, A136T, S137F, R138C, R138H, S139I, I140F, S141N, R144C, R144H, S146I, F147L, F147S, H148Y, Q149R, G150C, G150S, G150V, C151Y, I153L, R157C, L158Q, T159M, P160L, A162T, R163Q, T166I, T169I, T169S, N170K, T172P, L175V, S178T, R179Q, D182G, F185L, Q189R, V191M, R192C, R192H, R192S,
b)deletion of S84, or
c)a combination of a) and b).

The website www.uniprot.org/uniprotkb/P32970/entry#disease_variants provides additional information on CD70 variants, the entire contents of which are incorporated by reference herein.

In some embodiments, at least one subunit of a soluble CD70 (e.g., each subunit of the soluble CD70) is derived from a CD70 mutein.

In some embodiments, a CD70 mutein comprises at least one cysteine substitution (e.g., at least one pair of cysteine substitution), at least one cysteine insertion, or any combination of the foregoing. In some embodiments, the at least one cysteine substitution (e.g., at least one pair of cysteine substitution) and/or the at least one cysteine insertion promote the formation of a disulfide bond with a cysteine residue on a neighboring CD70. Non-limiting examples of CD70 muteins include those disclosed in U.S. Patent Application Publication No. US20220098263, the entire contents of which are incorporated by reference herein. In some embodiments, a naturally occurring cysteine residue of a CD70, e.g., residue C151 of human CD70 antigen isoform 1, is mutated (e.g., to a serine residue) to avoid undesired dimerization.

In some embodiments, at least one subunit of a soluble CD70 (e.g., each subunit of the soluble CD70) lacks an intracellular domain and a transmembrane domain. In some embodiments, at least one subunit of a soluble CD70 (e.g., each subunit of the soluble CD70) is derived from the extracellular domain of a human CD70 (e.g., SEQ ID NO:22 or SEQ ID NO:47.

QRFAQAQQQLPLESLGWDVAELQLNHTGPQQDPRLYWQGGPALGRSFLHGPELDKGQLR
IHRDGIYMVHIQVTLAICSSTTASRHHPTTLAVGICSPASRSISLLRLSFHQGCTIASQRLTPL
ARGDTLCTNLTGTLLPSRNTDETFFGVQWVRP (SEQ ID NO:22)
QRFAQAQQQLPLESLGWDVAELQLNHTGPQQDPRLYWQGGPALGRSFLHGPELDKGQLR
IHRDGIYMVHIQVTLAICSSTTASRHHPTTLAVGICSPASRSISLLRLSFHQGLFGFWNWGL
KVKCFLRHLIWTAHCFIPLTQLVFMQALQSWRNHHCSHFTDEENRGVNR (SEQ ID NO:47)

In some embodiments, at least one subunit of a soluble CD70 (e.g., each subunit of the soluble CD70) has at least about 70% sequence identity to a human soluble CD70 sequence (e.g., SEQ ID NO:22, SEQ ID NO:47, or both). For example, at least one subunit of a soluble CD70 has at least about: 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to a human soluble CD70 sequence (e.g., SEQ ID NO:22, SEQ ID NO:47, or both). In some embodiments, at least one subunit of a soluble CD70 has about: 70-99%, 70-98%, 75-98%, 75-96%, 80-96%, 80-95%, 85-95%, or 85-90% sequence identity to a human soluble CD70 sequence (e.g., SEQ ID NO:22, SEQ ID NO:47, or both). In some embodiments, a soluble CD70 has at least about 80% amino acid sequence identity to a human soluble CD70 sequence (e.g., SEQ ID NO:22, SEQ ID NO:47, or both). In some embodiments, a soluble CD70 has at least about 85% amino acid sequence identity to the human soluble CD70 sequence (e.g., SEQ ID NO:22, SEQ ID NO:47, or both).

In some embodiments, at least one subunit of a soluble CD70 (e.g., each subunit of the soluble CD70) has at least about 90% sequence identity to a human soluble CD70 sequence (e.g., SEQ ID NO:22, SEQ ID NO:47, or both). For example, at least one subunit of a soluble CD70 has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to a human soluble CD70 sequence (e.g., SEQ ID NO:22, SEQ ID NO:47, or both). In some embodiments, at least one subunit of a soluble CD70 has about: 90-99.9%, 90-99.8%, 92-99.8%, 92-99.6%, 94-99.6%, 94-99.5%, 95-99.5%, 95-99.4%, 96-99.4%, 96-99.2%, 97-99.2% or 97-99% sequence identity to a human soluble CD70 sequence (e.g., SEQ ID NO:22, SEQ ID NO:47, or both). In some embodiments, at least one subunit of a soluble CD70 (e.g., each subunit of the soluble CD70) has at least about 95% amino acid sequence identity to a human soluble CD70 sequence (e.g., SEQ ID NO:22, SEQ ID NO:47, or both). In some embodiments, at least one subunit of a soluble CD70 (e.g., each subunit of the soluble CD70) has at least about 98% amino acid sequence identity to a human soluble CD70 sequence (e.g., SEQ ID NO:22, SEQ ID NO:47, or both).

In some embodiments, at least one subunit of a soluble CD70 (e.g., each subunit of the soluble CD70) comprises SEQ ID NO:22.

In some embodiments, at least one subunit of a soluble CD70 (e.g., each subunit of the soluble CD70) has at least about 70% sequence identity to SEQ ID NO:22. For example, at least one subunit of a soluble CD70 has at least about: 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO:22. In some embodiments, at least one subunit of a soluble CD70 has about: 70-99%, 70-98%, 75-98%, 75-96%, 80-96%, 80-95%, 85-95%, or 85-90% sequence identity to SEQ ID NO:22. In some embodiments, a soluble CD70 has at least about 80% amino acid sequence identity to SEQ ID NO:4. In some embodiments, a soluble CD70 has at least about 85% amino acid sequence identity to SEQ ID NO:22.

In some embodiments, at least one subunit of a soluble CD70 (e.g., each subunit of the soluble CD70) has at least about 90% sequence identity to SEQ ID NO:22. For example, at least one subunit of a soluble CD70 has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to SEQ ID NO:22. In some embodiments, at least one subunit of a soluble CD70 has about: 90-99.9%, 90-99.8%, 92-99.8%, 92-99.6%, 94-99.6%, 94-99.5%, 95-99.5%, 95-99.4%, 96-99.4%, 96-99.2%, 97-99.2% or 97-99% sequence identity to SEQ ID NO:22. In some embodiments, at least one subunit of a soluble CD70 (e.g., each subunit of the soluble CD70) has at least about 95% amino acid sequence identity to SEQ ID NO:22. In some embodiments, at least one subunit of a soluble CD70 (e.g., each subunit of the soluble CD70) has at least about 98% amino acid sequence identity to SEQ ID NO:22.

In some embodiments, at least one subunit of a soluble CD70 (e.g., each subunit of the soluble CD70) comprises SEQ ID NO:47.

In some embodiments, at least one subunit of a soluble CD70 (e.g., each subunit of the soluble CD70) has at least about 70% sequence identity to SEQ ID NO:47. For example, at least one subunit of a soluble CD70 has at least about: 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO:47. In some embodiments, at least one subunit of a soluble CD70 has about: 70-99%, 70-98%, 75-98%, 75-96%, 80-96%, 80-95%, 85-95%, or 85-90% sequence identity to SEQ ID NO:47. In some embodiments, a soluble CD70 has at least about 80% amino acid sequence identity to SEQ ID NO:47. In some embodiments, a soluble CD70 has at least about 85% amino acid sequence identity to SEQ ID NO:47.

In some embodiments, at least one subunit of a soluble CD70 (e.g., each subunit of the soluble CD70) has at least about 90% sequence identity to SEQ ID NO:47. For example, at least one subunit of a soluble CD70 has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to SEQ ID NO:47. In some embodiments, at least one subunit of a soluble CD70 has about: 90-99.9%, 90-99.8%, 92-99.8%, 92-99.6%, 94-99.6%, 94-99.5%, 95-99.5%, 95-99.4%, 96-99.4%, 96-99.2%, 97-99.2% or 97-99% sequence identity to SEQ ID NO:47. In some embodiments, at least one subunit of a soluble CD70 (e.g., each subunit of the soluble CD70) has at least about 95% amino acid sequence identity to SEQ ID NO:47. In some embodiments, at least one subunit of a soluble CD70 (e.g., each subunit of the soluble CD70) has at least about 98% amino acid sequence identity to SEQ ID NO:47.

In some embodiments, at least one subunit of a soluble CD70 (e.g., each subunit of the soluble CD70) comprises at least one amino acid addition, deletion, and/or substitution (e.g., at least one conservative substitution such as highly conservative amino acid substitution), relative to a human soluble CD70 sequence (e.g., SEQ ID NO:22 or SEQ ID NO:47). For example, the number of amino acid addition, deletion, and/or substitutions can be at least about: 2, 3, 4, 5, 6, 7, 8, 9, 10, 11, 12, 13, 14, 15, 16, 17, 18, 19, 20, 21 or 22, or about: 1-22, 1-20, 2-20, 2-18, 3-18, 3-16, 4-16, 4-14, 5-14, 5-12, 6-12, 6-10, 7-10 or 7-8.

In some embodiments, at least one subunit of a soluble CD70 (e.g., each subunit of the soluble CD70) comprises at least one amino acid addition, deletion, and/or substitution (e.g., at least one conservative substitution such as highly conservative amino acid substitution), relative to SEQ ID NO:22. For example, the number of amino acid addition, deletion, and/or substitutions can be at least about: 2, 3, 4, 5, 6, 7, 8, 9, 10, 11, 12, 13, 14, 15, 16, 17, 18, 19, 20, 21 or 22, or about: 1-22, 1-20, 2-20, 2-18, 3-18, 3-16, 4-16, 4-14, 5-14, 5-12, 6-12, 6-10, 7-10 or 7-8.

In some embodiments, at least one subunit of a soluble CD70 (e.g., each subunit of the soluble CD70) comprises at least one amino acid addition, deletion, and/or substitution (e.g., at least one conservative substitution such as highly conservative amino acid substitution), relative to SEQ ID NO:47. For example, the number of amino acid addition, deletion, and/or substitutions can be at least about: 2, 3, 4, 5, 6, 7, 8, 9, 10, 11, 12, 13, 14, 15, 16, 17, 18, 19, 20, 21 or 22, or about: 1-22, 1-20, 2-20, 2-18, 3-18, 3-16, 4-16, 4-14, 5-14, 5-12, 6-12, 6-10, 7-10 or 7-8.

### CAR Construct Design

In some embodiments, CAR constructs disclosed herein comprise an antigen-binding domain, a spacer domain, a hinge domain, a signal peptide domain, a transmembrane domain, and one or more costimulatory domains. Selecting components of CAR constructs, based upon cancer type, desired clinical efficacy and safety profiles, is understood by one of skill in the art.

### Antigen Binding Domain

Antigen-binding domains described herein are specific to at least one cell surface antigen of interest, for example, a pathologic antigen such as a cancer (e.g., tumor) antigen.

In some embodiments, a cell surface antigen is a tumor-associated antigen "TAA." In some embodiments, a tumor-associated antigen is expressed at a higher level in tumor cells than in non-tumor cells. In some embodiments, a tumor-associated antigen is a universal tumor antigen, i.e., broadly expressed by most types of tumors. In some embodiments, a tumor antigen is exclusively expressed in a specific type of tumor cells. In some embodiments, a tumor-associated antigen is expressed at low levels in non-tumor cells. In some embodiments, a tumor-associated antigen is not expressed in non-tumor cells. In some embodiments, a tumor antigen is a "tumor-specific antigen" or "TSA," i.e., is unique to a tumor cell. In some embodiments, a tumor-associated antigen is a differentiation antigen, a mutational antigen, an overexpressed cellular antigen, a viral antigen, or a combination thereof.

In some embodiments, a cell surface antigen is expressed in a solid tumor cell, a hematologic cancer cell, or both. In some embodiments, a cell surface antigen is expressed in a hematologic cancer cell. In some embodiments, a cell surface antigen is expressed in a solid tumor cell and a hematologic cancer cell.

In some embodiments, a cell surface antigen is expressed in a solid tumor cell. In some embodiments, a cell surface antigen is expressed in a liver cancer cell. In some embodiments, a cell surface antigen is expressed in a hepatocellular carcinoma (HCC) cell.

In some embodiments, a TAA is selected from colon cancer antigen 19.9, a gastric cancer mucin, antigen 4.2, glycoprotein A33 (gpA33), ADAM-9, gastric cancer antigen AH6, ALCAM, malignant human lymphocyte antigen APO-1, cancer antigen B1, B7 H3, beta-catenin, blood group ALeb/Ley, Burkitt's lymphoma antigen-38.13, colonic adenocarcinoma antigen C14, ovarian carcinoma antigen CA125, carboxypeptidase M, CD5, CD19, CD20, CD22, CD23, CD25, CD27, CD30, CD33, CD36, CD45, CD46, CD52, CD79a/CD79b, CD103, CD317, CDK4, carcinoembryonic antigen (CEA), CEACAM5, CEACAM6, C017-iA, CO-43 (blood group Leb), CO-514 (blood group Lea), CTA-1, CTLA4, cytokeratin 8, antigen D1.1, antigen D 156-22, DR5, Ei series (blood group B), EGFR (Epidermal Growth Factor Receptor), Ephrin receptor A2 (EphA2), ErbB1, ErbB3, ErbB4, GAGE-1, GAGE-2, GD2/GD3/GM2, lung adenocarcinoma antigen F3, antigen FC10.2, G49, ganglioside GD2, ganglioside GD3, ganglioside GM2, ganglioside GM3, GD2, GD3, GICA 19-9, GM2, gpOO, glypican-3 (GPC3), human leukemia T cell antigen Gp37, melanoma antigen gp75, gpA33, human epidermal growth factor receptor 2 (HER2) antigen (e.g., pi85 HER2), human milk fat globule antigen (HMFG), human papillomavirus E6/human papillomavirus-E7, high molecular weight melanoma antigen (HMW MAA), I antigen (differentiation antigen) I(Ma), integrin α-V-β-6 integrinP6 (ITGB6), interleukin-13, receptor α2 (IL13Rα2), JAM-3, KID3, KID31, KS 1/4 pan carcinoma antigen, human lung carcinoma antigens L6 and L20, LEA, LUCA-2, Mi:22:25:8, M18, M39, MAGE-1, MAGE-3, MART, MUC-1, MUM-1, Myl, N acetylglucosaminyltransferase, neoglycoprotein, NS-10, OFA-1, OFA-2, oncostatin M, p15, melanoma-associated antigen p97, polymorphic epithelial mucin (PEM), polymorphic epithelial mucin antigen (PEMA), PIPA, prostate-specific antigen (PSA), prostate-specific membrane antigen (PSMA), prostatic acid phosphate, RORi, sphingolipids, SSEA-1, SSEA-3, SSEA-4, sTn, T-cell receptor derived peptide, TAG-72, TL5 (blood group A), TNF-α receptor, TNF-β receptor, TNF- receptor, TRA-1-85 (blood group H), transferrin receptor, tumor-specific transplantation antigen (TSTA), oncofetal antigen-alpha-fetoprotein (AFP), VEGF, VEGFR, VEP8, VEP9, VIM-D5, and Y hapten, Ley. In some embodiments, the TAA is CEA, GPC3, MUC-1, EpCAM, HER receptor, PEM, Caludi6, Cluadi-18.2, mesothelin, A33, G250, carbohydrate antigens Ley, Lex, Leb, PSMA, TAG-72, STEAP1, CD166, CD24, CD44, E-cadherin, SPARC, ErbB2, ErbB3, MUC1, LMP2, idiotype, HPVE6&E7, EGFR, EGFRvIII, HER-2/neu, MAGEA3, NY-ESO-1, GD2, PSMA, PCSA, PSA, MelanA/MART1, CD19, CD20, CD22, CD33, CD5, CD70, or BCMA.

In some embodiments, a cell surface antigen is selected from IL13Rα2, HER2, EGFR, EGFR variant III, GPC3, DLL3, GUCY2C, Claudin 18.2 (CLDN18.2) and combinations thereof.

In some embodiments, a cell surface antigen comprises a glypican 3 (GPC3) antigen, a DLL3 (Delta-like ligand 3) antigen, a GUCY2C(GCC) antigen, a Claudin 18.2 (CLDN18.2) antigen and combinations thereof.

In some embodiments, an antigen-binding domain comprises a mutein, a single-chain variable fragment (scFv), a heavy-chain antibody (sdAb) (e.g., a Vhh, a nanobody), or any combination of the foregoing.

In some embodiments, an antigen binding domain is an antigen-binding fragment of an immunoglobulin molecule (e.g., antibody). In some embodiments, an antigen binding domain comprises a mutein, a single-chain variable fragment (scFv), a heavy-chain antibody (e.g., Vhh), a nanobody, or a combination thereof. In some embodiments, a CAR comprises one antigen binding domain. In some embodiments, a CAR comprises two or more antigen binding domains, for example, a mutein and a scFv, two nanobodies, a mutein and two nanobodies, or a scFv and a nanobody.

In some embodiments, an antigen binding domain is an scFv. In some embodiments, an scFv comprises a humanized VH region, a humanized VL region, or both. In some embodiments, an scFv comprises a fully human VH region, a fully human VL region, or both.

In some embodiments, a VH region and a VL region of an scFv are linked via a peptide linker. In some embodiments, a peptide linker has stretches of hydrophilic residues, for example, glycine and/or serine to increase flexibility, and glutamate and/or lysine to increase solubility.

In some embodiments, an antigen binding domain comprises an scFv that binds a glypican 3 (GPC3) antigen, a DLL3 (Delta-like ligand 3) antigen, a GUCY2C(GCC) antigen, or a Claudin 18.2 (CLDN18.2) antigen. In some embodiments, an scFv targeting GPC3, DLL3, GUCY2C or Claudin 18.2 comprises VH and VL regions from one or more antibodies specific for GPC3, DLL3, GUCY2C or Claudin 18.2.

A non-limiting example of scFv or VHH targeting DLL3, GUCY2C, Claudin 18.2 or GPC3 is provided below.
anti DLL3 (Delta-like ligand 3) scFv 
DIVMTQSPLSLPVTPGEPASISCRASQTISDYLHWYLQKPGQSPQLLIKYASQSISGVPDRFS
GSGSGSDFTLKISRVEAEDVGVYYCQQGHSFPLTFGQGTKLEIKGGGGSGGGGSGGGGSE
VQLVQSGAEVKKPGASVKVSCKASGYTFTSYWMHWVRQAPGQRLEWMGTIYPGNSDT
RYNQKFKDRVTITADTSASTAYMELSSLRSEDTAVYYCTRSYDYGDLDYWGQGTTVTVSS
(SEQ ID NO:13)
 
DIVMTQSQKFMSTSVGDRVSITCKASQNVGTAVAWYQQKPGQSPKLLIFSASIRYTGVPDR
FTGSGSGTDFTLTISNMQSEDLADYFCQQYSSSPYTFGGGTKLEIKGGGGSGGGGSGGGG
SEVQLQQSGPVLVKPGASVKMSCKASGYTFTDYYMNWVKRSHGKSLEWIGIINPYNGGT
SYNQKFRGKATLTVDKSSNTAYMELNSLTSEDSAVYYCSRYDYDYDVEGAMDYWGQGT
SVTVSS (SEQ ID NO:48)
 
DIVMTQSPATLSVTPGDRVSLSCRASQTISDYLHWYQQRSHESPRLLIKYASQSISGIPSRFS
GSGSGSDFTLSINSVEPEDVGVYYCQNGHSFPLTFGAGTKLELKGGGGSGGGGSGGGGSE
VQLQQSGTVLARPGASVKMSCKTSGYTFTSYWMHWVKQSPGQGLEWIGTIYPGNSDTR
YNQKFKDKAYVTAVTSASTAYMELSSLTNEESAVYYCTRSYDYGDLDYWGQGTTLTVSS(
SEQ ID NO:49)
 
DIVLTQSPASLAVSLGQRATISCRASKSVSTSGFSYMHWYQQKPGQPPKLLIYLASNLESG
VPARFSGSGSGTDFTLNIHPVEEEDAATYYCQHSRELPFTFGSGTKLEIKGGGGSGGGGSG
GGGSQVQLQQSGAELVKPGASVRISCKASGYAFSNYWINWVKQRPGKGLEWIGQINPGD
GDTIYNGKFKGKATLTADKSSSTAYMQLSSLTSEDSAVYFCEREGRNYGDFAYWGQGTTL
TVSS(SEQ ID NO:50)
anti CLDN18.2 scFv 
QVQLVQSGAEVKKPGSSVKVSCKASGYAFSNYLIEWVKQAPGQGLEWIGLINPGSGGTN
YNEKFKGKATITADKSTSTAYMELSSLRSEDTAVYYCARVYYGNSFAYWGQGTLVTVSSG
GGGSGGGGSGGGGSDIVMTQSPDSLAVSLGERATISCKSSQSLLNSGNQKNYLTWYQQKP
GQPPKLLIYWASTRESGVPDRFSGSGSGTDFTLTISSLQAEDVAVYYCQNDYFYPFTFGQG
TKLEIK(SEQ ID NO:27)
 
DIVMTQSPDSLAVSLGERATISCKSSQSLLNSGNQKNYLTWYQQKPGQPPKLLIYWASTRE
SGVPDRFSGSGSGTDFTLTISSLQAEDVAVYYCQNDYFYPFTFGQGTKLEIKGGGGSGGGG
SGGGGSQVQLVQSGAEVKKPGSSVKVSCKASGYAFSNYLIEWVKQAPGQGLEWIGLINP
GSGGTNYNEKFKGKATITADKSTSTAYMELSSLRSEDTAVYYCARVYYGNSFAYWGQGTL
VTVSS(SEQ ID NO:51)
 
DIVMTQSPDSLAVSLGERATINCKSSQSLFNSGNQKNYLTWYQQKPGQPPKLLIYWASTRE
SGVPDRFSGSGSGTDFTLTISSLQAEDVAVYYCQNAYSFPYTFGGGTKLEIKGGGGSGGGG
SGGGGSQVQLQESGPGLIKPSQTLSLTCTVSGGSISSGYNWHWIRQPPGKGLEWIGYIHYT
GSTNYNPALRSRVTISVDTSKNQFSLKLSSVTAADTAIYYCARIYNGNSFPYWGQGTTVTV
SS(SEQ ID NO:52)
anti GPC3 scFv

DVVMTQSPLSLPVTPGEPASISCRSSQSLVHSNANTYLHWYLQKPGQSPQLLIYKVSNRFS
GVPDRFSGSGSGTDFTLKISRVEAEDVGVYYCSQNTHVPPTFGQGTKLEIKRGGGGSGGG
GSGGGGSQVQLVQSGAEVKKPGASVKVSCKASGYTFTDYEMHWVRQAPGQGLEWMG
ALDPKTGDTAYSQKFKGRVTLTADESTSTAYMELSSLRSEDTAVYYCTRFYSYTYWGQGT
LVTVSS (SEQ ID NO:64)

In some embodiments, a scFv has at least 90% sequence identity to the amino acid sequence set forth in SEQ ID NO:13, 27, 48-52 and 64. For example, a scFv has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to the amino acid sequence set forth in SEQ ID NO:13, 27, 48-52 and 64.

In some embodiments, an antibody targeting DLL3, GUCY2C, Claudin 18.2 or GPC3 comprises at least one amino acid addition, deletion, and/or substitution (e.g., at least one conservative substitution such as highly conservative amino acid substitution), relative to the amino acid sequence set forth in SEQ ID NO:13, 27, 48-52 and 64. For example, the number of amino acid addition, deletion, and/or substitutions can be at least about: 2, 3, 4, 5, 6, 7, 8, 9, 10, 11 or 12, or about: 1-12, 1-10, 2-10, 2-9, 3-9, 3-8, 4-8, 4-7, 5-7 or 5-6.

In some embodiments, an antigen binding domain comprises a VHH (e.g., a humanized VHH) that binds to the GUCY2C (GCC) antigen.
anti GUCY2C (GCC) VHH

EVQLVESGGGLVKPGGSLRLSCAASGRTSTIHRMGWFRQAPGKEREFVSSISRVSSTTYYA
DSVKGRFTISRDNAKNSLYLQMNSLRAEDTAVYYCAASEYEVSPGKSKYWGQGTMVTV
SS(SEQ ID NO:39)

EVQLVESGGGLVQPGRSLRLSCAASVLNLDYYAITWFRQAPGKEREGVSCIQGRGTSTNY
ADSVKGRFTISRDRAKKSAYLQMNSLRAEDTALYYCAVRYVSYGRNTACGLLPESVDHW
GQGTMVTVSS(SEQ ID NO:53)

EVQLVESGGGLVQPGGSLRLSCAASGFTLDDYAIGWFRQAPGKEREGVICLTSGGLPKYA
DSVKGRFTISRDNSKNTLYLQMNSLRAEDTAVYYCAASGATSRWNCPPQYDHWGQGTM
VTVSS(SEQ ID NO:54)

In some embodiments, non-limiting examples of VHH targeting GPC3 are provided below.

QVQLVESGGGLVQPGGSLRLSCAASGSISTYTMAWYRQAPGEQRESVAAISSGGRTDYIDS
VKGRFTISRDNAKNMVYLQMNSLKPEDTAVYYCNSADGLKIGTYYFKGLGWGQGTQVT
VSA (SEQ ID NO:65)

QLQLVESGGGLVQPGGSLRLSCAASGFTLDVYAIGWFRQAPGKEREGVSYITSGDGIFYAD
SVKGRFTISRDNAKNTVYLQMNSLKPEDTAVYFCATLKRTTAGGWPIPGRIGGQGTQVTV
SS (SEQ ID NO:66)

QVQLVESGGGLVQPGGSLRLSCAASGFTLDAYAIGWFRQAPGKEREGVSYITSGTGTTLY
ADSVKGRFTISRDNAKNTVYLQMNSLKPEDTAVYFCTTLKRTTAGGWPIPGRIGGQGTQV
TVSS (SEQ ID NO:67)

QVQLVESGGGLVQAGGSLRLSCAASGFTLENYAIGWFRQAPGKEREGVSYITGGTGTTVY
ADSVKGRFTISRDNTKNTVYLQMNSLKPEDTAVYFCATLKRTTAGGWPKPGRIGGQGTQ
VTVSS (SEQ ID NO:68)

QLQLVESGGGLVQPGGSLRLSCAASGFTLDVYAIGWFRQAPGKEREGVSYITGGTGTTVY
ADSVKGRFTISRDNTKNTVYLQMNSLKPEDTAVYFCATLKRTTAGGWPKPGRIGGQGTQ
VTVSS (SEQ ID NO:69)

QVQLVESGGGLVQAGGSLSLSCAASGRRFSTNVMGWFRQAPGKEREFLAAINWVIGNTN
YAESVKGRFTISRDNAKETVYLQMDNLKVEDTAVYYCAGRSSYYTSSRREDYDYWGQG
TQVTVSS (SEQ ID NO:70)

QVQLVESGGGLVQLGGSLTLSCVASGDVHKINAKGWYRQAPGKEREVVAIITSGGTPYYA
DAVKGRFTISRHDDKNTVTLQMNSLKPEDTAMYYCAAGLRGMGPYDYDYRGPGTQVTV
SS (SEQ ID NO:71)

QVQLVESGGGLVQAGGSLKLSCAASGRSFSPYAMGWFRQAPGKDREFVAAISNSGGSTY
YADAVKGRFSISRDNAKNTVYLQMNNLEPEDTAVYYCTGPVKRYSTDFQGGDYWGQGT
QVTVSS (SEQ ID NO:72)

QVQLVESGGGLVQAGGSLRLSCAFSGRTFSKYAMAWFRQAPGKGRDFVARVGPSGRTTD
YADSVKGRFTVSRDNAKNTVSLQMSSLKPDDAALYYCAATSGIYGESYNLYNYWGQGT
QVTVSS (SEQ ID NO:73)

QVQLVESGGGLVRPGGSLRVSCAASGVDISTYTMAWYRQAPGEQRESVAAISTTGRSIYID
AVQGRFTMSRDNAKNTVYLQMNNLKPEDTAVYYCNSADDLKIGTQYFKGLGWGQGTQ
VTVSS (SEQ ID NO:74)

QVQLVESGGGLVQAGASLRLSCLGSGRSFGLRAMGWFRQAPGKELEFVAAIGKAGDTTY
YTDSVKGRFTISRDNVKNAVYLQMNSLKPEDTAVYVCATAARWEPPTITPGSYRGPGTQV

TVSS (SEQ ID NO:75)

QVQLVQSGAEVKKPGASVKVSCKASGYTFTDYEMHWVRQAPGQGLEWMGALDPKTGD
TAYSQKFKGRVTLTADESTSTAYMELSSLRSEDTAVYYCTRFYSYTYWGQGTLVTVSS
(SEQ ID NO:76)

In some embodiments, a VHH has at least 90% sequence identity to an amino acid sequence set forth in any one of SEQ ID NOs:39, 53-54 and 65-76. For example, a VHH has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to an amino acid sequence set forth in any one of SEQ ID NOs:39, 53-54 and 65-76.

In some embodiments, a VHH comprises at least one amino acid addition, deletion, and/or substitution (e.g., at least one conservative substitution such as highly conservative amino acid substitution), relative to an amino acid sequence set forth in any one of SEQ ID NOs:39, 53-54 and 65-76. For example, the number of amino acid addition, deletion, and/or substitutions can be at least about: 2, 3, 4, 5, 6, 7, 8, 9, 10, 11 or 12, or about: 1-12, 1-10, 2-10, 2-9, 3-9, 3-8, 4-8, 4-7, 5-7 or 5-6.

In some embodiments, a Vhh comprises an amino acid sequence set forth in any one of SEQ ID NOs:39, 53-54 and 65-76.

In some embodiments, an antigen binding domain comprises one Vhh, for example, comprising an amino acid sequence set forth in any one of SEQ ID NOs:39, 53-54 and 65-76.

In some embodiments, an antigen binding domain comprises two or more Vhhs (e.g., two Vhhs or three or more Vhhs). In some embodiments, an antigen binding domain comprises two Vhhs, for example, comprising any one (two identical Vhhs) or two sequences (two different Vhhs). In some embodiments, two Vhh sequences are connected by a (GGGGS)n linker, for example a (GGGGS)1 linker (e.g., SEQ ID NO:55), a (GGGGS)2 linker (e.g., SEQ ID NO:56), a (GGGGS)3 linker (e.g., SEQ ID NO:11) or a (GGGGS)4 linker (e.g., SEQ ID NO:12), or a Whitlow linker (e.g., SEQ ID NO:57).
GGGGS (SEQ ID NO:55)
GGGGSGGGGS (SEQ ID NO:56)
GGGGSGGGGSGGGGS(SEQ ID NO:11)
GGGGSGGGGSGGGGSGGGGS (SEQ ID NO: 12)
GSTSGSGKPGSGEGSTKG(SEQ ID NO:57)

### Signal Peptide

In some embodiments, a CAR comprises a signal peptide at the N-terminus for surface expression. Non-limiting examples of signal peptides include a CD8α signal polypeptide (e.g., SEQ ID NO:1), a GM-CSF receptor signal polypeptide (e.g., SEQ ID NO:58), a CD33 signal peptide, a signal sequence for granulocyte-macrophage colony stimulating factor, an IgG1 heavy chain signal polypeptide (e.g., SEQ ID NO:59), and Ig κ or λ light chain signal peptides.
MALPVTALLPLALLLHAARP (SEQ ID NO:1)
MVLLVTSLLLCELPHPAFLLIP (SEQ ID NO:58)
MEFGLSWVFLVALLRGVQC (SEQ ID NO:59)

In some embodiments, a signal peptide is a CD8α signal polypeptide (e.g., SEQ ID NO:1). In some embodiments, a signal peptide is a GM-CSF receptor signal polypeptide (e.g., SEQ ID NO:58).

In some embodiments, a signal peptide has at least 90% sequence identity to any one of SEQ ID NOs:1, 58 and 59. For example, a signal peptide has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to any one of SEQ ID NOs:1, 58 and 59. In some embodiments, a signal peptide comprises the amino acid sequence set forth in SEQ ID NO:1, 58 and 59.

In some embodiments, a signal peptide has at least 90% sequence identity to SEQ ID NO:1. For example, a signal peptide has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to SEQ ID NO:1. In some embodiments, a signal peptide comprises the amino acid sequence set forth in SEQ ID NO:1.

### Hinge/Spacer Domain

In some embodiments, a CAR comprises one or more hinge/spacer domains. In some embodiments, a hinge/spacer domain facilitates binding between a surface antigen and a CAR, or a domain thereof, for example, by achieving an optimal distance for binding, by providing conformational freedom, by reducing (e.g., occluding) steric hindrance, or any combination thereof.

In some embodiments, a hinge/spacer domain is located between an extracellular domain (comprising an antigen binding domain) and a transmembrane domain of a CAR. In some embodiments, a hinge/spacer domain is located between a cytoplasmic domain and a transmembrane domain of a CAR.

In some embodiments, a hinge/spacer domain is about 5-300 amino acids, for example, about: 5-20, 5-30, 5-60, 10-20, 10-30, 10-60, 10-100, 60-100, 60-180, 100-140, 110-130, 180-260, 200-240, 210-230, or 200-300 amino acids. In some embodiments, a hinge/spacer domain is a CD8α hinge/spacer domain (e.g., SEQ ID NO:2). In some embodiments, a hinge/spacer domain is a CD28 hinge/spacer domain (e.g., SEQ ID NO:3).
TTTPAPRPPTPAPTIASQPLSLRPEACRPAAGGAVHTRGLDFACD (SEQ ID NO:2)
IEVMYPPPYLDNEKSNGTIIHVKGKHLCPPSPLFPGPSKP(SEQ ID NO:3)

In some embodiments, a hinge/spacer domain has at least 90% amino acid sequence identity to SEQ ID NO:2 or SEQ ID NO:3. For example, a hinge/spacer domain has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to SEQ ID NO:2 or SEQ ID NO:3. In some embodiments, a hinge/spacer domain comprises the amino acid sequence set forth in SEQ ID NO:2 or SEQ ID NO:3.

### Transmembrane Domain

In some embodiments, a CAR comprises a transmembrane domain. In some embodiments, a transmembrane domain comprises a hydrophobic α helix that spans the cell membrane.

In some embodiments, a transmembrane domain is derived from CD28, CD8α, or CD4. In some embodiments, a transmembrane domain is a CD8α transmembrane domain (e.g., SEQ ID NO:4). In some embodiments, a transmembrane domain is a CD28 transmembrane domain (e.g., SEQ ID NO:5). In some embodiments, a transmembrane domain is a chimera, e.g., of CD8α and CD28 transmembrane domains.
IYIWAPLAGTCGVLLLSLVITLYC (SEQ ID NO:4)_{∘}
FWVLVVVGGVLACYSLLVTVAFIIFWV (SEQ ID NO:5) _{∘}

In some embodiments, a transmembrane domain has at least 90% sequence identity to SEQ ID NO:4 or SEQ ID NO:5. For example, a transmembrane domain has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to SEQ ID NO:4 or SEQ ID NO:5. In some embodiments, a transmembrane domain comprises the amino acid sequence set forth in SEQ ID NO:4 or SEQ ID NO:5.

In some embodiments, a transmembrane domain has at least 90% sequence identity to SEQ ID NO:4. For example, a transmembrane domain has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to SEQ ID NO:4. In some embodiments, a transmembrane domain comprises the amino acid sequence set forth in SEQ ID NO:4.

### Intracellular Stimulatory Domain

In some embodiments, a CAR comprises one or more intracellular stimulatory domains. As a functional end of a CAR, an intracellular stimulatory domain potentiates and/or modulates response of immune effector cells following antigen recognition, receptors clustering, and signal transmission to the cell.

In some embodiments, an intracellular stimulatory domain comprises CD3ζ (e.g., SEQ ID NO:60 or SEQ ID NO:8). CD3ζ is the cytoplasmic signaling domain of the T-cell receptor (TCR) complex. CD3ζ comprises three immunoreceptor tyrosine-based activation motifs (ITAMs). After a T cell is engaged with a surface antigen, the ITAMs transmit an activation signal to the T cell.

RVKFSRSADAPAYKQGQNQLYNELNLGRREEYDVLDKRRGRDPEMGGKPRRKNPQEGL
YNELQKDKMAEAYSEIGMKGERRRGKGHDGLYQGLSTATKDTYDALHMQALPPR(SEQ
ID NO:60)

RVKFSRSADAPAYQQGQNQLYNELNLGRREEYDVLDKRRGRDPEMGGKPRRKNPQEGL
YNELQKDKMAEAYSEIGMKGERRRGKGHDGLYQGLSTATKDTYDALHMQALPPR(SEQ
ID NO:8)

In some embodiments, an intracellular stimulatory domain has at least 90% sequence identity to SEQ ID NO:8 or SEQ ID NO:60. For example, an intracellular stimulatory domain has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to SEQ ID NO:8 or SEQ ID NO:60. In some embodiments, an intracellular stimulatory domain comprises the amino acid sequence set forth in SEQ ID NO:8 or SEQ ID NO:60.

In some embodiments, an intracellular stimulatory domain has at least 90% sequence identity to SEQ ID NO:8. For example, an intracellular stimulatory domain has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to SEQ ID NO:8. In some embodiments, an intracellular stimulatory domain comprises the amino acid sequence set forth in SEQ ID NO:8.

### Co-stimulatory Domain

In some embodiments, a CAR comprises one or more co-stimulatory domains, for example, to transmit a full proliferative/survival signal, together with the intracellular stimulatory domain (e.g., CD3ζ). Non-limiting examples of co-stimulatory domains include 4-1BB, CD28, OX-40, ICOS, and CD27 co-stimulatory domains.

In some embodiments, a co-stimulatory domain is a 4-1BB co-stimulatory domain (e.g., SEQ ID NO:6), for example, to expand and persist, to increase oxidative metabolism, to reduce exhaustion, and/or to generate central memory T cells.
KRGRKKLLYIFKQPFMRPVQTTQEEDGCSCRFPEEEEGGCEL(SEQ ID NO:6)

In some embodiments, a co-stimulatory domain is a CD28 co-stimulatory domain (e.g., SEQ ID NO:7), for example, to yield a potent, effector-like phenotype (e.g., high levels of cytolytic capacity, interleukin-2 (IL-2) secretion, and/or glycolysis).

LVVVGGVLACYSLLVTVAFIIFWVRSKRSRLLHSDYMNMTPRRPGPTRKHYQPYAPPRDF
AAYRS (SEQ ID NO:7)

In some embodiments, a CAR comprises two or more co-stimulatory domains, for example, a 4-1BB co-stimulatory domain (e.g., SEQ ID NO:6) and a CD28 co-stimulatory domain (e.g., SEQ ID NO:7).

In some embodiments, a CAR disclosed herein comprises a CD3ζ intracellular signaling domain (e.g., SEQ ID NO:8 or SEQ ID NO:60) and a 4-1BB co-stimulatory domain (e.g., SEQ ID NO:6). In some embodiments, a CAR disclosed herein comprises a CD3ζ intracellular signaling domain (e.g., SEQ ID NO:8 or SEQ ID NO:60) and a CD28 co-stimulatory domain (e.g., SEQ ID NO:7). In some embodiments, a CAR disclosed herein comprises a CD3ζ intracellular signaling domain (e.g., SEQ ID NO:8 or SEQ ID NO:60), a 4-1BB co-stimulatory domain (e.g., SEQ ID NO:6), and a CD28 co-stimulatory domain (e.g., SEQ ID NO:7).

In some embodiments, a co-stimulatory domain has at least 90% sequence identity to SEQ ID NO:6 or SEQ ID NO:7. For example, a co-stimulatory domain has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to SEQ ID NO:6 or SEQ ID NO:7. In some embodiments, a co-stimulatory domain comprises the amino acid sequence set forth in SEQ ID NO:6 or SEQ ID NO:7.

### CAR Construct

Various components of a CAR construct can be selected based on desired function, for example, clinical efficacy and safety profile. In some embodiments, a CAR comprises a signal peptide, an antigen binding domain, a hinge/spacer domain, a transmembrane domain, a costimulatory domain, and an intracellular stimulatory domain.

In some embodiments, a CAR disclosed herein comprises an antigen-binding domain (e.g., a scFv or Vhh binding a GPC3 antigen, a DLL3 antigen, a Claudin 18.2 antigen, or a GUCY2C antigen, ), a transmembrane domain (e.g., of CD28 or CD8) and an intracellular stimulatory domain (e.g., of CD3ζ). In some embodiments, the CAR further comprises a signal peptide (e.g., of CD8α), a hinge/spacer domain (e.g., of CD8α), a costimulatory domain (e.g., 4-1BB), or any combination of the foregoing.

In some embodiments, a CAR comprises the following elements:
a signal peptide,
an antigen-binding domain,
a hinge/spacer domain,
a transmembrane domain,
a costimulatory domain, and
an intracellular stimulatory domain.

In some embodiments, a CAR comprises the following elements (e.g., in the following sequence):
a signal peptide,
a scFv,
a hinge/spacer domain,
a transmembrane domain,
a costimulatory domain, and
an intracellular stimulatory domain.

In some embodiments, a CAR comprises the following elements (e.g., in the following sequence):
a CD8α signal peptide,
an anti-GPC3 scFv,
a CD8α hinge/spacer domain,
a CD8α transmembrane domain,
a 4-1BB costimulatory domain, and
a CD3ζ intracellular stimulatory domain.

In some embodiments, a CAR comprises:
a signal peptide having at least 90% sequence identity to SEQ ID NO:1;
an antigen-binding domain having at least 90% sequence identity to SEQ ID NO:64;
a CD8α hinge having at least about 90% amino acid sequence identity to SEQ ID NO:2;
a CD8α transmembrane domain having at least about 90% amino acid sequence identity to SEQ ID NO:4;
a 4-1BB costimulatory domain having at least about 90% amino acid sequence identity to SEQ ID NO:6;
a CD3ζ signaling domain having at least about 90% amino acid sequence identity to SEQ ID NO:60,
or any combination of the foregoing.

In some embodiments, a CAR has at least 90% sequence identity to the amino acid sequence set forth in SEQ ID NO:77. For example, a CAR has at least about: 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to the amino acid sequence set forth in SEQ ID NO:77.

MALPVTALLLPLALLLHAARPDVVMTQSPLSLPVTPGEPASISCRSSQSLVHSNANTYLHW
YLQKPGQSPQLLIYKVSNRFSGVPDRFSGSGSGTDFTLKISRVEAEDVGVYYCSQNTHVPP
TFGQGTKLEIKRGGGGSGGGGSGGGGSQVQLVQSGAEVKKPGASVKVSCKASGYTFTD
YEMHWVRQAPGQGLEWMGALDPKTGDTAYSQKFKGRVTLTADESTSTAYMELSSLRSE
DTAVYYCTRFYSYTYWGQGTLVTVSSASTTTPAPRPPTPAPTIASQPLSLRPEACRPAAGG
AVHTRGLDFACDIYIWAPLAGTCGVLLLSLVITLYCKRGRKKLLYIFKQPFMRPVQTTQEE
DGCSCRFPEEEEGGCELRVKFSRSADAPAYKQGQNQLYNELNLGRREEYDVLDKRRGRD

PEMGGKPRRKNPQEGLYNELQKDKMAEAYSEIGMKGERRRGKGHDGLYQGLSTATKDT
YDALHMQALPPRKRRSGSGATNFSLLKQAGDVEENPGPMALPVTALLLPLALLLHAARP
(SEQ ID NO:77)

In some embodiments, a CAR comprises an amino acid sequence having at least one amino acid addition, deletion, and/or substitution (e.g., at least one conservative substitution such as highly conservative amino acid substitution), relative to the amino acid sequence set forth in SEQ ID NO:24, 36, 43 or 77. For example, the number of amino acid addition, deletion, and/or substitutions can be at least about: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44 or 45, or about: 1-45, 2-45, 2-40, 3-40, 3-35, 4-35, 4-30, 5-30, 5-25, 6-25, 6-20, 7-20, 7-15, 8-15, 8-14, 9-14, 9-12 or 10-12.

In some embodiments, a CAR comprises the amino acid sequence set forth SEQ ID NO:24, 36, 43 or 77.

### Self-Cleaving Peptide

A polynucleotide disclosed herein further comprises a sequence encoding a self-cleaving peptide, e.g., a self-cleaving peptide can be positioned between the CAR and the CD70 armor molecule to achieve separation of the two in vivo.

In some embodiments, a self-cleaving peptide is a self-cleaving P2A (e.g., SEQ ID NO:78), T2A (e.g., SEQ ID NO:79), E2A (e.g., SEQ ID NO:80 or SEQ ID NO:81), F2A peptide (e.g., SEQ ID NO:82 or SEQ ID NO:83) or I2A peptide (e.g., SEQ ID NO:84). In some embodiments, a self-cleaving peptide is a self-cleaving P2A peptide. In some embodiments, a self-cleaving peptide comprises the amino acid sequence set forth in any one of SEQ ID NOs:78-84.
GSGATNFSLLKQAGDVEENPGP (SEQ ID NO:78)
GSGEGRGSLLTCGDVEENPGP (SEQ ID NO:79)
GSGQCTNYALLKLAGDVESNPGP (SEQ ID NO:80)
QCTNYALLKLAGDVESNPGP (SEQ ID NO:81)
GSGVKQTLNFDLLKLAGDVESNPGP (SEQ ID NO:82)
VKQTLNFDLLKLAGDVESNPGP (SEQ ID NO:83)
TRAEIEDELIRRGIESNPGP (SEQ ID NO:84)

In some embodiments, a self-cleaving peptide has at least about 90% sequence identity to a sequence set forth in any one of SEQ ID NOs:78-84. For example, a self-cleaving peptide has about: 91%, 92%, 93%, 94%, 95%, 96%, 97%, 98% or 99% sequence identity to a sequence set forth in any one of SEQ ID NOs:78-84. In some embodiments, a self-cleaving peptide comprises the amino acid sequence set forth in any one of SEQ ID NO:78-84.

In some embodiments, a self-cleaving peptide comprises at least one amino acid addition, deletion, and/or substitution (e.g., at least one conservative substitution such as highly conservative amino acid substitution), relative to a sequence set forth in any one of SEQ ID NOs:78-84.

In some embodiments, a self-cleaving peptide has at least about 90% sequence identity to a sequence set forth in SEQ ID NO:78. For example, a self-cleaving peptide has about: 91%, 92%, 93%, 94%, 95%, 96%, 97%, 98% or 99% sequence identity to a sequence set forth in SEQ ID NO:78. In some embodiments, a self-cleaving peptide comprises the amino acid sequence set forth in SEQ ID NO:78.

In some embodiments, a polynucleotide disclosed herein further comprises a sequence encoding a self-cleaving peptide. In some embodiments, a self-cleaving peptide is a self-cleaving P2A (e.g., SEQ ID NO:9), T2A (e.g., SEQ ID NO:10). In some embodiments, a self-cleaving peptide is a self-cleaving P2A peptide. In some embodiments, a self-cleaving peptide comprises the amino acid sequence set forth in any one of SEQ ID NOs:9-10.
EGRGSLLTCGDVEENPGP (SEQ ID NO:9)
ATNFSLLKQAGDVEENPGP (SEQ ID NO:10)

In some embodiments, a self-cleaving peptide has at least about 90% sequence identity to a sequence set forth in any one of SEQ ID NOs:9-10. For example, a self-cleaving peptide has about: 91%, 92%, 93%, 94%, 95%, 96%, 97%, 98% or 99% sequence identity to a sequence set forth in any one of SEQ ID NOs:9-10. In some embodiments, a self-cleaving peptide comprises the amino acid sequence set forth in any one of SEQ ID NO:9-10.

In some embodiments, a self-cleaving peptide comprises at least one amino acid addition, deletion, and/or substitution (e.g., at least one conservative substitution such as highly conservative amino acid substitution), relative to a sequence set forth in any one of SEQ ID NOs:9-10.

In some embodiments, a self-cleaving peptide has at least about 90% sequence identity to a sequence set forth in SEQ ID NO:9-10. For example, a self-cleaving peptide has about: 91%, 92%, 93%, 94%, 95%, 96%, 97%, 98% or 99% sequence identity to a sequence set forth in SEQ ID NO:9-10. In some embodiments, a self-cleaving peptide comprises the amino acid sequence set forth in SEQ ID NO:9-10.

In some embodiments, a self-cleaving peptide has at least about 90% sequence identity to a sequence set forth in SEQ ID NO:9. For example, a self-cleaving peptide has about: 91%, 92%, 93%, 94%, 95%, 96%, 97%, 98% or 99% sequence identity to a sequence set forth in SEQ ID NO:9. In some embodiments, a self-cleaving peptide comprises the amino acid sequence set forth in SEQ ID NO:9.

In some embodiments, a polynucleotide comprises the following elements (e.g., in the following sequence), (a) a chimeric antigen receptor (CAR), wherein the CAR comprises an antigen-binding domain specific for a cell surface antigen, (b) a self-cleaving peptide, and (c) an armoring molecule capable of activating a CD70/CD27 signaling pathway.

In some embodiments, a polypeptide encoded by a polynucleotide comprises the following elements (e.g., in the following sequence), (a) a chimeric antigen receptor (CAR), wherein the CAR comprises an antigen-binding domain specific for a cell surface antigen, (b) an internal ribosome entry site (IRES), and (c) an armoring molecule capable of activating a CD70/CD27 signaling pathway.

In some embodiments, a self-cleaving peptide is a P2A Peptide (e.g., SEQ ID NO:9 or SEQ ID NO:78).

### Vectors

In another aspect, the disclosure provides vectors comprising any one or more of the polynucleotides disclosed herein.

In some embodiments, a vector is a non-viral vector. Non-limiting examples of non-viral vectors include plasmids, bacterial artificial chromosomes (BACs), cosmids, linear artificial chromosomes.

In some embodiments, a vector is a viral vector. Non-limiting examples of viral vectors include adeno-associated virus (AAV) vectors, adenovirus vectors, anellovirus vectors, coronavirus vectors, herpes virus vectors, lentivirus vectors, polyomavirus vectors, rabies virus vectors, recombinant simian virus 40 vectors, reovirus vectors, retrovirus vectors, rhinovirus vectors, sindbis virus vectors, vaccinia virus vectors, vesicular stomatitis virus vectors, semliki forest virus vectors and yellow fever virus vectors. In some embodiments, the viral vector is a moloney murine sarcoma virus (MoMSV), Harvey murine sarcoma virus (HaMuSV), murine mammary tumor virus (MuMTV), gibbon ape leukemia virus (GaLV), feline leukemia virus (FLV), spumavirus, Friend murine leukemia virus, Murine Stem Cell Virus (MSCV) and Rous Sarcoma Virus (RSV)) and lentivirus. Non-limiting examples of lentiviruses include human immunodeficiency virus (e.g., HIV type 1 and HIV type 2), visna-maedi virus (VMV), caprine arthritis-encephalitis virus (CAEV), equine infectious anemia virus (EIAV), feline immunodeficiency virus (FIV), bovine immune deficiency virus (BIV), or simian immunodeficiency virus (SIV) vector.

In some embodiments, a vector is a lentiviral vector (LV), a retroviral vector (RV), or a transposon vector.

In some embodiments, a vector (e.g., a viral vector) is a gene therapy vector.

In some embodiments, a vector is an expression vector.

In some embodiments, a vector (e.g., expression vector) further comprises an expression control polynucleotide sequence operably linked to the polynucleotide, a polynucleotide sequence encoding a selectable marker, or both. In some embodiments, an expression control polynucleotide sequence comprises a promoter sequence, an enhancer sequence, or both. In some embodiments, an expression control polynucleotide sequence comprises an inducible promoter sequence.

In some embodiments, an expression control polynucleotide sequence comprises an EF1α Core Promoter sequence (e.g., SEQ ID NO:61), a MNDU3 Promoter sequence (e.g., SEQ ID NO:62), or a combination thereof. In some embodiments, an expression control polynucleotide sequence comprises an EF1α Core Promoter sequence. In some embodiments, an expression control polynucleotide sequence comprises a MNDU3 Promoter sequence.

GGGCAGAGCGCACATCGCCCACAGTCCCCGAGAAGTTGGGGGGAGGGGTCGGCAATT
GAACCGGTGCCTAGAGAAGGTGGCGCGGGGTAAACTGGGAAAGTGATGTCGTGTACT
GGCTCCGCCTTTTTTCCCGAGGGTGGGGGAGAACCGTATATAAGTGCAGTAGTCGCCG
TGAACGTTCTTTTTCGCAACGGGTTTGCCGCCAGAACACAG(SEQ ID NO:61)
TCGATTAGTCCAATTTGTTAAAGACAGGATATCAGTGGTCCAGGCTCTAGTTTTGACTC
AACAATATCACCAGCTGAAGCCTATAGAGTACGAGCCATAGATAGAATAAAAGATTTTA
TTTAGTCTCCAGAAAAAGGGGGGAATGAAAGACCCCACCTGTAGGTTTGGCAAGCTA
GGATCAAGGTTAGGAACAGAGAGACAGCAGAATATGGGCCAAACAGGATATCTGTGG
TAAGCAGTTCCTGCCCCGGCTCAGGGCCAAGAACAGTTGGAACAGCAGAATATGGGC
CAAACAGGATATCTGTGGTAAGCAGTTCCTGCCCCGGCTCAGGGCCAAGAACAGATG
GTCCCCAGATGCGGTCCCGCCCTCAGCAGTTTCTAGAGAACCATCAGATGTTTCCAGG
GTGCCCCAAGGACCTGAAATGACCCTGTGCCTTATTTGAACTAACCAATCAGTTCGCTT
CTCGCTTCTGTTCGCGCGCTTCTGCTCCCCGAGCTCAATAAAAGAGCCCACAACCCCT
CACTCGGCGCGATC (SEQ ID NO:62)

In some embodiments, an expression control polynucleotide sequence comprises a recombinantly modified promoter sequence.

### Fusion Proteins

In another aspect, the disclosure provides fusion proteins encoded by any one of the polynucleotides or vectors (e.g., expression vectors) disclosed herein.

In another aspect, the disclosure provides polypeptides comprising an amino acid sequence that has at least 90% sequence identity to the amino acid sequence set forth in SEQ ID NO:25, 37, 44 and 63.

In another aspect, the disclosure provides fusion proteins, wherein the fusion proteins comprise: (a) a CAR comprising an antigen-binding domain specific for a cell surface antigen, and (b) an armoring molecule capable of activating a CD70/CD27 signaling pathway. In some embodiments, a fusion protein comprises the following elements (e.g., in the following sequence): (a) a CAR comprising an antigen-binding domain specific for a cell surface antigen, (b) a self-cleaving peptide, and (c) an armoring molecule capable of activating a CD70/CD27 signaling pathway. The CAR can be any one of the CARs described herein, and the armoring molecule can be any one of the armoring molecules described herein. The self-cleaving peptide can be any one of the self-cleaving peptides described herein.

In some embodiments, a fusion protein comprises (a) an anti-DLL3 CAR, an anti-GCC CAR, an anti-CLDN18.2 CAR or an anti-GPC3 CAR, (b) a self-cleaving peptide, and (c) a soluble human CD70 (sCD70).

Fusion proteins disclosed herein can be produced recombinantly or synthetically, using routine methods and reagents that are well known in the art. For example, a fusion protein can be produced recombinantly in a suitable host cell (e.g., bacteria) according to methods known in the art. See, e.g., Current Protocols in Molecular Biology, Second Edition, Ausubel et al. eds., John Wiley & Sons, 1992; and Molecular Cloning: a Laboratory Manual, 2nd edition, Sambrook et al., 1989, Cold Spring Harbor Laboratory Press. In some embodiment, a polynucleotide encoding a fusion protein disclosed herein is introduced and expressed in suitable host cell (e.g., E. coli), and the expressed fusion protein is isolated/purified from the host cell (e.g., in inclusion bodies) using routine methods and readily available reagents. In some embodiment, DNA fragments coding for different protein sequences (e.g., a CAR, a sCD70) are ligated together in-frame in accordance with conventional techniques. In some embodiment, a fusion gene is synthesized by conventional techniques including automated DNA synthesizers. Alternatively, PCR amplification of nucleic acid fragments can be carried out using anchor primers that give rise to complementary overhangs between two consecutive nucleic acid fragments that can subsequently be annealed and re-amplified to generate a chimeric nucleic acid sequence (see Ausubel et al., Current Protocols in Molecular Biology, 1992).

In some embodiments, a fusion protein further comprises a self-cleaving peptide (e.g., a P2A Peptide).

### Host Cells

In another aspect, the disclosure provides host cells comprising any one or more of the polynucleotides or expression vectors disclosed herein.

In some embodiments, a host cell is useful for receiving, maintaining, reproducing and/or amplifying a vector.

Non-limiting examples of expression host cells include mammalian cells such as immune cells (e.g., T lymphocytes, B lymphocytes, NK cells), hybridoma cells, Chinese hamster ovary (CHO) cells, COS cells, human embryonic kidney (HEK), yeast cells such as Pichia pastoris cells, or bacterial cells such as DH5α, etc.

In some embodiments, a polynucleotide is delivered into a host cell using a vector system (e.g., a lentiviral vector (LV), a retroviral vector (RV), or a transposon vector).

The one or more polynucleotides of the disclosure may be introduced into a host cell using physical or chemical methods, for example, by transfection, transformation, or transduction. Many transfection techniques are known in the art and include, for example, calcium phosphate DNA coprecipitation (see, e.g., Murray E.J. (ed.), Methods in Molecular Biology, Vol. 7, Gene Transfer and Expression Protocols, Humana Press (1991)); DEAE-dextran; electroporation; cationic liposome-mediated transfection; tungsten particle-facilitated microparticle bombardment (Johnston, Nature, 346: 776-77 (1990)); and strontium phosphate DNA co- precipitation (Brash et al., Mol. Cell Biol., 7: 2031-34 (1987)). Phage or viral vectors can be introduced into host cells, after growth of infectious particles in suitable packaging cells, many of which are commercially available.

In some embodiments, a retrovirus is used to deliver a polynucleotide disclosed herein into a host cell described herein. Retroviruses are a common tool for gene delivery (Miller, 2000, Nature 357: 455-60). Non-limiting examples of retroviruses suitable for use in particular embodiments include Moloney murine leukemia virus (M-MuLV), Moloney murine sarcoma virus (MoMSV), Harvey murine sarcoma virus (HaMuSV), murine mammary tumor virus (MuMTV), gibbon ape leukemia virus (GaLV), feline leukemia virus (FLV), spumavirus, Friend murine leukemia virus, Murine Stem Cell Virus (MSCV) and Rous Sarcoma Virus (RSV)) and lentivirus. Non-limiting examples of lentiviruses include human immunodeficiency virus (e.g., HIV type 1 and HIV type 2), visna-maedi virus (VMV), caprine arthritis-encephalitis virus (CAEV), equine infectious anemia virus (EIAV), feline immunodeficiency virus (FIV), bovine immune deficiency virus (BIV), and simian immunodeficiency virus (SIV).

In some embodiments, a polynucleotide disclosed herein is integrated into a host cell's genome. In some embodiments, a polynucleotide is inserted into a gene. In some embodiments, a polynucleotide is extrachromosomal in the host cell.

### Immune Effector Cells

In another aspect, the disclosure provides immune effector cells (e.g., T lymphocytes), comprising any one or more of the polynucleotides, expression vectors, or fusion proteins disclosed herein.

In another aspect, the disclosure provides immune effector cells (e.g., T lymphocytes), wherein the immune effector cells comprise a polynucleotide encoding a soluble CD70. In some embodiments, a soluble CD70 binds CD27. In some embodiments, a soluble CD70 triggers CD27 signaling in an immune effector cell. The soluble CD70 can be any one of the soluble CD70 described herein.

In some embodiments, an immune effector cell is a T cell, a natural killer (NK) cell, a natural killer T (NKT) cell, an invariant natural killer T (iNKT) cell, an α β T cell, a γδT cell, a viral-specific T (VST) cell, a cytotoxic T lymphocyte (CTL), or a regulatory T cell (Treg). In some embodiments, an immune effector cell is a lymphocyte (e.g., a T lymphocyte or a natural killer (NK) cell). In some embodiments, an immune effector cell is a NK cell.

In some embodiments, an immune effector cell is a T lymphocyte (e.g., a CD3+, CD4+, CD8+ and/or cytotoxic T lymphocyte (CTL)). A T cell can be any T cell, such as a cultured T cell, e.g., a primary T cell, or a T cell from a cultured T-cell line, or a T cell obtained from a mammal. If obtained from a mammal, a T cell can be obtained from numerous sources, including but not limited to blood, bone marrow, lymph node, the thymus, or other tissues or fluids. T cells can also be enriched for or purified.

In some embodiments, a T cell is a human T-cell (e.g., isolated from a human). A T cell can be of any developmental stage, including but not limited to, a CD4+/CD8+ double positive T cell, a CD4+ helper T cell, e.g., Th, and Th2 cells, a CD8+ T cell (e.g., a cytotoxic T cell), a tumor infiltrating cell, a memory T cell, a naive T cell, and the like. In one embodiment, a T cell is a CD8+ T cell or a CD4+ T cell. T-cell lines are available from, e.g., the American Type Culture Collection (ATCC, Manassas, VA), and the German Collection of Microorganisms and Cell Cultures (DSMZ) and include, for example, Jurkat cells (ATCC TIB-152), Sup-Tl cells (ATCC CRL-1942), RPMI 8402 cells (DSMZ ACC-290), Karpas 45 cells (DSMZ ACC-545), and derivatives thereof.

In some embodiments, an immune effector cell is not genetically modified.

In some embodiments, an immune effector cell is a lymphocyte (e.g., a T lymphocyte) removed from a human patient's tumor. In some embodiments, an immune effector cell is a tumor-infiltrating lymphocyte.

In some embodiments, an immune effector cell (e.g., T lymphocyte) is genetically modified. In some embodiments, the immune effector cell is a TCR-engineered lymphocyte, e.g., to express a T cell receptor (TCR) targeting a tumor antigen. In some embodiments, an immune effector cell is a chimeric antigen receptor (CAR) lymphocyte (e.g., a CAR-T or CAR-NK cell).

In some embodiments, an immune effector cell expresses CD27.

In some embodiments, a polynucleotide encoding a soluble CD70 is integrated into an immune effector cell' s genome.

In some embodiments, a polynucleotide disclosed herein is delivered into an immune effector cell using a vector system.

In some embodiments, a retrovirus is used to deliver a polynucleotide disclosed herein into an immune effector cell (e.g., a T lymphocyte) disclosed herein. Retroviruses are a common tool for gene delivery (Miller, 2000, Nature 357: 455-60). Non-limiting examples of retroviruses suitable for use in particular embodiments include Moloney murine leukemia virus (M-MuLV), Moloney murine sarcoma virus (MoMSV), Harvey murine sarcoma virus (HaMuSV), murine mammary tumor virus (MuMTV), gibbon ape leukemia virus (GaLV), feline leukemia virus (FLV), spumavirus, Friend murine leukemia virus, Murine Stem Cell Virus (MSCV) and Rous Sarcoma Virus (RSV)) and lentivirus. Non-limiting examples of lentiviruses include human immunodeficiency virus (e.g., HIV type 1 and HIV type 2), visna-maedi virus (VMV), caprine arthritis-encephalitis virus (CAEV), equine infectious anemia virus (EIAV), feline immunodeficiency virus (FIV), bovine immune deficiency virus (BIV), and simian immunodeficiency virus (SIV).

In some embodiments, a polynucleotide disclosed herein is integrated into an immune effector cell' s genome. In some embodiments, a polynucleotide is inserted into a gene. In some embodiments, a polynucleotide is extrachromosomal in the immune effector cell.

In some embodiments, an immune effector cell (e.g., a T lymphocyte) disclosed herein is maintained with the use of cytokines such as, for example, IL-2, IL-4, IL-7, IL-9, IL-15 and/or IL-21.

In another aspect, the disclosure provides immune effector cells (e.g., T lymphocytes), wherein the immune effector cells express a CAR comprising an antigen-binding domain specific for a cell surface antigen, and wherein the immune effector cells secret a recombinant armoring molecule capable of activating a CD70/CD27 signaling pathway.

In another aspect, the disclosure provides immune effector cells (e.g., T lymphocytes), wherein the immune effector cells express: (a) a CAR comprising an antigen-binding domain specific for a cell surface antigen, and (b) a recombinant armoring molecule capable of activating a CD70/CD27 signaling pathway. In some embodiments, an immune effector cell expresses (a) the CAR, (b) a self-cleaving peptide, and (b) the recombinant armoring molecule.

The CAR can be any one of the CARs described herein, and the recombinant armoring molecule can be any one of the armoring molecules described herein. The self-cleaving peptide can be any one of the self-cleaving peptides described herein.

Immune effector cells (e.g., T lymphocytes) disclosed herein can be contacted with a population of cancer cells (e.g., solid tumor cells) ex vivo, in vivo, or in vitro. For example, Immune effector cells (e.g., T lymphocytes) can be cultured ex vivo under conditions to express the CAR and sCD70, and then directly transferred into a subject (e.g., a mammal such as a human) affected by cancer (e.g., a solid tumor). Such a cell transfer method is referred to in the art as "adoptive cell transfer (ACT)," in which immune-derived cells are passively transferred into a new recipient host to transfer the functionality of the donor immune-derived cells to the new host. Adoptive cell transfer methods to treat various types of cancers are known in the art and disclosed in, for example, Gattinoni et al., Nat. Rev. Immunol, 6(5): 383-93 (2006); June, J. Clin. Invest., 117(6): 1466-76 (2007); Rapoport et al., Blood, 117(3): 788-97 (2011); and Barber et al., Gene Therapy, 18: 509-16 (2011)).

Immune effector cells (e.g., T lymphocytes) disclosed herein may be introduced into a mammal, e.g., a human, using a variety of techniques and reagents known to those of skill in the art. In some embodiments, immune effector cells (e.g., T lymphocytes) are introduced at the site of the tumor. In some embodiments, immune effector cells (e.g., T lymphocytes) are modified to hone to the cancer. The number of cells that are employed will depend upon circumstances, such as the purpose for the introduction, the lifetime of the immune effector cells (e.g., T lymphocytes), the number of administrations, etc.

### Compositions, Pharmaceutical Compositions, and Kits

In another aspect, the disclosure provides compositions comprising any one or more of the polynucleotides, vectors, fusion proteins, host cells, or immune effector cells (e.g., T lymphocytes) disclosed herein. In some embodiments, a composition comprises any one or more of the immune effector cells (e.g., T lymphocytes) described herein.

In another aspect, the disclosure provides pharmaceutical compositions comprising any one or more of the compositions described herein and a pharmaceutically acceptable carrier, excipient, stabilizer, diluent or tonifier.

In some embodiments, a composition or pharmaceutical composition further comprises a cryopreservation medium comprising about 2%, about 5%, or about 10% dimethyl sulfoxide (DMSO), wherein the cryopreservation medium is substantially free of serum.

In some embodiments, a composition or pharmaceutical composition is in a storage vial. Suitable pharmaceutically acceptable carriers, excipients, or stabilizers are nontoxic to recipients at the dosages and concentrations employed (Remington's Pharmaceutical Sciences 16th edition, Osol, A. Ed. (1980)). Non-limiting examples of pharmaceutically acceptable carriers, excipients, stabilizers, diluents or tonifiers include buffers (e.g., phosphate, citrate, histidine), antioxidants (e.g., ascorbic acid or methionine), preservatives, proteins (e.g., serum albumin, gelatin or immunoglobulins); hydrophilic polymers, amino acids, carbohydrates (e.g., monosaccharides, disaccharides, glucose, mannose or dextrins); chelating agents (e.g., EDTA), sugars (e.g., sucrose, mannitol, trehalose or sorbitol), salt-forming counter-ions (e.g., sodium), metal complexes (e.g., Zn-protein complexes); non-ionic surfactants (e.g., Tween), PLURONICS^{™} and polyethylene glycol (PEG).

In some embodiments, a composition (e.g., a pharmaceutical composition) disclosed herein is formulated for a suitable administration schedule and route. Non-limiting examples of administration routes include oral, rectal, mucosal, intravenous, intramuscular, subcutaneous and topical, etc. In some embodiments, a composition (e.g., a pharmaceutical composition) is stored in the form of an aqueous solution or a dried formulation (e.g., lyophilized).

In some embodiments, a composition (e.g., a pharmaceutical composition) is formulated to be administered by infusion (e.g., intracranial ventricular injection, intracranial infusion or intravenous infusion).

In some embodiments, a composition (e.g., a pharmaceutical composition) is formulated to be administered with a second therapeutic agent as a combination therapy.

In another aspect, the disclosure provides kits, wherein the kits comprise a container and, optionally, an instruction for use, wherein the container comprises any one or more of the compositions or pharmaceutical compositions described herein.

### Methods of Use

In another aspect, the disclosure provides use of any one or more of the polynucleotides, vectors, fusion proteins, host cells, immune effector cells (e.g., T lymphocytes), compositions (e.g., pharmaceutical compositions), or kits disclosed herein for the preparation of a medicament for treating cancer in a subject in need thereof.

In another aspect, the disclosure provides use of any one or more of the immune effector cells (e.g., T lymphocytes), compositions (e.g., pharmaceutical compositions), or kits described herein for the preparation of a medicament for treating cancer (e.g., a solid tumor) in a subject in need thereof.

In another aspect, the disclosure provides any one or more of the polynucleotides, vectors, fusion proteins, host cells, immune effector cells (e.g., T lymphocytes), compositions (e.g., pharmaceutical compositions), or kits disclosed herein for use in treating cancer in a subject in need thereof.

In another aspect, the disclosure provides any one or more of the immune effector cells (e.g., T lymphocytes), compositions (e.g., pharmaceutical compositions), or kits described herein for use in treating cancer in a subject in need thereof.

In another aspect, the disclosure provides methods of treating a cancer in a subject in need thereof, comprising administering to the subject an effective dosage of any one or more of the immune effector cells (e.g., T lymphocytes), compositions, or pharmaceutical compositions disclosed herein.

In another aspect, the disclosure provides methods of inducing cytolysis of cancer cells, comprising contacting the cancer cells with any one or more of the immune effector cells (e.g., T lymphocytes), compositions, or pharmaceutical compositions disclosed herein.

In some embodiments, a cancer is hepatocellular carcinoma, a liver cancer, an embryonal sarcoma, a rhabdoid tumor, Wilms tumor, a choriocarcinoma, or a yolk sac tumor.

In some embodiments, a cancer is a solid tumor, e.g., breast, lung, prostate, colon, bladder, ovary, kidney, stomach, colon, rectum, testes, head and/or neck, pancreas, brain, or skin cancer. Accordingly, in some embodiments, the cancer is a solid tumor cell selected from breast, lung, prostate, colon, bladder, ovarian, renal, gastric, rectal, colorectal, testicular, head and neck, pancreatic, brain and skin cancer.

In some embodiments, a cancer is a liver cancer, pancreatic cancer, ovarian cancer, lung cancer, or mesothelioma. In some embodiments, a liver cancer is hepatocellular carcinoma (HCC), hepatoblastoma, hemangioma, hepatic adenoma, focal nodular hyperplasia. In some embodiments, a liver cancer is HCC.

In some embodiments, a cancer is a hematologic cancer, for example, leukemia, lymphoma, or myeloma. Hematologic cancers that can be treated according to the methods disclosed herein include leukemias (e.g., acute leukemias, chronic leukemias), lymphomas (e.g., B-cell lymphoma, T-cell lymphoma) and multiple myeloma. Accordingly, in some embodiments, the cancer is a hematologic cancer cell selected from leukemia (e.g., acute leukemias, chronic leukemias), lymphoma (e.g., B-cell lymphoma, T-cell lymphoma) and multiple myeloma.

In some embodiments, a cancer is lymphoma, leukemia, or myeloma.

In some embodiments, a solid tumor is a brain tumor, breast cancer, lung cancer or liver cancer. In some embodiments, a liver cancer is hepatocellular carcinoma (HCC).

In some embodiments, a human subject is a female. In some embodiments, a human subject is a male.

In some embodiments, a human subject is an infant (less than 1 year old). In some embodiments, a human subject is less than 11 years old. In some embodiments, a human subject is 11 years or older. In some embodiments, a human subject is 12 years or older. In some embodiments, a human subject is 12-17 years old. In some embodiments, a human subject is less than 18 years old. In some embodiments, a human subject is an adult (18 years or older). In some embodiments, a human subject is 40 years or older, e.g., at least: 45, 50, 55, 60, 65, 70, 75, 80, 85, 90 years old. In some embodiments, a human subject is elderly (65 years or older). In some embodiments, a human subject is 18 years or older.

In some embodiments, a human subject is between the ages of 18-95 years, for example, 18-85 years, 18-75 years, 18-65 years, 18-55 years, 55-95 years, 55-85 years, 55-75 years, 55-65 years, 65-95 years, 65-85 years, 65-75 years, 75-95 years or 75-85 years. In some embodiments, a human subject is between the ages of 2-12 years, for example, 2-10 years, 2-8 years, 2-6 years, 2-5 years, 2-4 years, 4-12 years, 4-10 years, 4-8 years, 4-6 years, 4-5 years, 5-12 years, 5-10 years, 5-8 years, 5-6 years, 6-12 years, 6-10 years, 6-8 years, 8-12 years, or 8-10 years. In some embodiments, a human subject is between the ages of 6 months to 17 years, for example, 6 months to 16 years, 6 months to 14 years, 6 months to 12 years, 6 months to 10 years, 6 months to 8 years, 6 months to 6 years, 6 months to 4 years, 6 months to 2 years, 1-17 years, 1-16 years, 1-14 years, 1-12 years, 1-10 years, 1-8 years, 1-6 years, 1-4 years, 1-2 years, 2-17 years, 2-16 years, 2-14 years, 2-12 years, 2-10 years, 2-8 years, 2-6 years, 2-4 years, 4-17 years, 4-16 years, 4-14 years, 4-12 years, 4-10 years, 4-8 years, 4-6 years, 6-17 years, 6-16 years, 6-14 years, 6-12 years, 6-10 years, 6-8 years, 8-17 years, 8-16 years, 8-14 years, 8-12 years, 8-10 years, 10-17 years, 10-16 years, 10-14 years, 10-12 years, 12-17 years, 12-16 years, 12-14 years, 14-17 years, or 14-16 years.

In some embodiments, a human subject is ≥6 months, for example, ≥7 months, ≥8 months, ≥9 months, ≥ 10 months, ≥ 11 months, ≥ 1 year, ≥2 years, ≥3 years, ≥4 years, ≥5 years, ≥6 years, ≥7 years, ≥ 8 years, ≥9 years, ≥ 10 years, ≥ 11 years, ≥ 12 years, ≥ 13 years, ≥ 14 years, ≥15 years, ≥16 years, ≥17 years, or ≥18 years. In some embodiments, a human subject is ≥2 years. In some embodiments, a human subject is ≥ 12 years. In some embodiments, a human (e.g., a healthy human donor) is ≥ 18 years.

In some embodiments, a human subject is about 6 months to about 17 years of age or is about 18 years or older.

A subject to be treated according to the methods disclosed herein may be one who has been diagnosed with a particular condition, or one at risk of developing such conditions. Diagnosis may be performed by any method or technique known in the art. One skilled in the art will understand that a subject to be treated according to the present disclosure may have been subjected to standard tests or may have been identified, without examination, as one at risk due to the presence of one or more risk factors associated with the disease or condition.

In some embodiments, a mammalian subject has cancer.

In some embodiments, a cancer is a hematologic cancer. In some embodiments, a cancer is a hematologic cancer and a CAR is capable of binding to (e.g., targets) CD19, CD20, CD22, CD30, CD33, CD123, CD138, BCMA, or a combination thereof.

In some embodiments, a hematologic cancer is leukemia.

In some embodiments, a leukemia is selected from acute lymphoblastic leukemia (ALL), acute myeloid leukemia (AML), chronic lymphocytic leukemia (CLL), chronic myeloid leukemia (CML), hairy cell leukemia (HCL), myelodysplastic syndromes (MDS), and combinations thereof.

In some embodiments, a hematologic cancer comprises lymphoma.

In some embodiments, a lymphoma comprises Hodgkin lymphoma.

In some embodiments, a Hodgkin lymphoma is selected from nodular sclerosis Hodgkin lymphoma (NSCHL), mixed cellularity Hodgkin lymphoma (MCcHL), lymphocyte-rich Hodgkin' s disease (LRCHL), lymphocyte-depleted Hodgkin' s disease (LDHL), and combinations thereof.

In some embodiments, a lymphoma comprises non-Hodgkin lymphoma (NHL).

In some embodiments, a non-Hodgkin lymphoma comprises a B cell lymphoma.

In some embodiments, a B cell lymphoma is selected from diffuse large B-cell lymphoma (DLBCL), primary mediastinal B cell lymphoma (PMBCL), follicular lymphoma (FL), small lymphocytic lymphoma (SLL), marginal zone lymphoma (MZL), mantle cell lymphoma (MCL), Waldenström' s macroglobulinemia (WMG), Burkitt lymphoma (BL), and combinations thereof.

In some embodiments, a non-Hodgkin lymphoma comprises a T cell lymphoma.

In some embodiments, a T cell lymphoma is selected from peripheral T-cell lymphoma (PTCL), anaplastic large cell lymphoma (ALCL), angioimmunoblastic T-cell lymphoma (AITL), cutaneous T cell lymphoma, and combinations thereof.

In some embodiments, a hematologic cancer comprises multiple myeloma.

In some embodiments, a multiple myeloma is selected from light chain multiple myeloma (LCMM), non-secretory multiple myeloma (NSMM), solitary plasmacytoma (SP), extramedullary plasmacytoma (EMP), monoclonal gammopathy of undetermined significance (MGUS), smoldering Multiple Myeloma (SMM), Immunoglobulin D multiple myeloma (IgD MM), Immunoglobulin E (IgE) multiple myeloma, and combinations thereof.

In some embodiments, a cancer is a solid tumor.

In some embodiments, a solid tumor is a tumor of the breast, lung, prostate, colon, bladder, ovary, kidney, stomach, colon, rectum, testes, head and/or neck, pancreas, brain, skin, or a combination thereof.

In some embodiments, a solid tumor is selected from bladder cancer, brain cancer, breast cancer, cervical cancer, colon cancer, colorectal cancer, fallopian tube cancer, gastric cancer, genitourinary cancer, head and neck cancer, liver cancer, lung cancer, melanoma, nasopharyngeal carcinoma (NPC), pancreatic cancer, prostate cancer, ovarian cancer, rectal cancer, renal cancer, skin cancer, stomach cancer, testicular cancer, thyroid cancer, urethral cancer, and combinations thereof.

In some embodiments, a solid tumor is selected from breast cancer, squamous non-small cell lung cancer (NSCLC), non-squamous NSCLC, lung adenocarcinoma, mesothelioma, kidney clear cell carcinoma, kidney papillary cell carcinoma, hepatocellular carcinoma (HCC), castration-resistant prostate cancer, squamous cell carcinoma of the head and neck, carcinomas of the esophagus, carcinomas of the gastrointestinal tract, endometriosis, and combinations thereof. In some embodiments, a solid tumor is selected from breast cancer, squamous non-small cell lung cancer (NSCLC), non-squamous NSCLC, lung adenocarcinoma, hepatocellular carcinoma (HCC), and combinations thereof. In some embodiments, a solid tumor is breast cancer. In some embodiments, a solid tumor is NSCLC. In some embodiments, a solid tumor is lung adenocarcinoma. In some embodiments, a solid tumor is mesothelioma. In some embodiments, a solid tumor is HCC.

In some embodiments, a solid tumor is a metastatic lesion of the cancer.

In some embodiments, a cancer is a glioblastoma (GBM), breast cancer, or lung cancer. In some embodiments, a cancer is GBM. In some embodiments, a cancer is breast cancer. In some embodiments, a breast cancer is HER2-positive breast cancer. In some embodiments, a cancer is lung cancer. In some embodiments, a lung cancer is brain metastatic lung cancer.

In some embodiments, a subject is newly diagnosed with a solid tumor (e.g., HCC). In some embodiments, a subject has relapsed from or is refractory to a prior cancer therapy.

In some embodiments, a cancer is a cancer (e.g., tumor) that expresses (e.g., overexpresses) glypican 3 (GPC3). Non-limiting examples of GPC3-positive cancers including breast cancer, CNS tumors (e.g., astrocytoma, atypical teratoid rhabdoid tumor, ependymoma, glioblastoma, meningioma, neuroblastoma, or oligodendroglioma), thyroid cancer (e.g., anaplastic, follicular, medullary, or papillary thyroid cancer), gastrointestinal cancer (anal squamous cell carcinoma (SCC), cholangiocarcinoma, colorectal adenocarcinoma, esophageal adenocarcinoma, esophageal SCC, gallbladder cancer, gastric adenocarcinoma, HCC, combined hepatocellular cholangiocarcinoma (CC), fibrolamellar hepatocellular carcinoma (FL-HCC), hepatoblastoma, pancreatic adenocarcinoma, or pancreatic cancer, small-bowel cancer), gynecological cancer (e.g., cervical SCC, endometrioid, endometrioid adenocarcinoma, endometrioid serous carcinoma, ovarian clear cell cancer, ovarian endometrioid cancer, or ovarian serous cancer), lung cancer (e.g., adenocarcinoma, large-cell carcinoma, mesothelioma, SCC, or small-cell carcinoma), skin cancer (e.g., basal cell carcinoma or melanoma), sarcoma (e.g., epithelioid sarcoma, Ewing sarcoma, fibrosarcoma, leiomyosarcoma, rhabdomyosarcoma, synovial sarcoma, or undifferentiated sarcoma), urogenital cancer (e.g., bladder cancer, prostate cancer, or urothelial cancer), renal cancer (e.g., chromophobe, clear cell, oncocytoma, papillary, or Wilms tumor), germ cell tumors (e.g., dysgerminomas, extragonadal yolk sac tumors (YST), nondysgerminomas, nonseminomas, or seminomas), malignant rhabdoid tumor, neuroendocrine tumors (NETs), neuroendocrine carcinoma (NEC), and salivary gland tumor.

In some embodiments, an immune effector cell (e.g., a CAR-T cell) for treating a subject is autologous. In some embodiments, an immune effector cell (e.g., a CAR-T cell) for treating a subject is from a genetically similar, but non-identical donor (allogeneic). In some embodiments, an immune effector cell (e.g., a CAR-T cell) for treating a subject is syngeneic.

In some embodiments, an immune effector cell (e.g., an allogeneic CAR-T cell) is a virus-specific T cell. In some embodiments, an immune effector cell (e.g., an allogeneic CAR-T cell) specifically recognizes BK virus, human herpesvirus 6, cytomegalovirus, Epstein-Barr virus, hepatitis B virus, hepatitis C virus, or adenovirus.

CAR-modified immune effector cells disclosed herein, such as CAR-T cells, may be administered alone or as a pharmaceutical composition with a diluent and/or other components (e.g., associated with cytokines or cell populations). In some embodiments, a pharmaceutical composition comprises, for example, an immune effector cell (e.g., a CAR-T cell) disclosed herein, with one or more pharmaceutically or physiologically acceptable carrier, diluent, and/or excipient. In some embodiments, a pharmaceutical composition comprises one or more buffers (e.g., neutral buffered saline, buffered saline), sulfates, carbohydrates (e.g., glucose, mannose, sucrose, dextrans, or mannitol), proteins, polypeptides, and/or amino acids (e.g., glycine), antioxidants, chelating agents (e.g., EDTA or glutathione), adjuvants (e.g., aluminum hydroxide), preservatives, or a combination thereof. In some embodiments, a pharmaceutical composition disclosed herein is adapted to the treatment (or prophylaxis).

The number of CAR cells (e.g., CAR-T cells) administered per dose, the number of doses, and frequency of dosing will depend on various parameters such as the subject' s age, weight, clinical assessment, tumor type, tumor burden, and other factors (e.g., judgment of a physician). Any acceptable route of administration is contemplated, for example, intravenous (e.g., intravenous infusion), parenteral, or subcutaneous routes of administration.

### Exemplification

CAR-T therapy has not achieved a significant antitumor efficacy in treatment of solid tumors. One of the major hurdles is that solid tumors maintain a hostile microenvironment where CAR-T cells hardly expand or persist to elicit sustained antitumor activity.

As described in Examples 1-6, a novel armored anti-glypican 3 CAR-T that co-expresses soluble CD70 (sCD70) was designed and developed for treatment of hepatocellular carcinoma. Without wishing to be bound by theory, the secreted sCD70 increases durable anti-tumor effects of the CAR-T cells by increasing CD70/CD27 signaling in an autocrine and paracrine manner. The data described in Examples 1-6 demonstrates that sCD70-armored anti-glypican CAR-T cells expressed anti-glypican CAR molecules on T cell surface and secreted soluble CD70 into culture medium. The sCD70-armored anti-glypican-3 CAR-T cells displayed enhanced expansion and more persistent cytotoxicity against glypican-3-expressing tumor cells. Moreover, the sCD70-armored anti-glypican-3 CAR-T cells generated more durable antitumor activity in a xenograft NSG model of hepatocellular carcinoma.

### Example 1. Material and Methods

### Cell Lines

293T (human embryonic kidney) cell line was purchased from Takara Bio USA, Inc. (San Jose, CA), and hepatocellular carcinoma cell lines HepG2, Hep3B, Huh-7 and SK-Hep1 were purchased from the American Type Culture Collection (ATCC, Manassas, VA). The cell lines were grown in RPMI or DMEM (HyClone Laboratories, GE HealthCare, Chicago, IL) with 10% FBS (HyClone Laboratories) and 2 mmol/L GlutaMAX-I (Invitrogen, Waltham, MA). All cell lines were tested on a regular basis for mycoplasma and were negative.

Generation of Lentiviral Vectors Encoding GC33 CAR and sCD70.

A construct encoding GC33 CAR, a glypican-3-specific CAR with a short CD8 hinge region, a CD8 transmembrane domain, and a 4-1BB/CD3ζ endodomain (FIG. 11A, the sequence of CAR is shown in SEQ ID NO.77), was generated as described in the previously reported method (Batr SA, Rathi P, Guo L, Courtney AN, Fleurence J, et al. 2020. Glypican-3-specific CAR T cells coexpressing IL15 and IL21 have superior expansion and antitumor activity against hepatocellular carcinoma. Cancer immune Res 8(3): 309-320). A construct encoding GC33-sCD70 CAR, a sCD70-armored GC33BBz, was generated by linking: (a) GC33 CAR, (b) a P2A self-cleaving peptide, (c) a 5' - leader sequence of CD8, and (d) a full-length human CD70 extracellular domain (FIG. 11A, the sequence of GC33-sCD70 CAR is shown in SEQ ID NO.63).

Recombinant lentiviral vector particles were generated by co-transfection of 293T cells using a transfer vector containing the GC33 CAR or GC33-sCD70 CAR construct, along with lentiviral packing plasmids and a envelop plasmid as described (8). Supernatants containing the lentiviral particles were collected 48 hours after transfection as described in the previously reported method (Scholler J, Brady TL, Binder-Scholl G, Hwang WT, Plesa G, et al. 2012. Decade-long safety and function of retroviral-modified chimeric antigen receptor T cells. Sci. Transl. Med 4:132ra53), and 50-fold concentrated using a one-step sucrose (10-20%) cushion centrifugation. Concentrated viruses were frozen at -80°C.

### Generation of CAR-T Cells

Fresh human peripheral blood leukopak (STEMCELL Technologies, Vancouver, Canada) was used to isolate T cells using EasySep Human T Cell Isolation Kit (STEMCELL Technologies) following the manufacturer' s instruction. To generate GC33 CAR-T or GC33BBz-sCD70 CAR-T cells, isolated T cells were activated via Human T Cell TransAct (Miltenyi Biotec, Bergisch Gladbach, Germany) (1:100) for 48 hours. Activated T cells were suspended in T-cell culture medium (X-VIVO Medium, Lanza, Santa Monica, CA) supplemented with polybrene (10ng/ml), seeded into a 24-well plate (0.5-1x106/well), added with the lentiviral particles (moi >5), and then centrifuged at 1,200g for 1.5 hours at 32°C. The transduced T cells were incubated overnight at 37°C, transferred into 6 ml T-cell culture medium supplemented with 200U IL-2 or 10ng/ml IL-7 and 10ng/ml IL15 in a well in a 6-well G-Rex plate (Wilson Wolf, New Brighton, MN), and cultured for an additional week.

### Flow Cytometry

The Attune NXT Flow Cytometer and Attune Cytometric Software (Thermo Fisher Scientific, Waltham, MA) were used to acquire and analyze immunofluorescence data, respectively. FlowJo10.8.0 (FlowJo, LLC, Ashland, OR) was used for final data analysis and graphic representation. GC33 CAR expression was detected by staining T cells with Fluorescein-5-isothiocyanate (FITC)-conjugated recombinant glypican-3 protein (ACROBiosystems, Newark, DE) or FITC-conjugated goat-anti-human IgG, F(ab' )2 (Jackson ImmunoResearch Laboratories, Inc., West Grove, PA). T cell phenotype was evaluated by staining T cells with CD4-Pacific Blue, CD8-PerCP, CC62L-FITC, and CD45RA-AF750 (BD Biosciences, Franklin Lakes, NJ). Forward and side scatter gating were used to distinguish normal lymphocytes. For immune staining, cells were collected and washed once with PBS (Sigma-Aldrich, St. Louis, MO) containing 1% FBS (HyClone Laboratories), and then incubated with the indicated antibodies for 30 minutes on ice in the dark, washed once, and resuspend in PBS (BD Biosciences).

### sCD70 Release Assay

The CAR-T cells were expanded in T-cell culture media supplemented with 200U/ml IL-2 or 10ng/ml IL-7 and 10ng/ml IL-15 for 5-6 days. The CAR-T cells were harvested and resuspended into fresh T-cell culture media and cultured for an additional 24 hours. The supernatants were collected, the total T cells were counted, and the CAR⁺ T cells were assessed via immune staining of FITC-conjugated goat-anti-human IgG, F(ab')2. The collected supernatants were used to test the secretion of sCD70 via the Human CD27 Ligand/TNFSF7 ELISA kit (Invitrogen). The value represents the amount that secreted from 2x10⁶ CAR+ T cells/ml.

### RTCA-Based Cytotoxicity Assay

For real-time cell analysis (RTCA), adherent target cells were seeded onto 96-well E-Plates (ACEA Biosciences, San Diego, CA) at a cell density of 2 × 10⁴ cells per well in 100µl of appropriate medium. Target cells were monitored in an incubator (5% CO₂, 95% humidity) at 37°C for 24 hours with the xCELLigence RTCA system (ACEA Biosciences), which uses impedance-based technology. Next, the medium was removed and replaced with T-cell culture medium containing control or CAR-modified effector cells at various E:T ratios. To control for differences in transduction efficiency, non-transduced T cells were supplemented to ensure that both the number of CAR+ T cells and total number of T cells remained constant across CAR-T cell groups. The cells in the E-Plates were monitored with the RTCA system for the next 72-250 hours. Analysis of the results was performed using RTCA Software Pro (ACEA Biosciences). The impedance changes were normalized to the baseline (at effector cell addition) and plotted over time as cytotoxicity.

### Monitoring Long-Term CAR-T Function

Adherent target cells (HepG2 or Hep3B) were seeded in a 6-well plate at 5x10⁵ per well (in 1 ml of appropriate medium) and incubated overnight. The plate was X-irradiated with 100µCi, and 2.5x10⁵ CAR-T cells were added in T-cell culture medium supplemented with 20U/ml IL-2. After 3-4 days of co-culturing, an aliquot of CAR-T cells was analyzed for cytotoxicity against Hep3B via RTCA-based cytotoxicity assay. The remaining CAR-T cells were counted and seeded into a fresh target cell-coated 6-well plate (2.5x10⁵ cells/well) and cultured for another 3-4 days before testing cytotoxicity against Hep3B via the RTCA assay. The procedure was repeated every 3-4 days until the CAR-T cells were continuously cocultured with the target tumor cells in the 6-well plate for a total of 18 days.

### Animal Studies

All animal experiments were performed in accordance with a protocol approved by Simnova Biotherapeutic Institutional Animal Care and Use Committee. Six to 12 week-old female NSG (NOD.Cg-Prkdcscid Il2rgtm1Wjl/SzJ) mice (The Jackson Laboratory, Bar Harbor, ME) were used. Hep3B hepatocellular carcinoma cells were subcutaneously inoculated into the NSG mice (2x10⁶ cells per mouse). The tumor-bearing mice were randomly grouped before receiving adoptive transfer of 1x10⁶ transduced CAR+ T cells intravenously 12 days after inoculation (as indicated in individual experiments). Hep3B carcinoma burden was evaluated weekly using the Xenogen IVIS Lumina imaging system (Caliper Life Sciences, Hopkinton, MA). The mice were injected intraperitoneally with 3 mg d-luciferin (Caliper Life Sciences) and imaged 20 minutes post luciferin administration with multiple exposure time (1 second Bin4, 1second Bin1, 5 second Bin4 and 10 second Bin4). Luminescence images were analyzed using the Living Image software (Caliper Life Sciences).

### Persistence of CAR-T Cells

CAR-T cell treated NSG mice were bled at the indicated days, and isolated leukocytes were analyzed via immune staining. Mouse CD45⁻ human CD3⁺ T cells were gated by FACS.

### Statistical analysis

The data were presented as mean SD or SEM, and the two-sided unpaired t test was used for comparison of two groups, as stated in Brief Description of Drawings. Statistical significance was defined at P < 0.05. Statistical analysis was performed with Prism 9 (GraphPad Software).

### Example 2. Generation & Characterization of sCD70-Armored GC33 CAR-T Cells.

To develop CAR-T therapies targeting GPC-3-expressing solid tumors, a second-generation GPC-3-specific CAR, termed as GC33CAR, was designed, incorporating the single chain variable fragment (scFv) derived from the monoclonal antibody GC33, the CD3ζ intracellular stimulatory domain, and the 4-1BB costimulatory domain. To generate a sCD70 armored GC33 CAR, termed GC33-sCD70 CAR, full-length human CD70 ectodomain was linked to the C-terminus of GC33CAR via a P2A self-cleaving peptide (FIG. 11A). GC33CAR and GC33-sCD70CAR were cloned into a proprietary lentiviral (LV) vector SR19 or SBIC to generate recombinant lentiviral particles, LV-GC33CAR and LV-GC33-sCD70CAR, respectively. Human peripheral blood T cells that were transduced with concentrated LV-GC33CAR or LV-GC33-sCD70CAR expressed similar percentages of GC33CAR (63.2% vs. 64.8%) on the cell surface (FIG. 11B), and CD70 was only detected in the supernatants of human peripheral blood T cells transduced with LV-GC33-sCD70CAR particle (FIG. 11C). The CD70 concentration was about 50ng/ml from about 2x10⁶ GC33CAR+ T cells. The results demonstrated that sCD70-armored GC33 CAR-T cells express GC33CAR on the cell surface and secrets sCD70.

### Example 3. GC33-sCD70 CAR-T Retained Potent and Durable Cytotoxicity Against HCC Cell Lines After Antigen Exposure In Vitro.

An in vitro killing assay was performed to analyze cytotoxicity of the transduced CAR-T cells against hepatocellular carcinoma cell lines that expressed various levels of glypican-3. Human peripheral blood T cells were transduced with recombinant LV-GC33 CAR or LV-GC33-sCD70 CAR. One week after transduction, T cells were harvested and analyzed with short-term real-time cell analysis (RTCA) (about 72 or 120 hours). GC33-sCD70 CAR-T showed a similar level of cytotoxicity against target cell HepG2 (FIG. 12A) or Hep3B (FIG. 12D), but a slightly inferior level of cytotoxicity against target cell Huh7 at the various E/T ratios in comparison with GC33 CAR-T (FIG. 12B). Huh7 expresses a lower intensity of GPC-3 on a single cell lever than HepG2 or Hep3B does. Both GC33-sCD70 CAR-T and GC33 CAR-T killed the HCC lines, which is tumor antigen GPC-3-specific, as either of them did not kill HCC line SK-Hep1B that does not express GPC-3 (Fig. 12C).

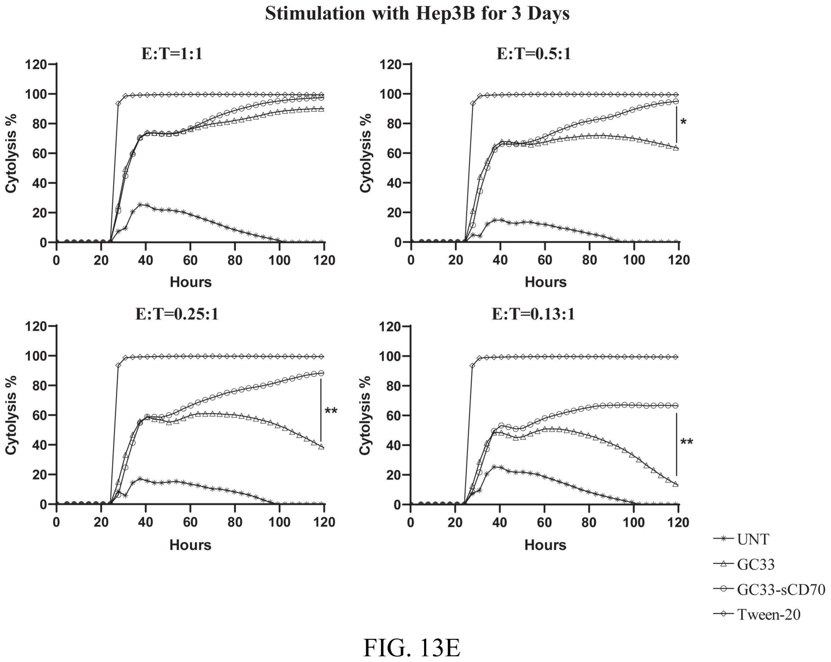

An in vitro protocol for monitoring impact of tumor chronic stimulation on CAR-T cytotoxicity was developed (FIG. 13A). Briefly, T cells transduced with recombinant LV-GC33 CAR or LV-GC33-sCD70 CAR were repetitively exposed to irradiated HCC cell line HepG2 (FIGs. 13B-13D) or Hep3B (FIGs. 13E-13G) at the ratio=0.5:1 in the presence of low dosage IL-2 (20U/ml) for continuous stimulation of tumor antigen. At day 3, 11, or 18 during the continuous exposure, an aliquot of the T cells was collected. After GC33CAR expression was defined via immune staining/FACS (data not shown), the CAR-T cells were analyzed via RTCA for killing activity against Hep3B.

Despite a comparable or slightly lower level of cell surface expression of GC33CAR at the earlier time points, GC33-sCD70CAR-T cells showed more potent and more durable cytotoxicity against Hep3B than GC33 CAR-T cells did at the various E/T ratios and time points (FIGs. 13B-13G). The HepG2-stimulated CAR-T cells (FIGs. 13B-13D) and the Hep3B-stimulated CAR-T cells (FIGs. 13E-13G) displayed different patterns in cytolyzing Hep3B. The differences may be due to Hep3B and HepG2 having medium and high levels of GPC3 on the cell surfaces, respectively. Together, the results show that sCD70-armored GC33 CAR-T resists tumor chronic stimulation, retaining more potent and more durable cytotoxicity against GPC-3-expressing hepatocellular carcinoma cell lines.

### Example 4. GC33-sCD70 CAR-T Retained a Central Memory Phenotype.

To test if armoring GC33-CAR with sCD70 enhances expansion and memory phenotypes of the CAR-T cells, the following experiment was repeated. The CAR-T cells were repetitively exposed to irradiated HCC lines, as described above, and phenotypic analyses of the tumor antigen-stimulated CAR-T cells were performed. As shown in FIG. 14A, T cells were transduced with recombinant LV-GC33 CAR or LV-GC33-sCD70 CAR. After expansion with IL-7 and IL-15 for one-week, large fractions of the T cells (92.7% vs. 81.5%) were GC33-CAR+, indicating efficient transduction and maintenance of the CAR-T cells. After coculturing with irradiated Hep3B, the percentage of GC33-CAR+ T cells from the LV-GC33 CAR transduction was relatively stable at first, at 86.4% and 89.3% after three and 15 days of co-culturing, respectively. However, after 22 days of co-culturing, only 71.4% of T cells were GC33CAR+, exhibiting a significant drop. In contrast, the percentage of GC33CAR+ T cells from LV-GC33-sCD70 CAR transduction increased steadily, at 86.5% and 93.6% after three and 15 days of co-culturing, respectively. Even after 22 days of co-culturing, the percentage of GC33-sCD70 CAR+ T cells was maintained at about 93.3%. The results suggest that co-expression of sCD70 confers a survival advantage to CAR-T cells, particularly under continuous stimulation of tumor antigen. 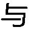

The memory phenotypes of the CAR-T cells were also analyzed. Prior to exposure to the tumor antigen, 11.7% of GC33 CAR-T cells were CD45RA+, whereas 45.9% of GC33-sCD70 CAR-T cells were CD45RA+ (FIG. 4B), indicating that armoring GC33CAR with sCD70 limited GC33 CAR-T differentiation, thereby preserving a higher percentage of GC33CAR+ T cells at the naive state. After continuously exposed to irradiated Hep3B cells for six days, about 77.7% of GC33-sCD70 CAR T cells and about 58% of GC33 CAR T cells differentiated into central memory T cells (CD45RA-CD62L+) (FIG. 14B).

### Example 5. GC33-sCD70 CAR-T exhibited more sustained anti-tumor activity in xenografted NSG mouse model.

The antitumor activities of GC33 CAR-T and GC33s-CD70 CAR-T cells were compared in a hepatocellular carcinoma xenograft mouse model. NSG mice were engrafted with Hep3B as outlined in FIG. 15A. Prior to T-cell infusion, mice were randomly grouped based on the tumor BLI to ensure a similar tumor burden distributed to each of the experimental groups. As shown in FIG. 15B, GC33CAR-T, resembling untransduced T (UNT) and mock (PBS) treatment, did not show any significant antitumor effect in the experimental setting. The tumor grew rapidly in all three Groups. All mice succumbed to euthanasia around Day 33, when the tumor reached the BLI value of ≥2000, in accordance with the Institutional Animal Care and Use Guideline.

In contrast, GC33-sCD70 CAR-T treatment steadily eradicated the tumor. At Day 33, the tumor was undetectable in all GC33-sCD70 CAR-T treated mice. The tumor recurred in one mouse on Day 40 and another mouse on Day 54. As revealed by IVIS imaging (FIG. 15B), the tumor grew at a slower pace in the two relapsed mice. All other mice in the GC33-sCD70CAR-T treatment group remained tumor-free until the end of the experiment (>100 days). The GC33-sCD70 CAR-T treatment did not show significant toxicity. For example, the body weights (BW) of GC33-sCD70 CAR-T treated mice were comparable to the mice treated with GC33 CAR-T or the control mice (FIG. 15C).

The results show that the GC33-sCD70 CAR-T cells have more potent and sustained antitumor activity useful for long-term control of tumor relapse in clinic.

### Example 6. GC33-sCD70 CAR-T Showed Enhanced Persistence in NSG Mice.

Whether the GC33sCD70 CAR-T cells have enhanced persistence in vivo was determined. To assess CAR-T existence in the peripheral blood, three mice were randomly selected, at Day 16 post-CAR-T infusion, from the GC33CAR-T- or GC33-sCD70CAR-T-treated group. As evidenced by immune staining with antibody against mouse CD45 (mCD45) and human CD3 (hCD3) (FIG. 16A), significantly more mCD45-hCD3+ T cells were detected in the peripheral blood of the GC33-sCD70 CAR-T-treated mice (n=3). The results demonstrate that human CD3+ T cells persisted better in GC33-sCD70 CAR-T-treated mice than in GC33 CAR-T-treated mice.

The fact that the GC33-sCD70 CAR-T treated mice remained tumor-free or bore tumor of small size enabled extended analyses of the persistence of human CD3+ T cells in vivo. As shown in FIG. 16B, mCD45-hCD3+ T cells were detected in four of the five mice in the GC33-sCD70 CAR-T treated group at Days 36, 52 or 66 post-tumor inoculation. In at least two mice, mCD45-hCD3+ T cells were detected in the peripheral blood weeks after complete tumor regression. In one mouse, mCD45-hCD3+ T cells were not detected at any of the test time points, consisting with a weaker control of tumor growth and relapse. The correlation between hCD3+ T cell existence and tumor control/clearance provides further evidence that armoring GC33 CAR with sCD70 enhances persistence of the CAR T cells and sustains antitumor effects in vivo.

In sum, the Examples 1-6 herein demonstrate that the sCD70-armored anti-glypican CAR-T cells express anti-glypican CAR molecules on T-cell surface and secrete sCD70. The sCD70-armored anti-glypican CAR-T cells displayed enhanced expansion and more persistent cytotoxicity against glypican-3-expressed tumor cells. Moreover, the sCD70-armored anti-glypican-3 CAR-T cells generated more durable antitumor activity in a xenograft NSG model of hepatocellular carcinoma.

### Example 7. sCD70-armored DLL3 CAR-T

In this example, a retroviral shuttle plasmid P-1 expressing a second-generation antigen receptor binding to DLL3, a retroviral shuttle plasmid P-2 expressing an sCD70-armored DLL3 second-generation antigen receptor, a retroviral shuttle plasmid P-3 expressing a membrane-bound CD70 armored DLL3 second-generation antigen receptor were constructed, respectively, and the numbers corresponding to CARs are DLL3-CAR09, DLL3-CAR15, and DLL3-CAR16. The specific steps are as follows.

### 1. Plasmid Construction

Referring to Figure 1, a chimeric antigen receptor for T cells is constructed, the nucleic acid encoding the chimeric antigen receptor for T cells is cloned into a retroviral shuttle plasmid, and viral packaging is performed through a packaging plasmid.

The plasmids are constructed using conventional molecular biological methods in the art. The chimeric antigen receptor used in the present invention is a second-generation chimeric antigen receptor, which has the following elements: CD8α signal peptide (SEQ ID NO: 1), antigen binding domain, CD8α hinge region (SEQ ID NO: 2), CD8α transmembrane domain (SEQ ID NO: 4), 4-1BB intracellular co-stimulatory domain (SEQ ID NO: 6), CD3ζ signal transduction domain (SEQ ID NO: 8), P2A cleavage peptide (SEQ ID NO: 10), or other intracellular domains.

The antigen binding domain is DLL3-scFv, the sequence of which is shown in SEQ ID NO: 13, wherein the amino acid sequence of the variable domain of heavy chain (VH) is shown in SEQ ID NO: 14 and has the complementary determining regions (CDR) HCDR1, HCDR2, and HCDR3 shown in SEQ ID NO: 16-18; the amino acid sequence of that variable domain of light chain (VL) is shown in SEQ ID NO: 15 and has the complementary determining regions (CDR) LCDR1, LCDR2, and LCDR3 shown in SEQ ID NO: 19-21; VH and VL are connected by a linker, and the amino acid sequence is shown in SEQ ID NO: 11. The amino acid sequences of each element are shown in Table 1.

**Table 1. Sequence information of each element of chimeric antigen receptor equipped with armor molecules**

| SEQ ID NO | Element | Amino acid sequence |
|---|---|---|
| 1 | CD8α signal peptide | MALPVTALLLPLALLLHAARP |
| 2 | CD8α hinge region | TTTPAPRPPTPAPTIASQPLSLRPEACRPAAGGAVHT RGLDFACD |
| 3 | CD28 hinge region | IEVMYPPPYLDNEKSNGTIIHVKGKHLCPSPLFPGPS |
| | | KP |
| 4 | CD8α transmembrane domain | IYIWAPLAGTCGVLLLSLVITLYC |
| 5 | CD28 transmembrane domain | FWVLVVVGGVLACYSLLVTVAFIIFWV |
| 6 | 4-1BB intracellular co-stimulatory domain | KRGRKKLLYIFKQPFMRPVQTTQEEDGCSCRFPEEE EGGCEL |
| 7 | CD28 intracellular co-stimulatory domain | RSKRSRLLHSDYMNMTPRRPGPTRKHYQPYAPPRD FAAYRS |
| 8 | CD3ζ signal transduction domain | RVKFSRSADAPAYQQGQNQLYNELNLGRREEYDVL DKRRGRDPEMGGKPRRKNPQEGLYNELQKDKMAE AYSEIGMKGERRRGKGHDGLYQGLSTATKDTYDAL HMQALPPR |
| 9 | T2A cleavage peptide | EGRGSLLTCGDVEENPGP |
| 10 | P2A cleavage peptide | ATNFSLLKQAGDVEENPGP |
| 11 | linker | GGGGSGGGGSGGGGS |
| 12 | linker | GGGGSGGGGSGGGGSGGGGS |
| 13 | DLL3-scFv (for constructing CAR09, 15-16) | DIVMTQSPLSLPVTPGEPASISCRASQTISDYLHWYL QKPGQSPQLLIKYASQSISGVPDRFSGSGSGSDFTLKI SRVEAEDVGVYYCQQGHSFPLTFGQGTKLEIKGGG GSGGGGSGGGGSEVQLVQSGAEVKKPGASVKVSC |
| 14 | DLL3-scFv VH | KASGYTFTSYWMHWVRQAPGQRLEWMGTIYPGNS DTRYNQKFKDRVTITADTSASTAYMELSSLRSEDTA VYYCTRSYDYGDLDYWGQGTTVTVSS |
| 15 | DLL3-scFv VL | DIVMTQSPLSLPVTPGEPASISCRASQTISDYLHWYL QKPGQSPQLLIKYASQSISGVPDRFSGSGSGSDFTLKI SRVEAEDVGVYYCQQGHSFPLTFGQGTKLEIK |
| 16 | DLL3-VH-CDR1 | SYWMH |
| 17 | DLL3-VH-CDR2 | TIYPGNSDTRYNQKFKD |
| 18 | DLL3-VH-CDR3 | SYDYGDLDY |
| 19 | DLL3-VL-CDR1 | RASQTISDYLH |
| 20 | DLL3-VL-CDR2 | YASQSIS |
| 21 | DLL3-VL-CDR3 | QQGHSFPLT |
| 22 | Soluble CD70 | QRFAQAQQQLPLESLGWDVAELQLNHTGPQQDPRL YWQGGPALGRSFLHGPELDKGQLRIHRDGIYMVHI QVTLAICSSTTASRHHPTTLAVGICSPASRSISLLRLSF HQGCTIASQRLTPLARGDTLCTNLTGTLLPSRNTDET FFGVQWVRP |
| 23 | membrane-bound CD70 | MPEEGSGCSVRRRPYGCVLRAALVPLVAGLVICLVV CIQRFAQAQQQLPLESLGWDVAELQLNHTGPQQDP RLYWQGGPALGRSFLHGPELDKGQLRIHRDGIYMV HIQVTLAICSSTTASRHHPTTLAVGICSPASRSISLLRL SFHQGCTIASQRLTPLARGDTLCTNLTGTLLPSRNTD ETFFGVQWVRP |

In this embodiment, the antibodies or antigen binding domains used in the chimeric antigen receptors all bind to the DLL3 antigen, wherein the antigen binding domains of CAR constructs DLL3-CAR09, DLL3-CAR15, and DLL3-CAR16 are single-chain variable fragments (scFv), and the heavy chain variable region and the light chain variable region in the scFv are connected by a linker.

DLL3-CAR15 is armored with soluble CD70 (SEQ ID NO: 22) on basis of DLL3-CAR09, and DLL3-CAR16 is armored with membrane-bound CD70 (SEQ ID NO: 23) on basis of DLL3-CAR09, and the specific sequences are shown in Table 2.

**Table 2. Amino acid sequence information of DLL3 chimeric antigen receptors (with and without armor molecules)**

| SEQ ID NO: | Plasmid No. | CAR No. | Amino acid sequence |
|---|---|---|---|
| 24 | P-1 | DLL3-CAR09 | MALPVTALLLPLALLLHAARPDIVMTQSPLSLP VTPGEPASISCRASQTISDYLHWYLQKPGQSPQ LLIKYASQSISGVPDRFSGSGSGSDFTLKISRVEA EDVGVYYCQQGHSFPLTFGQGTKLEIKGGGGS GGGGSGGGGSEVQLVQSGAEVKKPGASVKVS CKASGYTFTSYWMHWVRQAPGQRLEWMGTI YPGNSDTRYNQKFKDRVTITADTSASTAYMELS SLRSEDTAVYYCTRSYDYGDLDYWGQGTTVT VSSAAAIEVMYPPPYLDNEKSNGTIIHVKGKHL CPSPLFPGPSKPFWVLVVVGGVLACYSLLVTVA FIIFWVRSKRSRLLHSDYMNMTPRRPGPTRKH YQPYAPPRDFAAYRSRVKFSRSADAPAYQQGQ NQLYNELNLGRREEYDVLDKRRGRDPEMGGK PRRKNPQEGLYNELQKDKMAEAYSEIGMKGER RRGKGHDGLYQGLSTATKDTYDALHMQALPP R |
| 25 | P-2 | DLL3-CAR15 | MALPVTALLLPLALLLHAARPDIVMTQSPLSLP VTPGEPASISCRASQTISDYLHWYLQKPGQSPQ LLIKYASQSISGVPDRFSGSGSGSDFTLKISRVEA EDVGVYYCQQGHSFPLTFGQGTKLEIKGGGGS GGGGSGGGGSEVQLVQSGAEVKKPGASVKVS CKASGYTFTSYWMHWVRQAPGQRLEWMGTI YPGNSDTRYNQKFKDRVTITADTSASTAYMELS SLRSEDTAVYYCTRSYDYGDLDYWGQGTTVT VSSTTTPAPRPPTPAPTIASQPLSLRPEACRPAAG GAVHTRGLDFACDIYIWAPLAGTCGVLLLSLVI TLYCKRGRKKLLYIFKQPFMRPVQTTQEEDGCS CRFPEEEEGGCELRVKFSRSADAPAYQQGQNQ LYNELNLGRREEYDVLDKRRGRDPEMGGKPR RKNPQEGLYNELQKDKMAEAYSEIGMKGERR RGKGHDGLYQGLSTATKDTYDALHMQALPPR KRRSGSGATNFSLLKQAGDVEENPGPMALPVT ALLLPLALLLHAARPQRFAQAQQQLPESLGW DVAELQLNHTGPQQDPRLYWQGGPALGRSFLH GPELDKGQLRIHRDGIYMVHIQVTLAICSSTTA SRHHPTTLAVGICSPASRSISLLRLSFHQGCTIAS QRLTPLARGDTLCTNLTGTLLPSRNTDETFFGV QWVRP |
| 26 | P-3 | DLL3-CAR16 | MALPVTALLLPLALLLHAARPDIVMTQSPLSLP VTPGEPASISCRASQTISDYLHWYLQKPGQSPQ |
| | | | LLIKYASQSISGVPDRFSGSGSGSDFTLKISRVEA EDVGVYYCQQGHSFPLTFGQGTKLEIKGGGGS GGGGSGGGGSEVQLVQSGAEVKKPGASVKVS CKASGYTFTSYWMHWVRQAPGQRLEWMGTI YPGNSDTRYNQKFKDRVTITADTSASTAYMELS SLRSEDTAVYYCTRSYDYGDLDYWGQGTTVT VSSTTTPAPRPPTPAPTIASQPLSLRPEACRPAAG GAVHTRGLDFACDIYIWAPLAGTCGVLLLSLVI TLYCKRGRKKLLYIFKQPFMRPVQTTQEEDGCS CRFPEEEEGGCELRVKFSRSADAPAYQQGQNQ LYNELNLGRREEYDVLDKRRGRDPEMGGKPR RKNPQEGLYNELQKDKMAEAYSEIGMKGERR RGKGHDGLYQGLSTATKDTYDALHMQALPPR KRRSGSGATNFSLLKQAGDVEENPGPMPEEGS GCSVRRRPYGCVLRAALVPLVAGLVICLVVCIQ RFAQAQQQLPLESLGWDVAELQLNHTGPQQDP RLYWQGGPALGRSFLHGPELDKGQLRIHRDGI YMVHIQVTLAICSSTTASRHHPTTLAVGICSPAS RSISLLRLSFHQGCTIASQRLTPLARGDTLCTNL TGTLLPSRNTDETFFGVQWVRP |

A polynucleotide encoding a chimeric antigen receptor is synthesized and an enzyme cleavage site and a shuttle plasmid homologous sequence are added at both ends thereof. The shuttle plasmid was digested with restriction endonuclease, and the linear plasmid was recovered and purified by agarose gel electrophoresis. The polynucleotide synthesized in the above step and the linearized vector were ligated by recombinase 5 × In-Fusion HD enzyme (TaKaRa, Cat # ST0344) in the following reaction system: 2 µl of polynucleotide fragment (50 ng/µl), 1 µl of linearized plasmid (50 ng/µl), 2 µl of 5 × HD In-Fusion enzyme, 5 µl of ddH20. After mixing, centrifuge briefly and react at 50 °C for 15 minutes. Add 10µl of recombinant reaction product to 100µl of bacterial competent cells, place it on ice for 5 minutes, evenly spread the transformed bacterial solution on LB plate containing 100µg/ml ampicillin, and incubate it inverted in a constant temperature incubator for 12-16 hours. Three-five clones were randomly picked from each plate for sequencing identification. The correctly sequenced bacterial suspension was transferred to 100 ml LB liquid medium containing 100 µg/ml ampicillin, cultured at 37°C overnight, and plasmids were extracted using the MN endotoxin-free plasmid extraction kit (MN, Cat#740420.50). After quantification, the plasmid was diluted to 1000 ng/µl with endotoxin-free ultrapure water.

### 2. Retrovirus preparation

293T cells (Cell Bank of Type Culture Collection Committee, Chinese Academy of Sciences, Cat # GNHu17) were inoculated into a 100 mm culture dish for culture in DMEM medium (Gibco, Cat # 10566016) with 10% FBS (Gibco, Cat # 10099141). When the cell fusion reaches about 70%, plasmid transfection is carried out: the retroviral shuttle plasmid expressing the chimeric antigen receptor was taken, mixed with the retroviral packaging plasmid, and then 50 µl Opti-MEM medium (Thermofisher Scientific, Cat#31985070) was added, and 420 µl Opti-MEM medium containing 30 µl Fugene HD (Promega, Cat#04709691001) was added, mixed and incubated at room temperature for 15 minutes. Finally, the mixture was added to 293T cells and cultured at 37° C, 5% CO2 for 3 days. The supernatant was collected, filtered through a 0.45 µm filter membrane, and concentrated for later use.

### Example 8. sCD70-armored DLL3 CAR-T cells preparation and characterization

### 1. Cell preparation

T cells were isolated from fresh PBMCs using the Stemcell Easy Sep Kit (Stemcell, Cat#19055). The isolated T cells were transferred to culture dishes pre-coated with 1 µg/ml CD3/CD28 antibodies (MY-ebioscience-16-0289-85, MY-ebioscience-16-0037-85). The culture medium consisted of X-VIVO15 (Lonza, Cat# BEBP02-054Q), 5% human AB serum (Gemini, Cat#100-512), 100 U/ml penicillin-streptomycin (Gibco, Cat#15140-122), and 200 IU/ml human IL2 (Beijing Shuanglu, Cat#S19991007).

The above T cells in good condition were taken, and a 24-well cell culture plate was coated with 5 µg/ml recombinant human fibrin (Takara, Cat # T100B), added with 500 µl of the concentrated retrovirus prepared in Example 7, and centrifuged at 2000 g at 4 °C for 1 hour. After the virus supernatant was removed, 3E5 T cells were added and centrifuged at 400g for 5min. After centrifugation, the cells were cultured at 37 °C and 5% CO2, counted twice a week, and when the cell density reached 2.5 * 10⁶/ml, they were subcultured and expanded. The results showed that all CAR-T cells had good expansion efficiency.

### 2. FACS detection of CAR transfection efficiency and T cell phenotypeF

The cells were washed twice with PBS buffer, and diluted to 2E6 cells/ml with PBS buffer after cell counting, and 50 µl Fc blocking reagent (BioLegend, Cat# 422302) was added. The cells were incubated at room temperature for 10 minutes, and then 100 µl cells per well was added to a 96-well FACS reaction plate, and 100 µl his-tagged DLL3 antigen protein (3 µg/ml) was added. The cells were incubated at 4°C for 30 minutes. The cells were washed twice with FACS buffer by centrifugation, and 100 µl of fluorescent (AF647) labeled secondary antibody (GenScript, Cat# A01802) was added per well, and the cells were incubated at 4°C for 20 minutes. The cells were washed three times with FACS buffer by centrifugation, and the cells were suspended with 200 µl FACS buffer. The results were detected and analyzed by FACS (CANTOII, purchased from BD). The level of DLL3 CAR expressed by T cells one week after in vitro expansion by viral transfection is shown in FIG. 2A, with CAR expression levels of DLL3-CAR09 and DLL3-CAR15 being 69% and 91%, respectively.

Flow cytometry was also used to detect the memory phenotype of T cells 8 days after transfection, and the expression of central memory T cells (CM, CD62L+CD45RO+), effector memory T cells (EM, CD62L-CD45RO+), stem cell-like memory T cells (SCM, CD62L+CD45RO-) and effector T cells (EF, CD62L-CD45RO-) were detected. Flow cytometry was used to detect the exhaustion phenotype of T cells and the expression of CD8+ and CD4+ 8 days after transfection.

2x10⁵ T cells were taken from each group and placed in a 96-well FACS reaction plate. The cells were centrifuged at 400g for 5 minutes and the supernatant was removed. The T cell phenotype antibody mixture diluted with FACS buffer was added and incubated at 4 degrees for 30 minutes. The cells were washed three times by centrifugation with FACS buffer, and the cells were suspended with 200µl FACS buffer. The results were detected and analyzed by FACS (CANTOII, purchased from BD). The results are shown in Figure 4A.

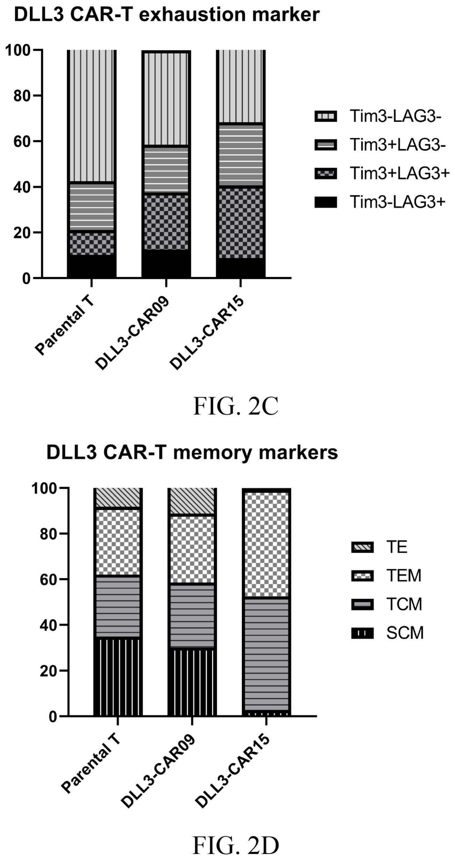

The flow cytometry results of T cell phenotypes are shown in Figures 2B, 2C, and 2D, in which the CD4+ T cell content of the DLL3-CAR15 group was slightly higher than that of DLL3-CAR09 and untransfected T cells (i.e., parental T cells) ( Figure 2B); the TIM3+LAG3+ double-positive depletion phenotypes of the DLL3-CAR09 and DLL3-CAR15 groups were slightly higher than those of the untransfected T cell group (Figure 2C); the TCM and TEM of the DLL3-CAR15 group were significantly higher than those of other groups. (Figure 2D).

### 3. ELISA detection of the level of CD70 protein secreted by CAR-T cells

During in vitro expansion culture, the culture medium supernatant was collected to detect the level of CD70 protein secreted by CAR-T cells. The supernatant of the culture medium (cultivated for 3 days) with a CAR-T cell density of about 2E6 was centrifuged to remove cell debris, and then tested using the Human CD70 ELISA kit (Abcam, ab264621). As shown in FIG. 2E, CD70 protein secretion at a level of 3.7 ng/ml was detected only in the medium supernatant of the DLL3-CAR15 group.

The above results indicate that CD70-secreting armored DLL3 CAR-T cells can normally express DLL3 CAR structure and secrete CD70 protein. This armored structure may increase the activation degree of DLL3 CAR-T cells during expansion, thus significantly increasing the proportion of central memory T cells and effector memory T cells.

### Example 9: Evaluation of tumor killing function of sCD70-armored DLL3 CAR-T cells in vitro

### 1. Evaluation of specific tumor cell killing function of DLL3 CAR-T in vitro

To verify the in vitro killing toxicity of sCD70-armored DLL3 CAR-T cells. The human DLL3 high-expression tumor cell line SHP-77 and the low-expression tumor cell line NCI-H2171 (purchased from ATCC) were used for in vitro tumor killing functional evaluation.

The culture medium containing suspended target cells in the culture flask was collected into a 50 ml centrifuge tube, centrifuged at 400 g for 5 min, the supernatant was removed, fresh culture medium was added, and the cells were blown evenly with a pipette. After counting the concentration of the cell suspension with a counting plate, 1 x 107 cells were taken out, labeled with CFSE (Biolegend, Cat# 423801), and 40,000 cells were added to each well of a 96-well FACS plate. T cells with different CAR structures and empty T cells were added according to the ratio of T cells: target cells of 1:2, 1:10, and 1:20. After 72 hours of incubation, the cells were centrifuged at 400 g for 5 min, the supernatant was removed, the cells were suspended with 200 µl FACS buffer, and the results were detected and analyzed by FACS (CANTOII, purchased from BD).

As shown in FIGS. 3A and 3B, the killing effect of CAR-T cells with DLL3-CAR15 structure, which is armored with secrete CD70, on SHP-77 target cells and NCI-H2171 target cells was significantly better than that of DLL3-CAR09 structure under different ET ratios during 72 h killing.

### 2. Multi-round killing functional evaluation of DLL3 CAR-T on specific tumor cells in vitro

An in vitro serial killing assay was performed to evaluate the persistence and long-term killing ability of the sCD70 armed DLL 3 CAR-T cells using the self-constructed NCI-H2171 stable strain NCI-H2171-luc that highly expresses human bacterial luciferase. The NCI-H2171-luc cells were transformed with luciferase reporter gene by conventional gene manipulation methods. In particular, 4E5 per well NCI-H2171-luc cells were plated in 24 well plates, and T cells with different CAR structures and untraduced T cells were added according to E: T = 1: 2 for 72 hours of co-culture. Then 200 µl cell suspension in each well was collected into blank 96-well plate, and firefly luciferase substrate D-Luciferin (Abcam Cat#ab143655) were added. A PE EnSight microplate reader was used to read the bioluminescence values and analyze the results. At the same time, the remaining cells in each group were counted for viable cells, and the number of T cells in the remaining mixture was calculated based on the number of viable cells and the killing results. 2E5 CAR-T cells were re-plated and 4E5 fresh NCI-H2171-luc target cells were added to each well for the second round of killing experiment. This process was repeated for a total of 3 rounds of killing.

The results are shown in Figure 3C. The sCD70 armored structure effectively improved the killing ability of DLL3 CAR-T cells against NCI-H2171-luc target cells, and significantly extended the killing persistence to the third round. The above results indicate that CD70 armor can further improve the killing function of the DLL3-CAR09 structure against SCLC tumor cell lines with different DLL3 expression levels, expand the use window of DLL3 CAR-T cells in clinical applications, and potentially reduce the dosage to ensure therapeutic efficacy. while improving the safety of cell therapy.

### Example 10: Detection of the anti-tumor efficacy of sCD70 armored DLL3 CAR-T cells on mouse subcutaneous tumor models

### 1. Detection of the anti-tumor efficacy of low-dose sCD70 armored DLL3 CAR-T cells in the SHP-77 mouse subcutaneous tumor model

The anti-tumor effect of DLL3 CAR-T cells was evaluated using the mouse subcutaneous tumor CDX model. The details are as follows:
NPG mice (combined immunodeficient mice, purchased from Beijing Weitongda Biotechnology Co., Ltd.) were subcutaneously inoculated with 3 x 106 SHP-77 cells in the logarithmic growth phase and in good growth condition.

On the third day after inoculation, the long and short diameters a and b of the mouse tumor were measured, and the mouse tumor volume V (mm3) = 1/2×(a × b2) was calculated. Mice with a tumor volume of about 45 mm3 were randomly divided into groups according to the random number principle. On the 10th day after tumor inoculation, low-dose CAR-T cells (4 x 106 CAR+/mouse) were injected into the tail vein. The CAR-T cell injection day was Day 0 (D0), and the administration was single. The specific grouping and administration are shown in Table 3.

**Table 3. Grouping of anti-tumor experiments of SHP-77 model in vivo**

| Group | Number of animals | Treatment drug | Dosage | Route of administration | Time of administration |
|---|---|---|---|---|---|
| G1 | 5 | PBS | PBS/200µL | i.v. | D0 |
| G2 | 5 | DLL3-CAR09 | 4e6 CAR+/200µL | i.v. | D0 |
| G3 | 5 | DLL3-CAR15 | 4e6 CAR+/200µL | 1.V. | D0 |

Tumor volume was continuously observed and measured, measurements were recorded twice a week and 70 µL of blood was taken each time for identification of CAR-T cell expansion in peripheral blood of mice.

The tumor volume detection results of mice are shown in FIG. 4A, and the body weight change is shown in FIG. 4B: The PBS control group was euthanized on D26 because the tumor volume exceeded 2000mm3; the body weight of animals in the PBS control group decreased slightly, which may be due to the poor animal condition caused by tumor progression, while the body weight of animals in other treatment groups was normal; the CAR-T DLL3-CAR09 group did not show obvious drug efficacy, and the tumor growth rate was similar to that of the PBS control group; the tumors of animals in the CAR-T DLL3-CAR15 group showed obvious drug efficacy to inhibit tumor growth on Day15.

The results of mouse peripheral blood T cell detection are shown in Figure 4C. No obvious T cell expansion was detected in the peripheral blood of animals in the DLL3-CAR09 group; T cell expansion was detected in the DLL3-CAR15 group on Day 15, which coincided with the drug efficacy time of tumor inhibition.

At the low dose of 4E6 CAR+/200µL/mouse, DLL3-CAR09 failed to show significant therapeutic efficacy. In contrast, significant T cell expansion and corresponding tumor-suppressing efficacy were observed in the DLL3-CAR15 group with sCD70 armoring.

This result shows that the sCD70 armor structure can better improve the activity and persistence of DLL3 CAR-T cells in the body, and ensure that the CAR-T cells can still show drug efficacy at low doses. Because CAR-T cell therapy requires the use of the patient's own T cells for preparation, the activity of the patient's own T cells varies greatly. The sCD70 armor structure can make up for the shortcomings of the poor activity of autologous DLL3 CAR-T cells by secreting CD70 protein, ensuring persistence in the body and increasing the possibility of exerting drug efficacy.

### 2. Detection of the anti-tumor efficacy of DLL3 CAR-T cells in the NCI-H2171 mouse subcutaneous tumor model under medium-dose conditions

The anti-tumor effect of DLL3 CAR-T cells was evaluated using a mouse subcutaneous tumor CDX model: NPG mice (combined immunodeficient mice, purchased from Beijing Weitongda Biotechnology Co., Ltd.) were subcutaneously inoculated with 3 x106 NCI-H2171 cells in the logarithmic growth phase and in good growth condition.

Mice with tumor volumes of about 30 mm3 were randomly grouped according to the random number principle. On the 10th day after tumor inoculation, CAR-T cells (8 x 106 CAR+/mouse) were injected into the tail vein. The day of CAR-T cell injection was day 0 (D0), and the drug was administered once. The specific grouping and drug administration are shown in Table 4.

**Table 4 Grouping of NCI-2171 model in vivo anti-tumor experiment**

| Group | Number of animals | Treatment drug | Dosage | Route of administration | Time of administration |
|---|---|---|---|---|---|
| G1 | 5 | PBS | PBS/200µL | i.v. | D0 |
| G2 | 5 | DLL3-CAR09 | 8e6 CAR+/200µL | 1.V. | D0 |
| G3 | 5 | DLL3-CAR15 | 8e6 CAR+/200µL | 1.V. | D0 |

The results of mouse tumor volume detection are shown in FIG. 4D, the weight changes are shown in FIG. 4E, and the tumor inhibition rate is shown in FIG. 4F: Animals in the PBS and DLL3-CAR09 groups were euthanized on D17 because the average tumor size exceeded 2000 mm3. The weight status of animals in each group was normal.

ue to the low dose, no T cell proliferation was detected in the peripheral blood. The DLL3 expression of NCI-H2171 tumor cells was low, and the tumor grew rapidly, making treatment more difficult. In the medium-dose CAR-T cell therapy plan, DLL3-CAR09 did not work, and a significant decrease in tumor growth was observed in all five animals in the DLL3-CAR15 treatment group, showing a 41% tumor inhibition efficiency compared to the PBS control group.

### Example 11. Preparation and comparison of sCD70 armored and membrane-bound CD70 armored DLL3 CAR-T cells

### 1. Preparation and identification of cells

Referring to Figure 1, the corresponding shuttle plasmids were prepared by the methods in Examples 7 and 8, and viral packaging, T cell in vitro amplification and viral infection were performed to construct non-armored DLL3 CAR-T cells DLL3-CAR09, sCD70 armored DLL3 CAR-T cells DLL3-CAR15 and membrane-bound CD70 armored DLL3 CAR-T cells DLL3-CAR16.

The T cell characteristics of DLL3-CAR09, DLL3-CAR15, and DLL3-CAR16 after in vitro expansion were evaluated using the method of Example 9.

The levels of DLL3 CAR expression in T cells one week after viral transfection and in vitro expansion are shown in Figure 5A , with CAR expression levels of DLL3-CAR09, DLL3-CAR15, and DLL3-CAR16 being 74%, 64%, and 71%, respectively.

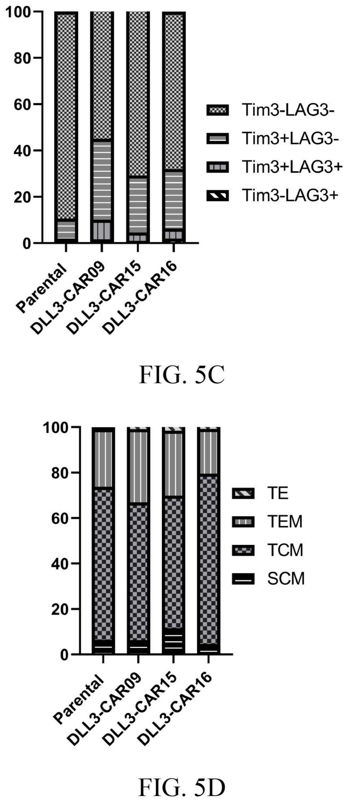

The flow cytometry results of T cell phenotype are shown in FIGS. 5B, 5C, and 5D. Among them, the content of CD4 + T cells in the DLL3-CAR15 group was similar to that of DLL3-CAR09 and untransfected T cells, and the content of CD4 + T cells in the DLL3-CAR16 group was significantly higher than that of other groups (FIG. 5B); The TIM3+LAG3 + double-positive depletion phenotype was higher in both the DLL3-CAR09 and DLL3-CAR15 groups than in the untransfected T cell group, with the highest depletion ratio in the DLL3-CAR09 group (Fig. 5C); The T cell memory phenotype of each group did not change significantly compared to the non-transfected group (FIG. 5D).

### 2. Detection of CD70 protein secreted by CAR-T cells by ELISA

During the in vitro expansion culture, the culture supernatant was collected to detect the level of CD70 protein secreted by CAR-T cells. The supernatant of the culture medium (5 days of cumulative culture) with a CAR-T cell density of about 2E6 was centrifuged to remove cell debris and then detected using the Human CD70 ELISA kit (Abcam, ab264621).

The results are shown in Figure 5E . The secretion level of CD70 protein was detected at the level of 22 ng/ml in the culture supernatant of the DLL3-CAR15 group, and the secretion level of CD70 protein was detected at the level of 4.3 ng/ml in the culture supernatant of the DLL3-CAR16 group.

The above results indicate that sCD70 armored DLL3 CAR-T cells can normally express the DLL3 CAR structure and secrete CD70 protein, which are significantly higher than non-armored and membrane-bound CD70 armored CAR-T cells. The expression detection results of CD70 and CD27 on the surface of T cells are shown in FIG. 5F. The expression level of CD70 on the cell membrane surface of the DLL3-CAR16 group was 80%, which was significantly higher than that of the non-armored (17%) and secretable CD70 (29%) groups. Correspondingly, the expression level of CD27 on the membrane surface of the DLL3-CAR16 group was 28%, which was significantly lower than that of the untransfected and other CAR-T groups (both above 90%). The above results indicate that membrane-bound CD70 armored DLL3 CAR-T cells can normally express membrane-bound CD70, while excessive binding of CD70 and CD27 leads to the shedding of CD27 on the surface of T cells, which in turn affects the activation degree of CAR-T cells.

### 3. In vitro killing experiments of sCD70-armored and membrane-bound CD70-armored DLL3 CAR-T cells

The killing effect of each group of CAR-T cells on the DLL3 high-expressing cell line SHP-77 and the low-expressing cell line NCI-H2171 was evaluated by the method of Example 9. As shown in FIGS. 5G and 5H, during the 72-hour killing process, at different E:T ratios, the killing effect of the sCD70-armored DLL3-CAR15 structure CAR-T cells on SHP-77 target cells and NCI-H2171 target cells was significantly better than that of the DLL3-CAR09 and DLL3-CAR16 structures.

The membrane-bound CD70-armored DLL3-CAR16 structure has a better ability to kill SHP-77, a high DLL3 expression target cell, than the non-armored structure, but its ability to kill NCI-H2171, a low DLL3 expression target cell, is weaker than that of the non-armored structure, which may be due to the significantly lower expression of CD27 than that of the non-armored and secretable CD70 armor groups, thus affecting the function of CAR-T cells. The above results prove that the CD70 armor structure can improve the activation degree and specific killing ability of CAR-T without affecting the expression level of CD27. Compared with the membrane-bound CD70 armor structure, it has the advantage of improving the function of CAR-T.

### 4. In vivo tumor inhibition experiment of sCD70-armored and membrane-bound CD70-armored DLL3 CAR-T cells

The anti-tumor effect of DLL3 CAR-T cells was evaluated using a mouse subcutaneous tumor CDX model: NPG mice (combined immunodeficient mice, purchased from Beijing Weitongda Biotechnology Co., Ltd.) were subcutaneously inoculated with 3 x106 NCI-H2171 cells in the logarithmic growth phase and in good growth condition.

Mice with tumor volumes of about 30 mm3 were randomly divided into groups according to the random number principle. On the 10th day after tumor inoculation, CAR-T cells (1.5 x 107 CAR+/mouse) were injected into the tail vein. The day of CAR-T cell injection was day 0 (D0), and the drug was administered once. The specific grouping and drug administration are shown in Table 5.

**Table 5. Grouping of NCI-2171 model in vivo anti-tumor experiment**

| Group | Number of animals | Treatment drug | Dosage | Route of administration | Time of administration |
|---|---|---|---|---|---|
| G1 | 5 | PBS | PBS/200µL | i.v. | D0 |
| G2 | 5 | DLL3-CAR09 | 1.5e7 CAR+/200µL | i.v. | D0 |
| G3 | 5 | DLL3-CAR15 | 1.5e7 CAR+/200µL | i.v. | D0 |
| G4 | 5 | DLL3-CAR16 | 1.5e7 CAR+/200µL | i.v. | D0 |

The results of mouse tumor volume detection are shown in Figure 6A. The CAR-T DLL3-CAR16 group did not show obvious drug efficacy; the tumors of mice in the CAR-T DLL3-CAR09 group began to show an obvious trend of slowing tumor growth at D11; the tumors of animals in the CAR-T DLL3-CAR15 group began to show obvious drug efficacy to inhibit tumor growth at D8. The body weight changes are shown in FIG. 6B. The animals in the PBS group were euthanized on D18 because the mean tumor value exceeded 2000 mm3, and the body weight status of the animals in other groups was normal. The detection results of T cells in peripheral blood of mice are shown in FIG. 6C. The T cell content in peripheral blood of animals in the DLL3-CAR09 group showed continuous expansion and reached the highest value on D11; The DLL3-CAR15 group showed obvious T cell expansion on D4 and reached the peak expansion on D8, which coincided with the drug effect time of tumor inhibition. Compared with the other two groups, the DLL3-CAR16 group showed significantly lower T cell expansion and the expansion speed was slow, and did not show obvious drug effect. The detection results of CD70 and CD27 expression levels on the surface of mouse peripheral blood T cells are shown in FIGS. 6D and 6E. The CD70 expression level on the membrane surface of CAR-T cells in the DLL3-CAR16 group was significantly higher than that in the other two groups, and the CD27 expression level showed a continuous decreasing trend with the expansion of CAR-T.

The above results show that the in vivo efficacy results in the low-expression cell line mouse model are consistent with the in vitro killing results, indicating that the sCD70 armored structure can increase the activation level and specific killing ability of CAR-T without affecting the expression level of CD27, and has the advantage of improving the function of CAR-T compared with the membrane-bound CD70 armored structure.

### Example 12. Preparation and comparison of sCD70-armored and CD70-armored Claudin 18.2 (CLDN18.2) CAR-T cells

In this example, a retroviral shuttle plasmid P-4 expressing a second-generation antigen receptor binding to CLDN18.2, a retroviral shuttle plasmid P-5 expressing an sCD70-armored CLDN18.2 second-generation antigen receptor, a retroviral shuttle plasmid P-6 expressing a membrane-bound CD70 armored CLDN18.2 second-generation antigen receptor were constructed, respectively, and the numbers corresponding to CARs are CLDN18.2, CLDN18.2-sCD70, CLDN18.2-mCD70. The specific steps are as follows.

### 1. Plasmid Construction

Referring to Figure 7, a chimeric antigen receptor for CLDN18.2 CAR-T cells is constructed, the nucleic acid encoding the chimeric antigen receptor for T cells is cloned into a retroviral shuttle plasmid, and viral packaging is performed through a packaging plasmid.

The plasmids are constructed using conventional molecular biological methods in the art. The chimeric antigen receptor used in the present invention is a second-generation chimeric antigen receptor, which has the following elements: CD8α signal peptide (SEQ ID NO: 1), antigen binding domain, CD8α hinge region (SEQ ID NO: 2), CD28 transmembrane domain (SEQ ID NO: 5), CD28 intracellular co-stimulatory domain (SEQ ID NO: 7), CD3ζ signal transduction domain (SEQ ID NO: 8), P2A cleavage peptide (SEQ ID NO: 10). The amino acid sequences of each element are shown in Table 1.

The antigen binding domain is CLDN18.2-scFv, the sequence of which is shown in SEQ ID NO: 27, wherein the amino acid sequence of the variable domain of heavy chain (VH) is shown in SEQ ID NO: 28 and has the complementary determining regions (CDR) HCDR1, HCDR2, and HCDR3 shown in SEQ ID NO: 30-32; the amino acid sequence of that variable domain of light chain (VL) is shown in SEQ ID NO: 29 and has the complementary determining regions (CDR) LCDR1, LCDR2, and LCDR3 shown in SEQ ID NO: 33-35; VH and VL are connected by a linker, and the amino acid sequence is shown in SEQ ID NO: 11. The amino acid sequences of the antigen binding domain are shown in Table 6.

CLDN18.2-sCD70 is armored with soluble CD70 (SEQ ID NO: 22) on basis of CLDN18.2, and CLDN18.2-mCD70 is armored with membrane-bound CD70 (SEQ ID NO: 23) on basis of CLDN18.2, and the specific sequences are shown in Table 7.

**Table 6. Sequence information of CLDN18.2 CAR-T antigen binding domain and chimeric antigen receptor**

| SEQ ID NO | Element | Amino acid sequence |
|---|---|---|
| 27 | CLDN18.2-scFv | QVQLVQSGAEVKKPGSSVKVSCKASGYAFSNYLIEW VKQAPGQGLEWIGLINPGSGGTNYNEKFKGKATITAD KSTSTAYMELSSLRSEDTAVYYCARVYYGNSFAYWG QGTLVTVSSGGGGSGGGGSGGGGSDIVMTQSPDSLA VSLGERATISCKSSQSLLNSGNQKNYLTWYQQKPGQP PKLLIYWASTRESGVPDRFSGSGSGTDFTLTISSLQAE DVAVYYCQNDYFYPFTFGQGTKLEIK |
| 28 | CLDN18.2-scFv VH | QVQLVQSGAEVKKPGSSVKVSCKASGYAFSNYLIEW VKQAPGQGLEWIGLINPGSGGTNYNEKFKGKATITAD KSTSTAYMELSSLRSEDTAVYYCARVYYGNSFAYWG QGTLVTVSS |
| 29 | CLDN 18.2-scFv VL | DIVMTQSPDSLAVSLGERATISCKSSQSLLNSGNQKN YLTWYQQKPGQPPKLLIYWASTRESGVPDRFSGSGSG TDFTLTISSLQAEDVAVYYCQNDYFYPFTFGQGTKLEI K |
| 30 | CLDN18.2-VH-CDR1 | NYLIE |
| 31 | CLDN18.2-VH-CDR2 | LINPGSGGTNYNEKFKG |
| 32 | CLDN18.2-VH-CDR3 | VYYGNSFAY |
| 33 | CLDN18.2-VL-CDR1 | KSSQSLLNSGNQKNYLT |
| 34 | CLDN18.2-VL-CDR2 | WASTRES |
| 35 | CLDN18.2-VL-CDR3 | QNDYFYPFT |

**Table 7. Amino acid sequence information of CLDN18.2 CAR-T chimeric antigen receptor (with and without armor molecules)**

| SEQ ID NO: | Plasmid No. | CAR No. | Amino acid sequence |
|---|---|---|---|
| 36 | P-4 | CLDN18.2 | MALPVTALLLPLALLLHAARPQVQLVQSGAEV KKPGSSVKVSCKASGYAFSNYLIEWVKQAPGQ GLEWIGLINPGSGGTNYNEKFKGKATITADKSTS TAYMELSSLRSEDTAVYYCARVYYGNSFAYWG QGTLVTVSSGGGGSGGGGSGGGGSDIVMTQSP DSLAVSLGERATISCKSSQSLLNSGNQKNYLTW YQQKPGQPPKLLIYWASTRESGVPDRFSGSGSG TDFTLTISSLQAEDVAVYYCQNDYFYPFTFGQGT KLEIKAAAIEVMYPPPYLDNEKSNGTIIHVKGK HLCPSPLFPGPSKPFWVLVVVGGVLACYSLLVT VAFIIFWVRSKRSRLLHSDYMNMTPRRPGPTRK HYQPYAPPRDFAAYRSRVKFSRSADAPAYQQGQ NQLYNELNLGRREEYDVLDKRRGRDPEMGGKP RRKNPQEGLYNELQKDKMAEAYSEIGMKGERR RGKGHDGLYQGLSTATKDTYDALHMQALPPR |
| 37 | P-5 | CLDN18.2-sCD 70 | MALPVTALLLPLALLLHAARPQVQLVQSGAEV KKPGSSVKVSCKASGYAFSNYLIEWVKQAPGQ GLEWIGLINPGSGGTNYNEKFKGKATITADKSTS TAYMELSSLRSEDTAVYYCARVYYGNSFAYWG QGTLVTVSSGGGGSGGGGSGGGGSDIVMTQSP DSLAVSLGERATISCKSSQSLLNSGNQKNYLTW YQQKPGQPPKLLIYWASTRESGVPDRFSGSGSG TDFTLTISSLQAEDVAVYYCQNDYFYPFTFGQGT KLEIKAAAIEVMYPPPYLDNEKSNGTIIHVKGK HLCPSPLFPGPSKPFWVLVVVGGVLACYSLLVT VAFIIFWVRSKRSRLLHSDYMNMTPRRPGPTRK HYQPYAPPRDFAAYRSRVKFSRSADAPAYQQGQ NQLYNELNLGRREEYDVLDKRRGRDPEMGGKP RRKNPQEGLYNELQKDKMAEAYSEIGMKGERR RGKGHDGLYQGLSTATKDTYDALHMQALPPRK RRSGSGATNFSLLKQAGDVEENPGPMALPVTAL LLPLALLLHAARPQRFAQAQQQLPLESLGWDV AELQLNHTGPQQDPRLYWQGGPALGRSFLHGP ELDKGQLRIHRDGIYMVHIQVTLAICSSTTASRH HPTTLAVGICSPASRSISLLRLSFHQGCTIASQRL TPLARGDTLCTNLTGTLLPSRNTDETFFGVQWV RP |
| 38 | P-6 | CLDN18.2-mCD 70 | MALPVTALLLPLALLLHAARPQVQLVQSGAEV KKPGSSVKVSCKASGYAFSNYLIEWVKQAPGQ GLEWIGLINPGSGGTNYNEKFKGKATITADKSTS TAYMELSSLRSEDTAVYYCARVYYGNSFAYWG QGTLVTVSSGGGGSGGGGSGGGGSDIVMTQSP DSLAVSLGERATISCKSSQSLLNSGNQKNYLTW YQQKPGQPPKLLIYWASTRESGVPDRFSGSGSG TDFTLTISSLQAEDVAVYYCQNDYFYPFTFGQGT KLEIKAAAIEVMYPPPYLDNEKSNGTIIHVKGK HLCPSPLFPGPSKPFWVLVVVGGVLACYSLLVT VAFIIFWVRSKRSRLLHSDYMNMTPRRPGPTRK HYQPYAPPRDFAAYRSRVKFSRSADAPAYQQGQ NQLYNELNLGRREEYDVLDKRRGRDPEMGGKP RRKNPQEGLYNELQKDKMAEAYSEIGMKGERR RGKGHDGLYQGLSTATKDTYDALHMQALPPRK RRSGSGATNFSLLKQAGDVEENPGPMPEEGSGC SVRRRPYGCVLRAALVPLVAGLVICLVVCIQRFA QAQQQLPLESLGWDVAELQLNHTGPQQDPRLY WQGGPALGRSFLHGPELDKGQLRIHRDGIYMV HIQVTLAICSSTTASRHHPTTLAVGICSPASRSISL LRLSFHQGCTIASQRLTPLARGDTLCTNLTGTLL PSRNTDETFFGVQWVRP |

### 2. Preparation and identification of cells

The corresponding shuttle plasmids were prepared according to the methods in Examples 7 and 8, and viral packaging, T cell in vitro amplification and viral infection were performed to construct non-armored CLDN18.2 CAR-T cells CLDN18.2, sCD70 armored CLDN18.2 CAR-T cells CLDN18.2-sCD70 and membrane-bound CD70 armored CLDN18.2 CAR-T cells CLDN18.2-mCD70.

The T cell characteristics of the two groups of CAR-T after in vitro expansion were evaluated by the method of Example 8.

The in vitro expansion fold results of CLDN18.2 CAR-T cells are shown in Figure 8A. After 12 days of culture, the expansion folds of the CLDN18.2 and CLDN18.2-sCD70 groups were both higher than 125 times, which was significantly better than that of the non-transfected (UT) group (100 times) and CLDN18.2-mCD70 group (60 times).

The levels of CAR expression by T cells 10 days after in vitro expansion of T cells after viral transfection are shown in Figure 8B , where the CAR expression levels of CLDN18.2, CLDN18.2-sCD70, and CLDN18.2-mCD70 were 81%, 88%, and 95%, respectively.

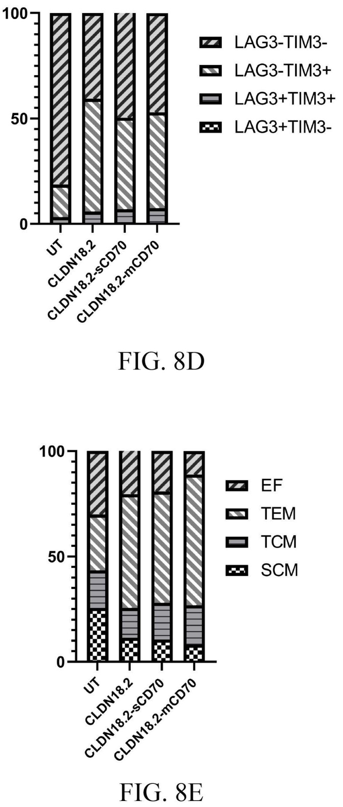

The flow cytometry results of T cell phenotype are shown in Figures 8C, 8D, and 8E. Among them, the content of CD4 + T cells in CLDN18.2-sCD70 and CLDN18.2-mCD70 groups was higher than that in CLDN18.2 and untransfected T cells, and the membrane-bound CD70 armor group was the highest (Figure 8C); Both the depletion phenotype and memory phenotype were higher in the CAR-transfected groups than in the untransfected group, and the proportion of phenotypes was similar in the CAR-transfected groups (Figure 8D and Figure 8E).

### 3. Detection of the level of CD70 protein secreted by CAR-T cells and the expression of CD70 and CD27 on the surface of T cells by ELISA

During in vitro expansion culture, the culture supernatant was collected and used to detect the level of CD70 protein secreted by CAR-T cells. The supernatant of the culture medium (cultured for a total of 5 days) in which the CAR-T cell density was maintained at about 3~5E6 was centrifuged to remove cell debris, and the Human CD70 ELISA kit (Abcam, ab264621) was used for detection. The results are shown in Figure 8F, the CD70 protein secretion level of 96 ng/ml was detected in the culture supernatant of CLDN18.2-sCD70 group, which was significantly higher than that of other groups. These results indicate that sCD70 armored CLDN18.2 CAR-T cells can normally express CLDN18.2 CAR structure and secrete CD70 protein, which is significantly higher than non-armored and membrane-bound CD70 armored CAR-T cells.

The expression detection results of CD70 and CD27 on the T cell surface are shown in Figure 8G. The expression of CD70 on the cell membrane surface of the CLDN18.2-mCD70 group was 96%, which was significantly higher than that of non-armored (13%) and secretable CD70 (50%) group, correspondingly, the expression of CD27 on the membrane surface of the CLDN18.2-mCD70 group was 1.5%, which was significantly lower than the non-transfected and other CAR-T groups (both close to 90%).

The above results indicate that membrane-bound CD70 armored CLDN18.2 CAR-T cells can normally express membrane-bound CD70, while excessive binding of CD70 and CD27 leads to the shedding of CD27 on the surface of T cells, which in turn affects the degree of activation of CAR-T cells.

### 4. In vitro killing experiments of sCD70-armored and membrane-bound CD70-armored CLDN18.2 CAR-T cells

The killing effect of each group of CLDN18.2 CAR-T cells on the CLDN18.2 high-expressing cell line NUGC4 and the low-expressing cell line SNU620 was evaluated using the method of Example 9. The NUGC4 and SNU620 cell lines were purchased from ATCC.

NUGC4-luc cells were transformed with luciferase reporter gene by conventional gene manipulation methods. As shown in FIGS. 8H and 8I, each group of CAR-T cells had similar short-acting (72 hours) and long-acting (multiple consecutive rounds) killing functions against the highly expressing cell line NUGC4. As shown in FIG. 8J, the killing ability of sCD70 armored CAR-T cells against low expression target cell SNU620 was significantly superior to non-armored and membrane-bound CD70 armored structures.

### Example 13: Preparation and comparison of sCD70-armored and membrane-bound CD70-armored GCC CAR-T cells

In this example, a retroviral shuttle plasmid P-7 expressing a second-generation antigen receptor binding to GCC, a retroviral shuttle plasmid P-8 expressing an sCD70-armored GCC second-generation antigen receptor, a retroviral shuttle plasmid P-9 expressing a membrane-bound CD70 armored GCC second-generation antigen receptor were constructed, respectively, and the numbers corresponding to CARs are GCC, GCC-sCD70, GCC-mCD70. The specific steps are as follows.

### 1. Plasmid Construction

Referring to Figure 9, a chimeric antigen receptor for GCC CAR-T cells is constructed, the nucleic acid encoding the chimeric antigen receptor for T cells is cloned into a retroviral shuttle plasmid, and viral packaging is performed through a packaging plasmid.

The plasmids are constructed using conventional molecular biological methods in the art. The chimeric antigen receptor used in the present invention is a second-generation chimeric antigen receptor, which has the following elements: CD8α signal peptide (SEQ ID NO: 1), antigen binding domain, CD8α hinge region (SEQ ID NO: 2), CD28 transmembrane domain (SEQ ID NO: 5), CD28 intracellular co-stimulatory domain (SEQ ID NO: 7), CD3ζ signal transduction domain (SEQ ID NO: 8), P2A cleavage peptide (SEQ ID NO: 10). The amino acid sequences of each element are shown in Table 1.

The antigen binding domain is GCC-VHH, the sequence of which is shown in SEQ ID NO: 39, has the complementary determining regions (CDR) HCDR1, HCDR2, and HCDR3 shown in SEQ ID NO: 40-42. The amino acid sequences of the antigen binding domain are shown in Table 8.

GCC-sCD70 is armored with soluble CD70 (SEQ ID NO: 22) on basis of GCC, and GCC-mCD70 is armored with membrane-bound CD70 (SEQ ID NO: 23) on basis of GCC, and the specific sequences are shown in Table 9.

**Table 8. Sequence information of GCC CAR-T antigen binding domain and chimeric antigen receptor**

| SEQ ID NO | Element | Amino acid sequence |
|---|---|---|
| 39 | GCC-VHH | EVQLVESGGGLVKPGGSLRLSCAASGRTSTIHRMGW FRQAPGKEREFVSSISRVSSTTYYADSVKGRFTISRDN AKNSLYLQMNSLRAEDTAVYYCAASEYEVSPGKSKY WGQGTMVTVSS |
| 40 | GCC-VH-CDR1 | IHRMG |
| 41 | GCC-VH-CDR2 | SISRVSSTTYYADSVKG |
| 42 | GCC-VH-CDR3 | SEYEVSPGKSKY |

**Table 9. Amino acid sequence information of GCC CAR-T chimeric antigen receptor (with and without armor molecules)**

| SEQ ID NO: | Plasmid No. | CAR No. | Amino acid sequence |
|---|---|---|---|
| 43 | P-7 | GCC | MALPVTALLLPLALLLHAARPEVQLVESGGGL VKPGGSLRLSCAASGRTSTIHRMGWFRQAPGK EREFVSSISRVSSTTYYADSVKGRFTISRDNAK NSLYLQMNSLRAEDTAVYYCAASEYEVSPGKS KYWGQGTMVTVSSAAAIEVMYPPPYLDNEKS NGTIIHVKGKHLCPSPLFPGPSKPFWVLVVVGG VLACYSLLVTVAFIIFWVRSKRSRLLHSDYMN MTPRRPGPTRKHYQPYAPPRDFAAYRSRVKFS RSADAPAYQQGQNQLYNELNLGRREEYDVLD KRRGRDPEMGGKPRRKNPQEGLYNELQKDK MAEAYSEIGMKGERRRGKGHDGLYQGLSTAT KDTYDALHMQALPPR |
| 44 | P-8 | GCC-sCD 70 | MALPVTALLLPLALLLHAARPEVQLVESGGGL VKPGGSLRLSCAASGRTSTIHRMGWFRQAPGK EREFVSSISRVSSTTYYADSVKGRFTISRDNAK NSLYLQMNSLRAEDTAVYYCAASEYEVSPGKS KYWGQGTMVTVSSAAAIEVMYPPPYLDNEKS NGTIIHVKGKHLCPSPLFPGPSKPFWVLVVVGG VLACYSLLVTVAFIIFWVRSKRSRLLHSDYMN MTPRRPGPTRKHYQPYAPPRDFAAYRSRVKFS RSADAPAYQQGQNQLYNELNLGRREEYDVLD KRRGRDPEMGGKPRRKNPQEGLYNELQKDK MAEAYSEIGMKGERRRGKGHDGLYQGLSTAT KDTYDALHMQALPPRKRRSGSGATNFSLLKQ AGDVEENPGPMALPVTALLLPLALLLHAARPQ RFAQAQQQLPLESLGWDVAELQLNHTGPQQD PRLYWQGGPALGRSFLHGPELDKGQLRIHRDG IYMVHIQVTLAICSSTTASRHHPTTLAVGICSPA SRSISLLRLSFHQGCTIASQRLTPLARGDTLCTN LTGTLLPSRNTDETFFGVQWVRP |
| 45 | P-9 | GCC-mCD 70 | MALPVTALLLPLALLLHAARPEVQLVESGGGL VKPGGSLRLSCAASGRTSTIHRMGWFRQAPGK |
| | | | EREFVSSISRVSSTTYYADSVKGRFTISRDNAK NSLYLQMNSLRAEDTAVYYCAASEYEVSPGKS KYWGQGTMVTVSSAAAIEVMYPPPYLDNEKS NGTIIHVKGKHLCPSPLFPGPSKPFWVLVVVGG VLACYSLLVTVAFIIFWVRSKRSRLLHSDYMN MTPRRPGPTRKHYQPYAPPRDFAAYRSRVKFS RSADAPAYQQGQNQLYNELNLGRREEYDVLD KRRGRDPEMGGKPRRKNPQEGLYNELQKDK MAEAYSEIGMKGERRRGKGHDGLYQGLSTAT KDTYDALHMQALPPRKRRSGSGATNFSLLKQ AGDVEENPGPMPEEGSGCSVRRRPYGCVLRA ALVPLVAGLVICLVVCIQRFAQAQQQLPLESLG WDVAELQLNHTGPQQDPRLYWQGGPALGRSF LHGPELDKGQLRIHRDGIYMVHIQVTLAICSST TASRHHPTTLAVGICSPASRSISLLRLSFHQGCTI ASQRLTPLARGDTLCTNLTGTLLPSRNTDETFF GVQWVRP |

### 2. Preparation and identification of cells

The corresponding shuttle plasmids were prepared according to the methods in Examples 7 and 8, and viral packaging, T cell in vitro amplification and viral infection were performed to construct non-armored GCC CAR-T cells GCC, sCD70 armored GCC CAR-T cells GCC-sCD70 and membrane-bound CD70 armored GCC CAR-T cells GCC-mCD70.

The T cell characteristics of the two groups of CAR-T after in vitro expansion were evaluated by the method of Example 8.

The in vitro expansion fold results of GCC CAR-T cells are shown in Figure 10A. After 12 days of culture, the expansion folds of the GCC and GCC-sCD70 groups were both higher than 100 times, GCC-mCD70 group (76 times).

The levels of CAR expression by T cells 10 days after in vitro expansion of T cells after viral transfection are shown in Figure 10B , where the CAR expression levels of GCC, GCC-sCD70, and GCC-mCD70 were 84%, 63%, and 80%, respectively.

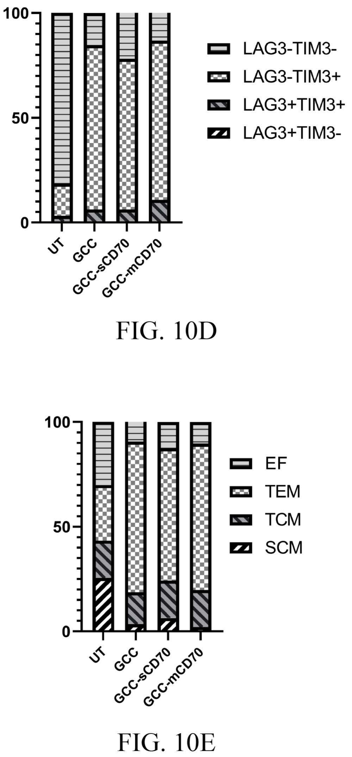

The flow cytometry results of T cell phenotype are shown in Figures 10C, 10D, and 10E. Among them, the content of CD4 + T cells in GCC and GCC-mCD70 groups was higher than that in GCC-sCD70 and untransfected T cells (Figure 10C); Both the depletion phenotype and memory phenotype were higher in the CAR-transfected groups than in the untransfected group, and the proportion of phenotypes was similar in the CAR-transfected groups (Figure 10D and Figure 10E).

### 5. Detection of the level of CD70 protein secreted by CAR-T cells and the expression of CD70 and CD27 on the surface of T cells by ELISA

During in vitro expansion culture, the culture supernatant was collected and used to detect the level of CD70 protein secreted by CAR-T cells. The supernatant of the culture medium (cultured for a total of 5 days) in which the CAR-T cell density was maintained at about 3~5E6 was centrifuged to remove cell debris, and the Human CD70 ELISA kit (Abcam, ab264621) was used for detection. The results are shown in Figure 10F, the CD70 protein secretion level of 74 ng/ml was detected in the culture supernatant of GCC-sCD70 group, which was significantly higher than that of other groups. These results indicate that sCD70 armored GCC CAR-T cells can normally express GCC CAR structure and secrete CD70 protein, which is significantly higher than non-armored and membrane-bound CD70 armored CAR-T cells.

The expression detection results of CD70 and CD27 on the T cell surface are shown in Figure 10G. The expression of CD70 on the cell membrane surface of the GCC-mCD70 group was 91.5%, which was significantly higher than that of non-armored (23%) and secretable CD70 (40%) group, correspondingly, the expression of CD27 on the membrane surface of the GCC-mCD70 group was 3.98%, which was significantly lower than the non-transfected and other CAR-T groups (75% to 90%). The above results indicate that membrane-bound CD70 armored GCC CAR-T cells can normally express membrane-bound CD70, while excessive binding of CD70 and CD27 leads to the shedding of CD27 on the surface of T cells, which in turn affects the degree of activation of CAR-T cells.

### 6. In vitro killing experiments of sCD70-armored and membrane-bound CD70-armored GCC CAR-T cells

The killing effect of each group of GCC CAR-T cells on the GCC low-expressing cell line LS-174T was evaluated using the method of Example 9. The LS-174T cell were transformed with luciferase reporter gene by conventional gene manipulation methods. As shown in FIGS. 10H and 10I, during the 72-hour killing process, at different E:T ratios, the killing effect of the sCD70-armored CAR-T cells on LS-174T-lu target cells was significantly better than that of the GCC and GCC-mCD70 structures (FIG. 10H). The sCD70 armored structure showed the most lasting killing function in serial killing experiments (FIG. 10I), while the killing ability of membrane-bound CD70 armor was weaker than that of non-armor structure, possibly because CD27 expression was significantly lower than that of non-armor and secretable CD70 armor groups, thus affecting the function of CAR-T cells.

The above results prove that the sCD70 armor structure can improve the activation degree and specific killing ability of CAR-T without affecting the expression level of CD27, and has the advantage of improving the function of CAR-T compared with the membrane-bound CD70 armor structure. The above results show that the sCD70 armor structure has the ability to improve the specific killing function and activity of T cells for various targeted CAR structures.

In sum, the Examples 1-13 demonstrate that by constructing CARs targeting different tumor antigens (DLL3, GCC, CLDN18.2 and GPC3) and utilizing different forms of antigen binding domains (scFv and VHH), the inventive concept of loading armored molecules (such as soluble human CD70) capable of activating the CD70/CD27 signaling pathway into CAR to improve the function of CAR-T was verified.

While example embodiments have been particularly shown and described, it will be understood by those skilled in the art that various changes in form and details may be made therein without departing from the scope of the embodiments encompassed by the appended claims. The full scope of the present application is given by the appended claims and any equivalents thereof.

## Claims

1. A polynucleotide, encoding:
a) a chimeric antigen receptor (CAR), wherein the CAR comprises an antigen-binding domain specific for a cell surface antigen; and
b) an armoring molecule capable of activating a CD70/CD27 signaling pathway.

2. The polynucleotide of claim 1, wherein the armoring molecule activates the CD70/CD27 signaling pathway by engaging CD27.

3. The polynucleotide of claim 1 or 2, wherein the armoring molecule is a soluble human CD70 (e.g., comprising the amino acid sequence shown in SEQ ID NO: 22 or 47) or variant thereof.

4. The polynucleotide of claim 3, wherein the armoring molecule has at least about 90% amino acid sequence identity to SEQ ID NO: 22 or 47.

5. The polynucleotide of any one of claims 1-4, wherein the cell surface antigen is expressed in a solid tumor cell or a hematologic cancer cell.

6. The polynucleotide of any one of claims 1-5, wherein the cell surface antigen is expressed in cancer cells selected from the group consisting of breast cancer, lung cancer, prostate cancer, colon cancer, bladder cancer, ovarian cancer, kidney cancer, stomach cancer, rectal cancer, colorectal cancer, testicular cancer, head and neck cancer, pancreatic cancer, brain cancer and skin cancer.

7. The polynucleotide of any one of claims 1-6, wherein the cell surface antigen is expressed in small cell lung cancer cells, colon adenocarcinoma cells, gastric cancer cells, liver cancer cells or hepatocellular carcinoma cells.

8. The polynucleotide of any one of claims 1-7, wherein the cell surface antigen comprises a DLL3 (Delta-like ligand 3) antigen, a GUCY2C (GCC) antigen, a Claudin 18.2 (CLDN18.2) antigen or a glypican 3 (GPC3) antigen, *e.g*., the antigen-binding domain comprises an amino acid sequence having at least 90% sequence identity to the amino acid sequence set forth in any one of SEQ ID NOs: 13, 27, 39, 48-54, and 64-76.

9. The polynucleotide of any one of claims 1-8, wherein the antigen-binding domain comprises a mutein, a single-chain variable fragment (scFv), single-domain antibody (sdAb) (*e.g.,* a VHH, a nanobody), or any combination of the foregoing.

10. The polynucleotide of any one of claims 1-9, wherein the antigen-binding domain comprises a single-chain variable fragment targeting DLL3 (Delta-like ligand 3), Claudin 18.2 (CLDN18.2) or glypican 3 (GPC3), or a single domain antibody (sdAb, *e.g.,* a VHH, a nanobody) targeting GUCY2C (GCC).

11. The polynucleotide of any one of claims 1-10, wherein the CAR comprises:
a CD8α signal peptide;
a CD8α hinge or a CD28 hinge;
a CD8α transmembrane domain or a CD28 transmembrane domain;
a 4-1BB costimulatory domain or a CD28 costimulatory domain; or
a CD3ζ signaling domain, or
any combination of the foregoing.

12. The polynucleotide of claim 11, wherein the CAR comprises:
the CD8α signal peptide having at least 90% sequence identity to SEQ ID NO:1, 58 or 59;
the antigen-binding domain having at least 90% sequence identity to SEQ ID NO:13, 27, 39, 48-54, or 64-76;
the CD8α hinge having at least about 90% amino acid sequence identity to SEQ ID NO:2, or the CD28 hinge having at least about 90% amino acid sequence identity to SEQ ID NO:3;
the CD8α transmembrane domain having at least about 90% amino acid sequence identity to SEQ ID NO:4, or the CD28 transmembrane domain having at least about 90% amino acid sequence identity to SEQ ID NO:5;
the 4-1BB costimulatory domain having at least about 90% amino acid sequence identity to SEQ ID NO:6, or the CD28 costimulatory domain having at least about 90% amino acid sequence identity to SEQ ID NO:7;
the CD3ζ signaling domain having at least about 90% amino acid sequence identity to SEQ ID NO:8 or 60,
or any combination of the foregoing.

13. The polynucleotide of claim 12, wherein the CAR comprises:
the CD8α signal peptide comprising the amino acid sequence of SEQ ID NO:1, 58 or 59;
the antigen-binding domain comprising the amino acid sequence of SEQ ID NO:13, 27, 39, 48-54, or 64-76;
the CD8α hinge comprising the amino acid sequence of SEQ ID NO:2, or the CD28 hinge comprising the amino acid sequence of SEQ ID NO:3;
the CD8α transmembrane domain comprising the amino acid sequence of SEQ ID NO:4, or the CD28 transmembrane domain comprising the amino acid sequence of SEQ ID NO:5;
the 4-1BB costimulatory domain comprising the amino acid sequence of SEQ ID NO:6, or the CD28 costimulatory domain comprising the amino acid sequence of SEQ ID NO:7;
the CD3ζ signaling domain comprising the amino acid sequence of SEQ ID NO:8 or 60, or any combination of the foregoing.

14. The polynucleotide of any one of claims 1-13, wherein the CAR has at least about 90% sequence identity to SEQ ID NO:24, 36, 43 or 77.

15. The polynucleotide of any one of claims 1-14, further comprising an internal ribosome entry site (IRES).

16. The polynucleotide of any one of claims 1-15, encoding:
a) the chimeric antigen receptor (CAR);
b) a self-cleaving peptide, *e.g.,* the amino acid sequence of the self-cleaving peptide has at least about 90% sequence identity to SEQ ID NOs: 9-10 and 78-84; and
c) the soluble human CD70 armoring molecule.

17. The polynucleotide of any one of claims 1-16, wherein the polynucleotide encodes an amino acid sequence having at least about 90% sequence identity to SEQ ID NO:25, 37, 44 or 63.

18. A vector comprising the polynucleotide of any one of claims 1-17.

19. A fusion protein encoded by the polynucleotide of any one of claims 1-17 or the vector of claim 18.

20. A host cell comprising the polynucleotide of any one of claims 1-17, the vector of claim 18, or the fusion protein of claim 19.

21. Immune effector cells that are genetically engineered to express chimeric antigen receptor and a soluble CD70.

22. Immune effector cells comprising the polynucleotide of any one of claims 1-17, the vector of claim 18, or the fusion protein of claim 19.

23. The immune effector cells of claim 21 or 22, wherein the immune effector cells are T lymphocytes.

24. The immune effector cells of claim 21 or 22, wherein the immune effector cells are natural killer (NK) cells.

25. A composition comprising the immune effector cells of any one of claims 21-24.

26. A pharmaceutical composition comprising the composition of claim 25 and a pharmaceutically acceptable carrier.

27. A kit comprising a container and optionally an instruction for use, wherein the container comprises the composition of claim 25 or the pharmaceutical composition of claim 26.

28. A method of treating cancer in a subject in need thereof, comprising administering to the subject an effective dosage of the immune effector cells of any one of claims 21-24.

29. The immune effector cells of any one of claims 21-24, the polynucleotide of any one of claims 1-17, the vector of claim 18, the fusion protein of claim 19, the host cell of claim 20, the composition of claim 25, the pharmaceutical composition of claim 26, or the kit of claim 27, for use in treating cancer in a subject in need thereof.

30. Use of the immune effector cells of any one of claims 21-24, the polynucleotide of any one of claims 1-17, the vector of claim 18, the fusion protein of claim 19, the host cell of claim 20, the composition of claim 25, the pharmaceutical composition of claim 26, or the kit of claim 27, for the preparation of a medicament for treating cancer in a subject in need thereof.

31. A method of treating cancer in a subject in need thereof, comprising administering to the subject an effective dosage of the immune effector cells of any one of claims 21-24, the polynucleotide of any one of claims 1-17, the vector of claim 18, the fusion protein of claim 19, the host cell of claim 20, the composition of claim 25, the pharmaceutical composition of claim 26, or the kit of claim 27.

32. A method of inducing cytolysis of cancer cells, comprising contacting the cancer cells with an effective dosage of the immune effector cells of any one of claims 21-24, the composition of claim 25, the pharmaceutical composition of claim 26, or the kit of claim 27.

33. The method of any one of claims 28-32, wherein the cancer is a hematologic cancer.

34. The method of any one of claims 28-32, wherein the cancer is breast cancer, lung cancer, prostate cancer, colon cancer, bladder cancer, ovarian cancer, kidney cancer, stomach cancer, rectal cancer, colorectal cancer, testicular cancer, head and neck cancer, pancreatic cancer, brain cancer or skin cancer.

35. The method of claim 34, wherein the cancer is lung, colorectal, or stomach cancer.

36. The method of claim 35, wherein the cancer is small cell lung cancer, colon adenocarcinoma, or stomach cancer.

37. The method of any one of claims 28-32, wherein the cancer is a solid tumor.

38. The method of claim 37, wherein the solid tumor is a liver cancer.

39. The method of claim 38, wherein the liver cancer is hepatocellular carcinoma (HCC).

40. The method of any one of claims 32-39, wherein the method occurs in vitro.

41. A polynucleotide combination comprising:
a) a first polynucleotide encoding a chimeric antigen receptor (CAR), wherein the CAR comprises an antigen-binding domain specific for a cell surface antigen; and
b) a second polynucleotide encoding an armor molecule capable of activating a CD70/CD27 signaling pathway.

42. A vector combination, comprising: a first vector comprising the first polynucleotide of claim 41 and a second vector comprising the second polynucleotide of claim 41.

43. A host cell comprising the first polynucleotide and the second polynucleotide of claim 41.
